# EUROPEAN PATENT APPLICATION

(11) **EP 1 281 755 A2**
(43) Date of publication of application: **05.02.2003**
(21) Application number: 02254972.9
(22) Date of filing: 16.07.2002
(51) Int. Cl.: C12N 9/02, C12N 15/53, C12Q 1/68, C07K 16/40

(54) **Variants of the human cyp2d6 gene**

(30) Priority: 31.07.2001 US 309111 P
(71) Applicant: Pfizer Products Inc., Groton, Connecticut 06340 (US)
(72) Inventor: Milos, Patrice Marie, Pfizer Global Res. & Dev., Groton, Connecticut 06340 (US); Webb, Suzin Marie, Pfizer Global Res. & Dev., Groton, Connecticut 06340 (US)
(74) Representative: Hayles, James Richard

(57) **Abstract**

The invention provides novel cytochrome P450 2D6 gene variants. Also provided, *inter alia*, are polypeptides, primers, vectors, host cells, antibodies, agonists, antagonists, gene and protein chips, methods for detecting susceptibility to drug sensitivity, methods of treatment, and kits.

## Description

### FIELD OF THE INVENTION

The present invention relates to interindividual variation in drug metabolism. The present invention also relates to genetic variation and attendant genetic markers. More specifically, the present invention relates to the identification of a novel mutant allele of the CYP2D6 gene locus that results in a frameshift in a critical region of the gene which, in the wild-type enzyme, is required for catalytic activity. The mutant allele is associated with a genetic deficiency for metabolizing agents that are substrates of P450 CYP2D6.

### BACKGROUND OF THE INVENTION

Polymorphic genes have been identified that result in interindividual variation in drug metabolism. See, for example, U.S. Patent No. 5,648,482. Interest remains in identifying genetic factors that influence or give rise to such interindividual variation.

More than 200 cytochrome P450 genes which encode products involved in Phase I drug metabolism have been identified. These enzymes are involved in the metabolism of numerous other xenobiotics such as, for example, carcinogens, environmental chemicals, and several classes of endobiotics, e.g., steroids and prostaglandins.

The cytochrome P450 2D6 gene (CYP2D6), localized to chromosome 22, encodes a major Phase I drug metabolizing enzyme, debrisoquine hydroxylase, the polymorphic oxidation of which is one of the most characterized interindividual variations of drug metabolism. See, for example, *Lancet*: 584-586 (1977); *Eur. J. Clin. Pharmacol.* 16: 183-187 (1979); *Genomics* 2: 174-179 (1988); *Nature* 331: 442-446 (1998).

Genetic factors, e.g., the presence of mutated nucleotide sequences in certain genes, can play a role in interindividual variation in drug metabolism. In the case of debrisoquine polymorphism, the poor metabolizer (PM) phenotype is associated with an inability to efficiently metabolize several drugs, which can cause exaggerated pharmacological responses. See, for example, *Pharmac. Ther.* 46: 297-308 (1990). Deciphering the genetic basis for the debrisoquine PM phenotype has led to a report that the PM phenotype is caused by the absence in the liver of the enzyme encoded by the CYP2D6 gene. See, for example, *DNA* 8: 1-13 (1989); and *Biochemistry* 27: 5447-5454 (1988).

Several mutant alleles of the CYP2D6 gene associated with the PM phenotype have been reported (i.e., genotypes). See, for example, *Proc. Natl. Acad. Sci. USA* 85: 5240-5243 (1988). Identification of such genotypes, and screening therefor, could enable one to predict an individual's metabolism of certain drugs, and thus assist in avoidance of, e.g., the exaggerated pharmacological responses mentioned hereinabove. Such genotype identification can be advantageous over administering the drug to the individual and assessing the phenotype resultant therefrom.

Given the interindividual variation in drug metabolism, and that genetic factors have been shown to influence the individual response to drugs, and that CYP2D6 encodes a major Phase I drug metabolizing enzyme which is involved in the metabolism of numerous drugs, and that mutant alleles of the CYP2D6 gene are associated with the debrisoquine PM phenotype, a need remains to continue to identify novel PM alleles for the CYP2D6 gene, and assays for screening of the such genotypes.

The present invention furthers this work by providing a novel mutant allele of the CYP2D6 gene locus. This mutation results in a frameshift in a critical region of the gene which, in the wild-type enzyme, is required for catalytic activity. Importantly, this mutant allele is associated with a genetic deficiency for metabolizing agents that are substrates of P450 CYP2D6, e.g., the PM phenotype, and, as such, can assist the art in further deciphering of interindividual variation in drug metabolism.

All of the documents cited herein, including the foregoing, are incorporated by reference herein in their entireties.

### SUMMARY OF THE INVENTION

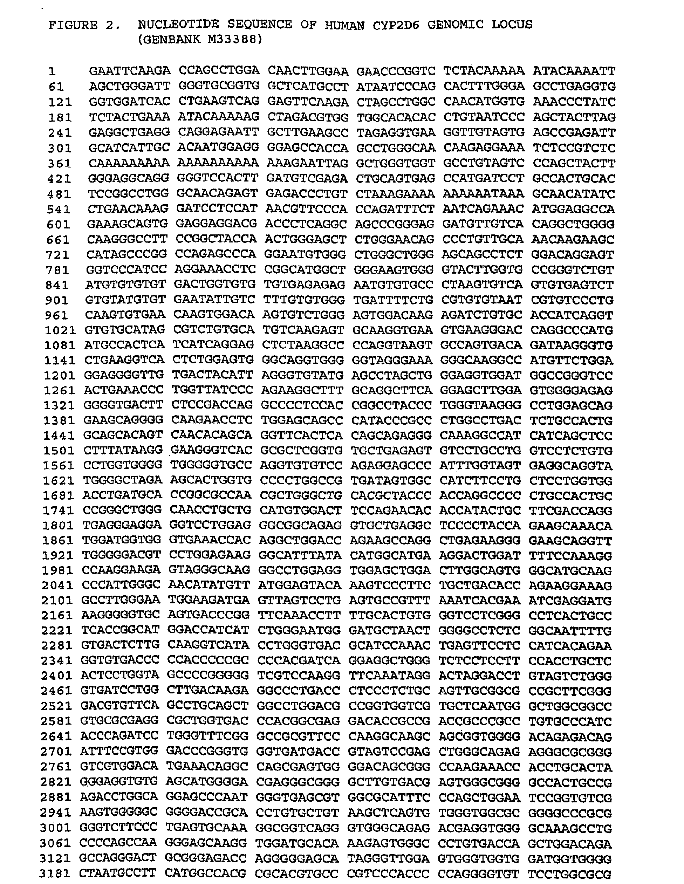

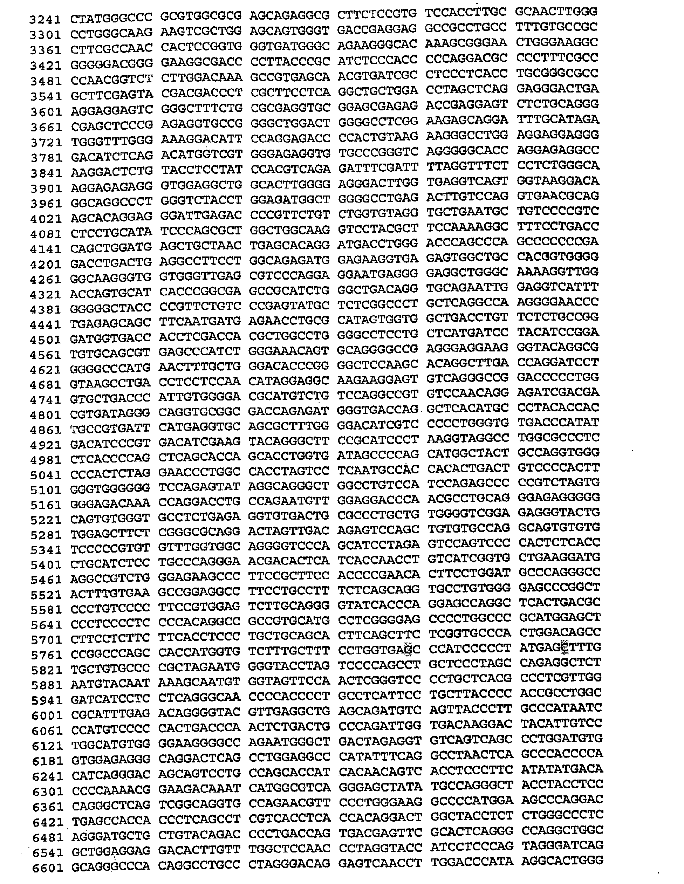

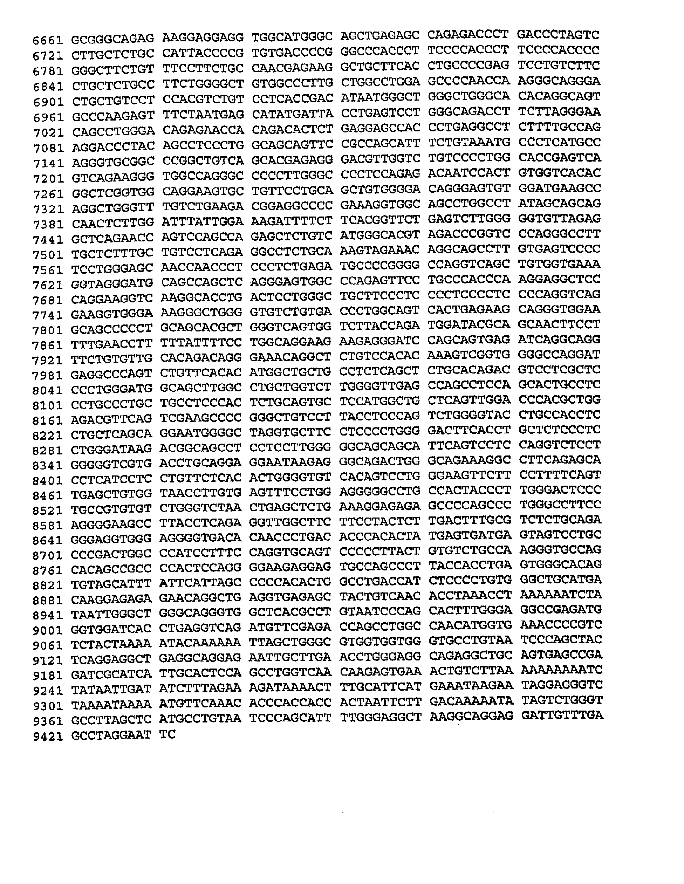

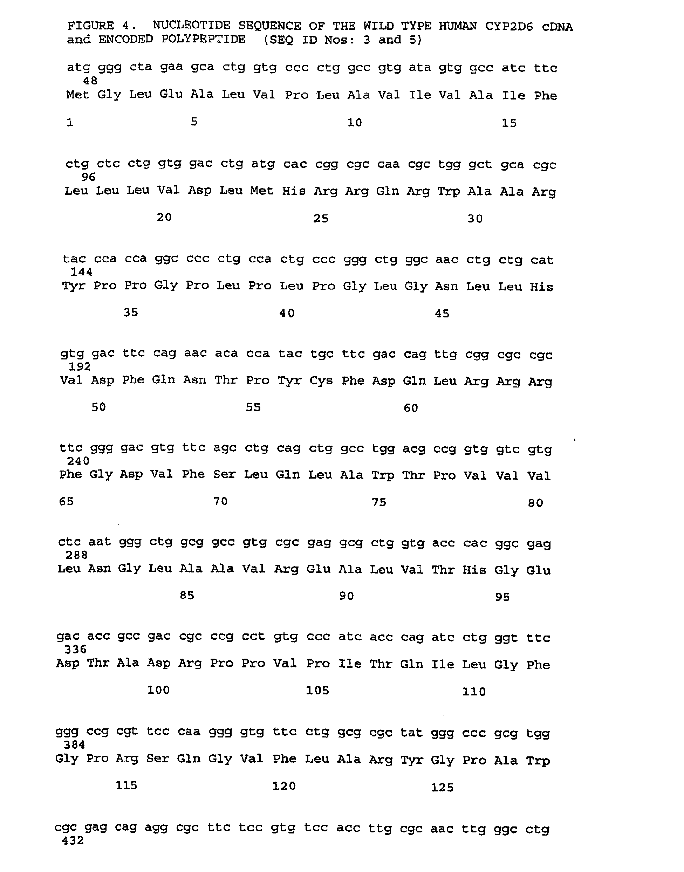

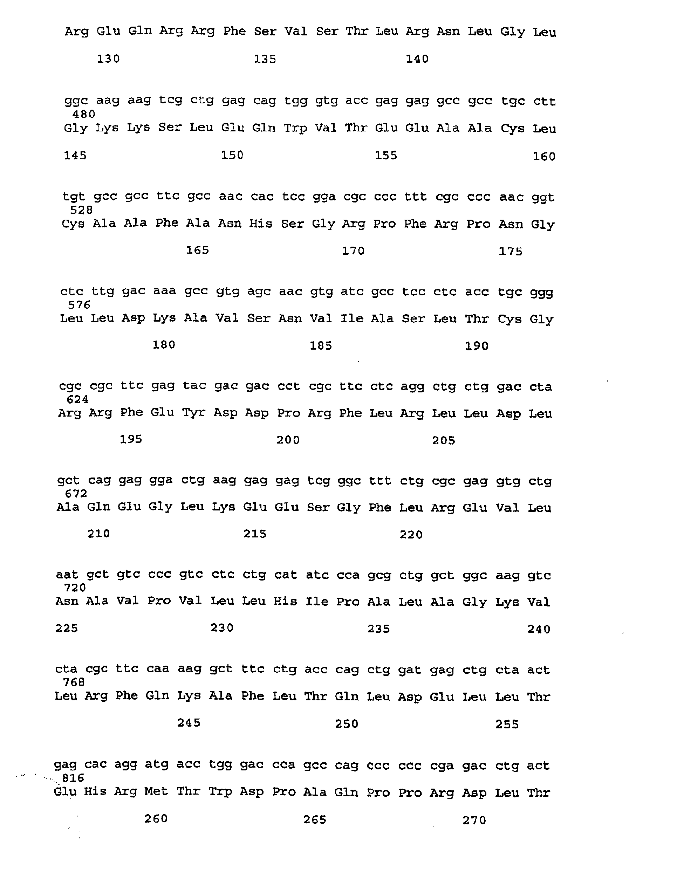

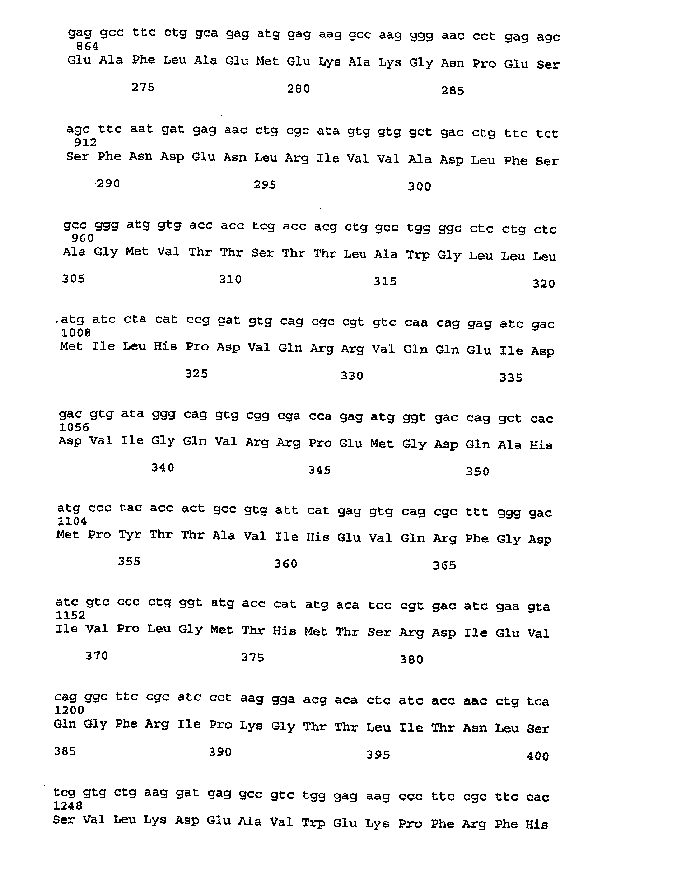

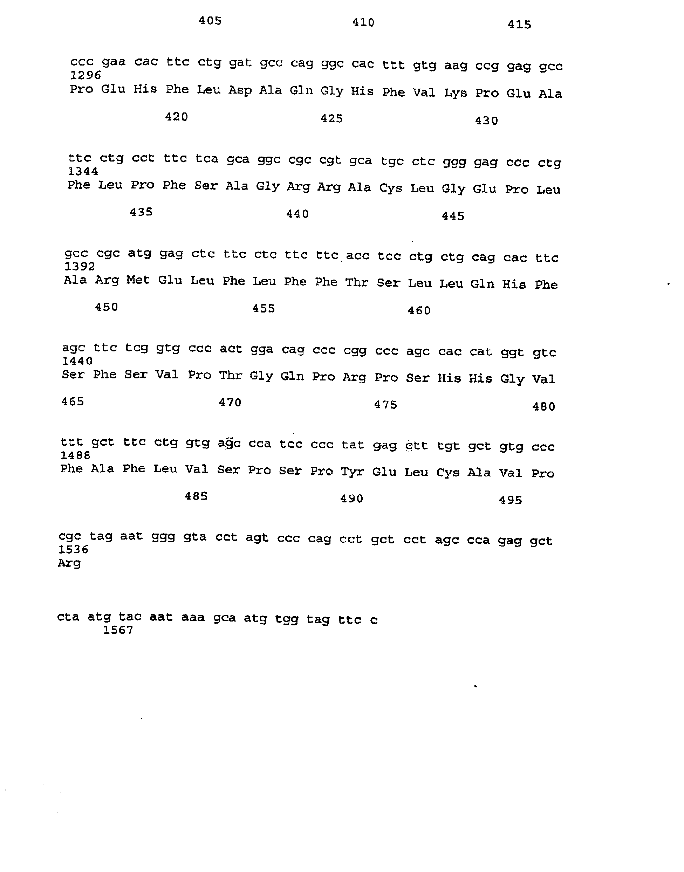

The present invention relates to novel CYP2D6 polymorphic variants that are linked or associated with a genetic deficiency for metabolizing certain drugs; specifically, individuals having differing variants of CYP2D6 may differ in their ability to metabolize drugs that are the substrates for P450CYP2D6 enzymes. Examples include variations at position 5816 and position 5799 of the CYP2D6 gene. Specifically, the variation at position 5816 corresponds to a substitution of the sequence "TA" for the "C" of the CYP2D6 genomic sequence (GenBank Accession No. M33388; SEQ ID NO. 1 shown in FIGURE 2) and at position 1474 of the CYP2D6 cDNA sequence (GenBank Accession No. NM_000106; SEQ ID NO. 3 shown in FIGURE 4). The variation at position 5799 corresponds to the substitution of G at this position of the genomic sequence with a C.

Accordingly, in one aspect, this invention provides nucleic acids comprising the CYP2D6 gene, preferably nucleic acid molecules comprising at least 20 consecutive nucleotides of an allele of a CYP2D6 gene having SEQ ID NO: 1, wherein the nucleotide sequence differs at either nucleotide position 5816 or position 5799 or at both positions from the nucleotide sequence of SEQ ID NO: 1. In one embodiment, the nucleotide C at position 5816 is replaced by TA. In another embodiment, C replaces the G at position 5799. In yet another embodiment, this invention includes nucleic acid sequence comprising both a TA at position 5816 and a C at position 5799 of SEQ ID No: 1. Other preferred nucleic acids include: SEQ ID No: 2 (C5816TA mutant genomic sequence), SEQ ID No: 4 (C5816TA mutant cDNA sequence), SEQ ID No: 7 (double mutant cDNA), SEQ ID No. 29 (G5799C mutant cDNA sequence), and SEQ ID No. 33 (mutant exon 9 sequence).

In another aspect the invention provides probes and primers to detect a genetic deficiency for metabolizing drugs, e.g., a poor metabolizer genotype. The nucleic acids of the invention can be used, for e.g., in prognostic, diagnostic, and therapeutic methods. For instance, the nucleic acids of the invention can be used as probes or primers to determine whether a subject has a genetic deficiency for metabolizing certain drugs. In particular, for determining whether a subject has a genetic deficiency for metabolizing drugs that are substrates of P450CYP2D6.

In yet another aspect, this invention provides an array of nucleic acid molecules attached to a support, wherein the array has an oligonucleotide that will hybridize to an allelic variant of CYP2D6 but will not substantially hybridize to the wild type sequence. In particular, the array has an oligonucleotide that will hybridize to SEQ ID NO. 2, but will not substantially hybridize to the nucleic acid sequence of SEQ ID NO. 1.

The invention further provides vectors comprising the nucleic acids of this invention; host cells transfected with said vectors whether prokaryotic or eukaryotic; and transgenic non-human animals which contain a heterologous form of a CYP2D6 P450 C(5816) variant described herein. Such a transgenic animal can serve as an animal model for studying, e.g., the effects of specific allelic variations, including mutations of the CYP2D6 gene in drug metabolism.

This invention also provides polypeptides encoded by the allelic variants of this invention. In a preferred embodiment, such polypeptides have a C-terminus comprising the amino acid sequence of YLCCAPLEWGT. The invention also provides purified antibodies that selectively bind to the mutant CYP2D6 amino acid sequence but do not substantially bind the wild type polypeptide sequence. In a preferred embodiment, such antibodies selectively bind an epitope comprising residues 481-502 of the amino acid sequence of SEQ ID NO. 6. In a more preferred embodiment, the antibodies selectively bind an epitope which comprises the amino acid sequence of YLCCAPLEWGT.

The methods of this invention can be used, e.g., for determining the identity of the allelic variant of a polymorphic region of a human CYP2D6 gene present in a subject. For example, the methods of the invention can be useful for determining whether a subject has a genetic deficiency for metabolizing certain drugs, for example drugs that are substrates of P450 CYP2D6. Genetic variations of this gene locus result in a genetic deficiency in drug metabolism or a drug sensitivity condition because of the altered enzymatic activities of the variant CYP2D6 gene products. In particular, the genetic variations at this gene locus are linked to aberrant CYP2D6 levels or aberrant CYP2D6 bioactivities. As those skilled in the art will appreciate, a majority of individuals possess normal activity (extensive metabolizers), some individuals possess slightly reduced activity (intermediate metabolizers) and some individuals show increased enzyme activity, in part due to gene duplications (rapid metabolizers). Individuals who lack enzyme activity, due to inactivating mutations in both copies of the CYP2D6 gene, are unable to metabolize drugs that require the CYP2D6 enzyme and are referred to as CYP2D6 poor metabolizers. Accordingly, the present methods provide means for determining if a subject has (diagnostic), or is at risk of developing (prognostic), a drug sensitivity condition or disorder that is associated with an aberrant CYP2D6 activity, e.g., an aberrant level of a CYP2D6 protein or an aberrant CYP2D6 bioactivity.

In one aspect of the present invention, the identity of a polymorphic region of a CYP2D6 gene may be determined by contacting a sample nucleic acid with a probe or a primer which hybridizes to a polymorphic region selected from the group consisting of nucleotides 5816 and 5799 of SEQ ID NO. 1. Exemplary known methods for determining the identity of a polymorphic region include: determining the nucleotide content of the polymorphic region by sequencing or by performing a restriction enzyme site analysis, by single-stranded conformation polymorphism, allele specific hybridization, primer specific extension, oligonucleotide ligation assay, and such methods may readily be performed by those skilled in the art based upon the present description.

In another embodiment, this invention provides methods for genotyping an individual by obtaining a sample of DNA from an individual and determining the identity of the nucleotide at position 5816 of the genomic sequence of CYP2D6. Other embodiments include methods for evaluating therapy with a drug metabolized by P450 CYP2D6., for example, evaluating therapy for a patient having a cardiovascular or psychiatric disorder with a drug metabolized by P450 CYP2D6. Exemplary methods include obtaining a sample of DNA from an individual, determining the identity of the nucleotide at position 5816 of the genomic sequence of CYP2D6, and then determining whether or not that patient should undergo therapy with a drug metabolized by P450 CYP2D6 if the nucleotide at position 5816 is not a cytosine.

The methods of the invention can therefore be used, e.g., in selecting the appropriate drugs or determining the course of treatment to administer to a subject to treat cardiovascular or psychiatric disorders. In a further embodiment, the invention provides a method for treating a subject having a drug sensitivity or disorder associated with a specific allelic variant of a polymorphic region of the CYP2D6 gene. In one embodiment, the drug sensitivity condition or disorder is associated with an aberrant CYP2D6 activity, e.g., an aberrant level or aberrant bioactivity. For example, in one aspect, the method comprises (a) determining the identity of the allelic variant; and (b) administering to the subject a compound that compensates for the effect of the specific allelic variant. In a preferred embodiment, the specific allelic variant is a mutation. In a preferred embodiment, the compound modulates (e.g., agonizes, antagonizes, inversely antagonizes) a CYP2D6 protein level or bioactivity. In a preferred embodiment, the compound is selected from the group consisting of a nucleic acid, a protein, a peptidomimetic, and a small molecule.

In another embodiment, the invention provides a kit for determining DNA variations in the CYP2D6 gene in a subject, comprising: a) at least one of PCR primer sets; and b) at least one of the allele-specific oligonucleotide (ASO) probe. The invention also provides kits, e.g., for amplifying and/or determining the identity or structure of a portion of the CYP2D6 gene comprising a probe or a primer capable of hybridizing to an allelic variant of a polymorphic region. In a preferred embodiment, the polymorphic region is located in an exon, such as exon 9. In a preferred embodiment, determining the molecular structure of a region of the CYP2D6 gene comprises determining the identity of at least one nucleotide or determining the nucleotide composition, e.g., the nucleotide sequence.

A kit of the invention can be used, e.g., for determining whether a subject has a genetic deficiency associated with a specific allelic variant of a polymorphic region of a CYP2D6 gene. In a preferred embodiment, the invention provides a kit for determining whether a subject has a genetic deficiency for metabolizing certain agents, such as drugs that are substrates for P450 CYP2D6. The kits of the invention can also be used, for example, in selecting the appropriate drug to administer to a subject having a drug sensitivity or condition associated with aberrant CYP2D6 activity or aberrant CYP2D6 levels. Thus, determining the allelic variants of CYP2D6 polymorphic regions of an individual can be useful in predicting how an individual will respond to a specific agent, e.g, a drug that is a substrate for P450CYP2D6.

Other features and advantages of the invention will be apparent from the following detailed description and appendant claims.

### BRIEF DESCRIPTION OF THE FIGURES

FIGURE 1 provides a preferred embodiment of a method of the present invention for identifying a novel CYP2D6 mutation.
FIGURE 2 provides the sequence of the human CYP2D6 genomic locus (GenBank No. M33388; SEQ ID NO. 1).
FIGURE 3 provide a preferred embodiment of a sequence of the present invention of a human CYP2D6 C5816TA mutant genomic locus (SEQ ID NO. 2).
FIGURE 4 provides a sequence of a human CYP2D6 cDNA and the encoded polypeptide (GenBank No. NM_000106; SEQ ID NO. 3 & SEQ ID NO. 5)
FIGURE 5 provides a preferred embodiment of a sequence of the present invention of a human CYP2D6 C5816TA mutant cDNA and encoded polypeptide (SEQ ID NO. 4 & SEQ ID NO. 6).
FIGURE 6 shows the partial sequence of wild type CYP2D6 exon 9 and the corresponding encoded P450 carboxy-terminal amino acid sequence (PANEL A), with the "C" at nucleotide 5816 highlighted (SEQ ID NO. 31 & SEQ ID NO. 32); the partial sequence of the CYP2D6 C5816TA mutant exon 9 sequence and the corresponding encoded mutant P450 carboxy-terminal amino acid sequence (PANEL B), with the "TA" substitution at position 5816 highlighted (SEQ ID NO. 33 & SEQ ID NO. 34). Panel C depicts an alignment of the wild-type and predicted C5816TA mutant P450 carboxy-terminal polypeptide sequences.

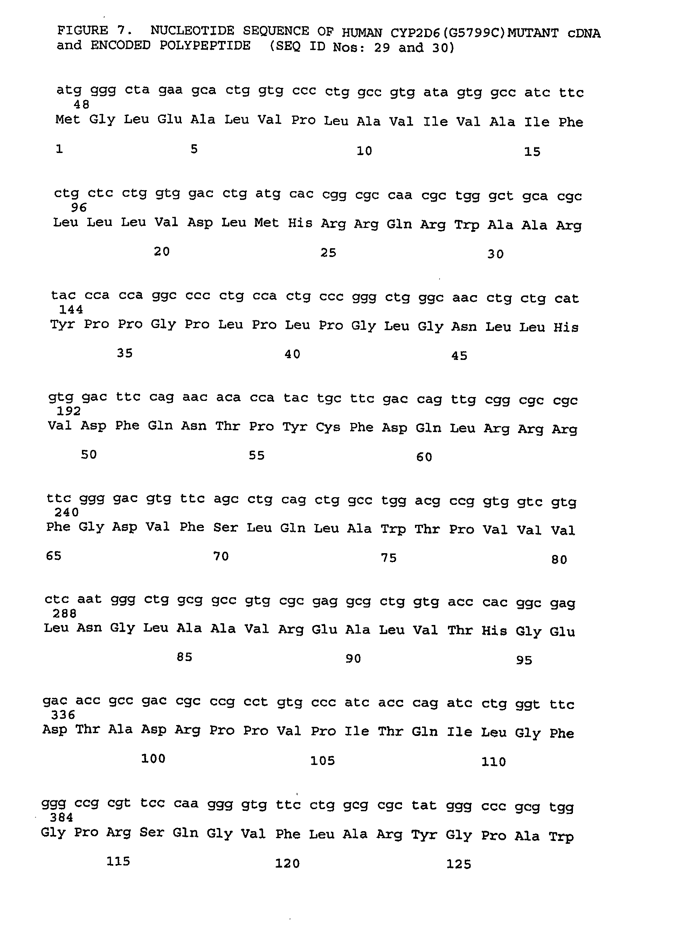

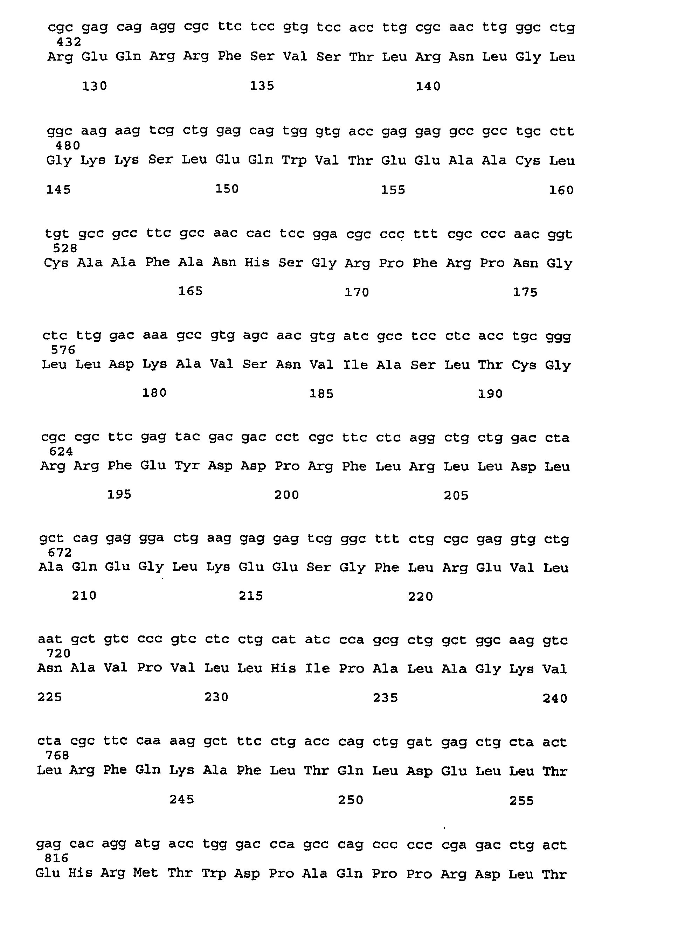

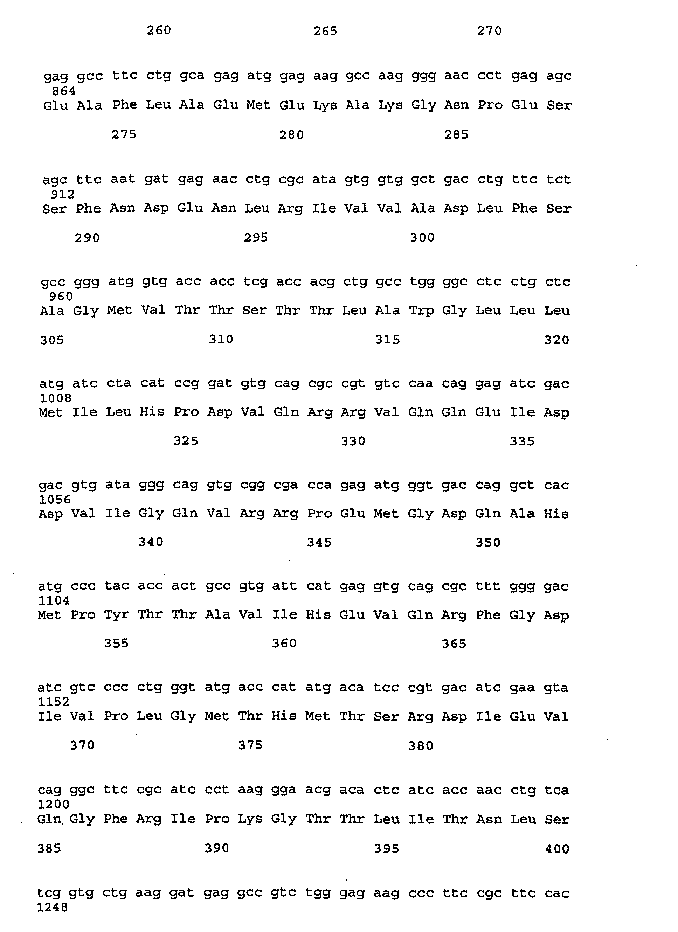

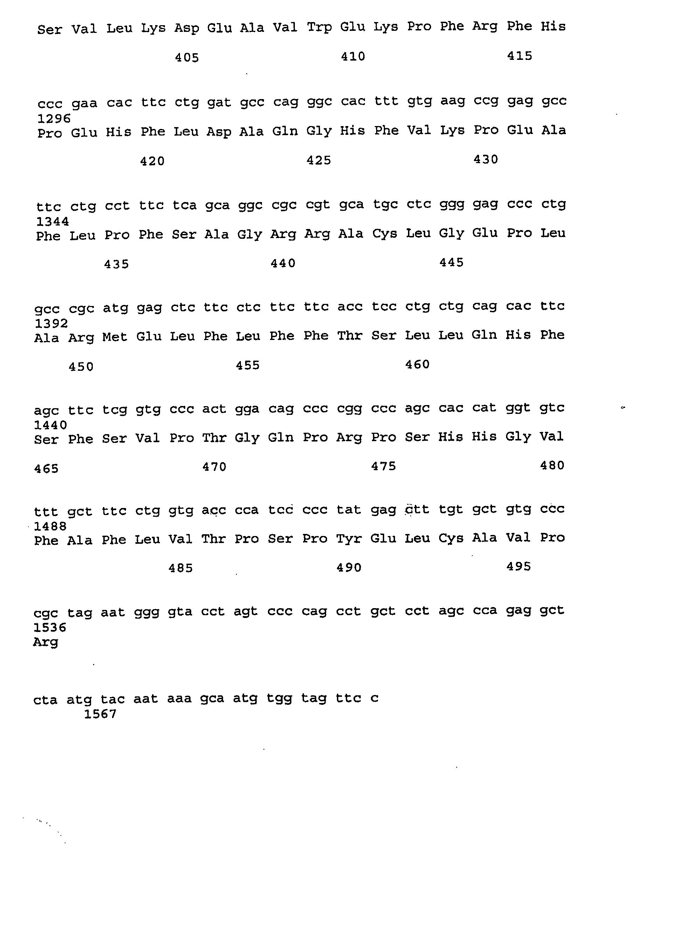

FIGURE 7 provides a preferred embodiment of a sequence of the present invention of a human CYP2D6 G5799C mutant cDNA and encoded polypeptide (SEQ ID NO. 29 & SEQ ID NO. 30).

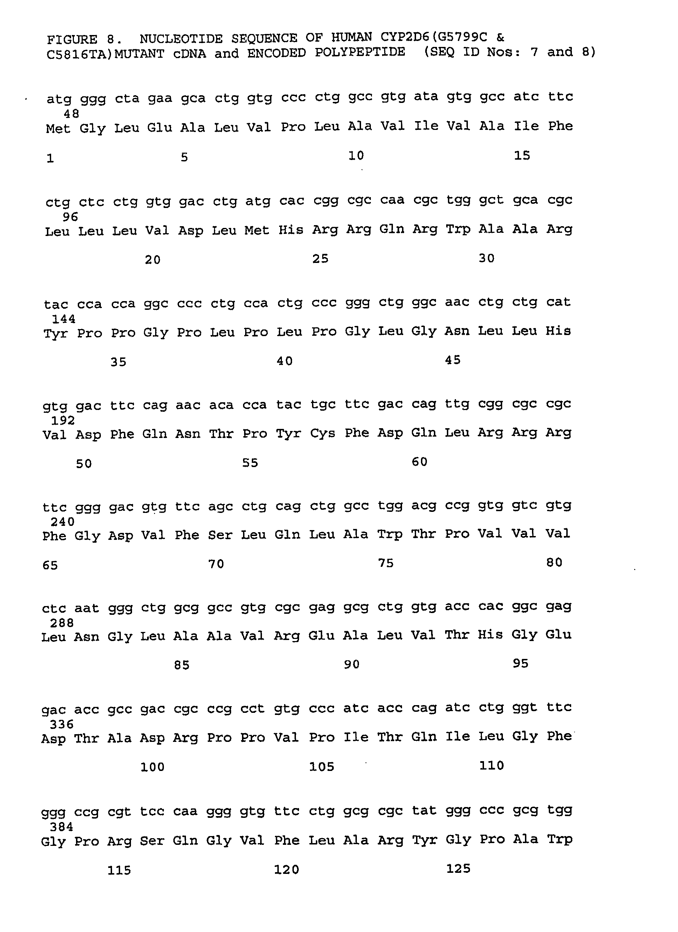

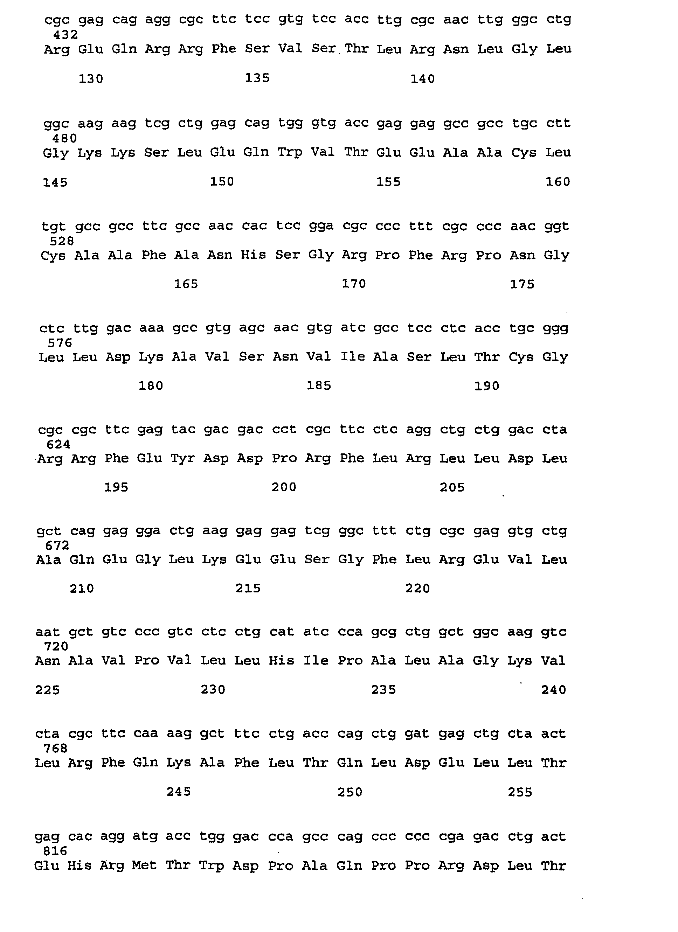

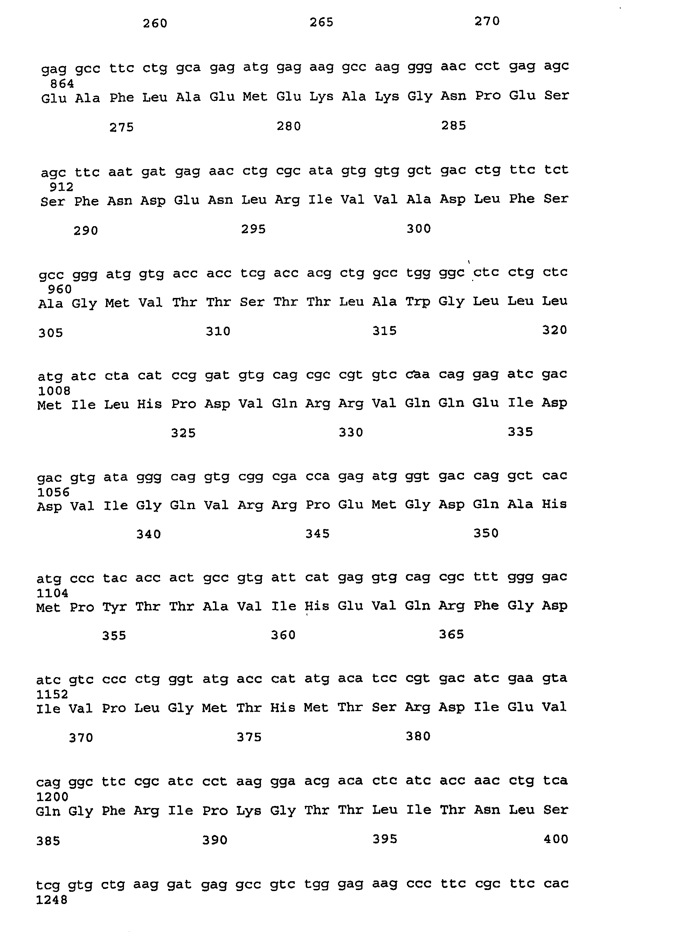

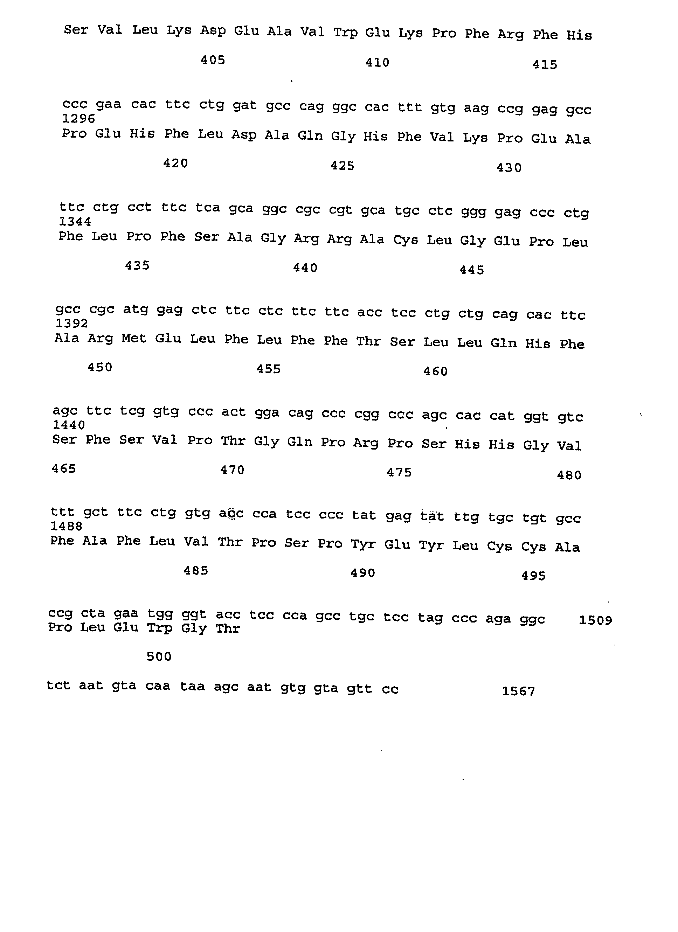

FIGURE 8 provides a preferred embodiment of a sequence of the present invention of a human CYP2D6 G5799C and C5816TA double mutant cDNA and encoded polypeptide (SEQ ID NO. 7 & SEQ ID NO. 8).

### DETAILED DESCRIPTION OF THE INVENTION

Unless described otherwise, all technical and scientific terms used herein have the same meanings as commonly understood by one of ordinary skill in the art. For convenience, the meaning of certain terms and phrases employed in the description and appendant claims are provided below.

"Aberrant activity," as applied to an activity of a polypeptide such as CYP2D6 P450, refers to an activity which differs from the activity of a polypeptide encoded by the wild-type or most common allele or which differs from the activity of the polypeptide in a healthy subject. An activity of a polypeptide can be aberrant, for example, because it is stronger than the activity of its native counterpart. Alternatively, an activity can be aberrant because it is weaker or absent relative to the activity of its native counterpart. An aberrant activity can also be a change in an activity. For example, an aberrant polypeptide can have altered substrate specificity. A cell can have an aberrant CYP2D6 P450 activity, e.g., due to overexpression or under expression of a gene encoding CYP2D6 P450, or due to expression of a CYP2D6 allelic variant that alters the sequence of the encoded P450 polypeptide;

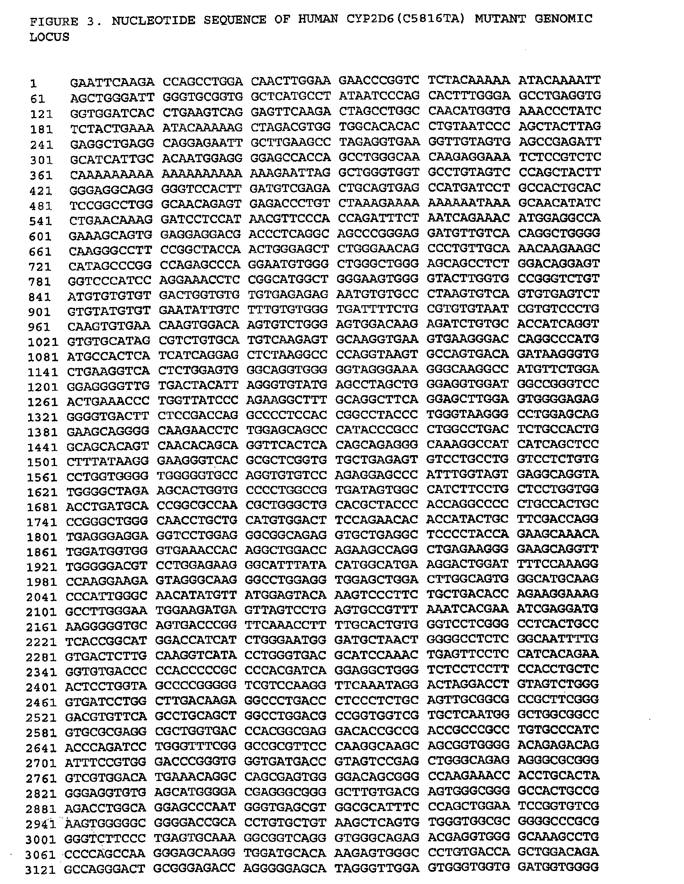

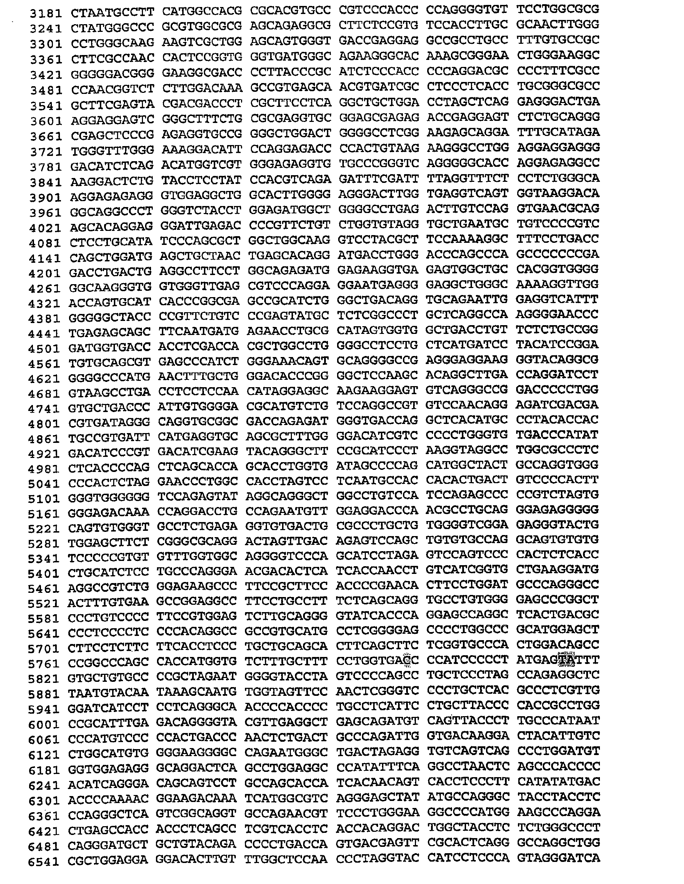

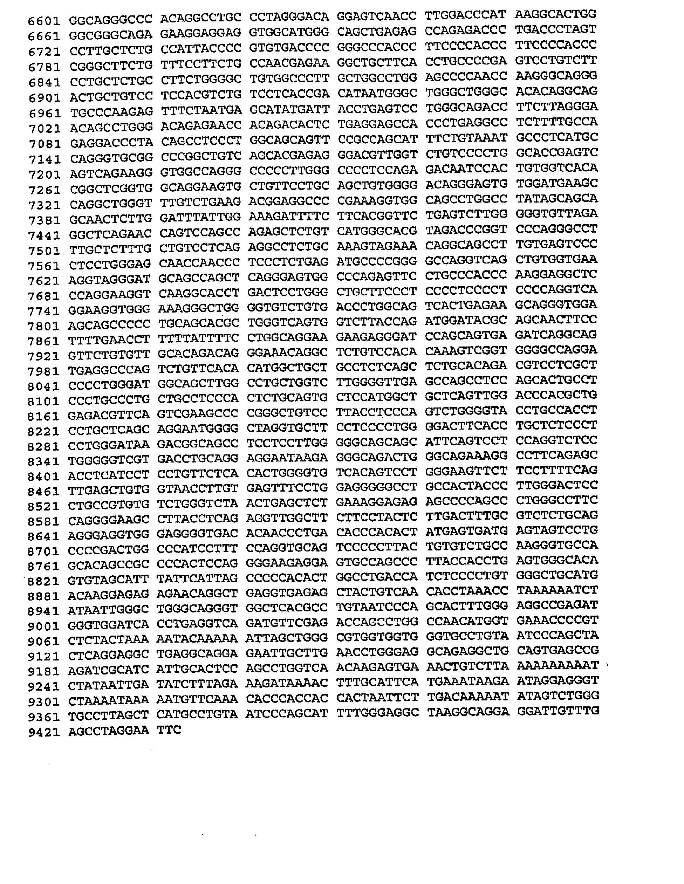

"allele" refers to the different sequence variants found at different polymorphic regions. For example, CYP2D6 P450 exon 9 has at least two different alleles (the wild type allele (see FIGURE 2) and the CYP2D6 C5816TA mutant allele (see FIGURE 3). A third allele of CYP2D6 P450 exon 9, consisting of an insertion/repetition of a 9 base sequence TCACCCGTG (SEQ ID No. 29), has also been reported in EP 0759476A1 ("A method of detecting human cytochrome P4502D6 (CYP2D6) gene polymorphism"). The sequence variants may be single or multiple base changes, including without limitation insertions, deletions, or substitutions, or may be a variable number of sequence repeats;

"amplification" in reference to nucleic acids encompasses essentially any method of generating many copies of a nucleic acid, either in single or double stranded form. Such methods include but are not limited to polymerase chain reaction (PCR) and replication of the nucleic acid in cells;

"antibody " refers to a binding agent including a whole antibody or a binding fragment thereof, which is specifically reactive with a wild type or mutant CYP2D6 P450 polypeptide. Antibodies can be fragmented using conventional techniques and the fragments screened for utility in the same manner as described above for whole antibodies. For example, F(ab)2 fragments can be generated by treating an antibody with pepsin. The resulting F(ab)2 fragment can be treated to reduce disulfide bridges to produce Fab fragments. The antibody of the present invention is further intended to include bispecific, single-chain, and chimeric and humanized molecules having affinity for a CYP2D6 P450 polypeptide conferred by at least one CDR region of the antibody; "bioactive portion of CYP2D6 P450" refers to a fragment of a full-length CYP2D6 P450, wherein the fragment specifically mimics or antagonizes at least one activity of a wild-type CYP2D6 P450;

"biological activity" or "bioactivity" or "activity" or "biological function," which are used interchangeably, for the purposes herein when applied to CYP2D6 P450 means an effector or antigenic function that is directly or indirectly performed by a CYP2D6 P450 (whether in its native or denatured conformation), or by any subsequence (fragment) thereof. A biological activity can include binding substrate, causing the transfer of lipids, effecting signal transduction from a receptor, modulation of gene expression or an antigenic effector function;

"cells", "host cells" or "recombinant host cells" are terms used interchangeably herein to refer not only to the particular subject cell, but to the progeny or potential progeny of such a cell. Because certain modifications may occur in succeeding generations due to either mutation or environmental influences, such progeny may not, in fact be identical to the parent cell, but is still included within the scope of the term as used herein;

"CYP2D6 P450" refers to the cytochrome P450 2D6 isozyme. The CYP2D6 P450 isozyme is also known as debrisoquine hydroxylase based upon a catalytic activity, is a monooygenase enzyme that catalyzes the oxidation and eventual elimination of a large number of pharmaceutical agents;

"CYP2D6 P450 agonist" refers to an agent that mimics, upregulates (potentiates or supplements) or otherwise increases a CYP2D6 P450 bioactivity. CYP2D6 P450 agonists may act on any of a variety of different levels, including regulation of CYP2D6 P450 gene expression, regulation of mRNA splicing mechanisms, stabilization of mRNA, or maturation CYP2D6 P450, or by affecting the biochemical activities of CYP2D6 P450;

"CYP2D6 P450 antagonist" refers to an agent that downregulates or otherwise decreases a CYP2D6 P450 bioactivity. CYP2D6 P450 agonists may act on any of a variety of different levels, including regulation of CYP2D6 P450 gene expression, regulation of mRNA splicing mechanisms, stabilization of mRNA, or maturation of CYP2D6 P450, or by affecting the biochemical activities of CYP2D6 P450;

"CYP2D6 P450 loci" include all the nucleic acid sequence at or near the CYP2D6 P450 gene, introns, exons and 5' and 3' untranslated regions. The GenBank Accession Nos. for the CYP2D6 P450 gene include M33388 (the CYP2D6 genomic sequence) and NM_000106 (the CYP2D6 cDNA sequence);

"CYP2D6 P450 functional mutation" refers to a mutation within or near the CYP2D6 P450 gene that results in an altered phenotype;

"CYP2D6 X (position #A) Y" refers to a particular allelic form of the CYP2D6 gene, wherein the nucleotide X of SEQ ID NO. 1 (GenBank M33388; FIGURE 2; ) present at position #A has been changed to nucleotide Y. For example, CYP2D6 C5816TA refers to a substitution at position 5816 of the CYP2D6 genomic sequence (and as shown in FIGURE 3; SEQ ID NO. 2). When a subject has two different CYP2D6 P450 alleles, the subject is said to be heterozygous, or to have the heterozygous state;

"CYP2D6 P450 polypeptide" and "CYP2D6 P450 protein" are intended to encompass polypeptides comprising the amino acid sequence encoded by the CYP2D6 P450 genomic DNA sequences or fragments thereof, and homologs thereof and include agonist and antagonist polypeptides;

"chimera," "mosaic," "chimeric mammal," and the like, refer to a transgenic mammal with a knock-out or knock-in construct in at least some of its genome-containing cells;

"control" or "control sample" refer to any sample appropriate to the detection technique employed. The control sample may contain the products of the allele detection technique employed or the material to be tested. Further, the controls may be positive or negative controls. By way of example, where the allele detection technique is PCR amplification, followed by size fractionation, the control sample may comprise DNA fragments of an appropriate size. Likewise, where the allele detection technique involves detection of a mutated protein, the control sample may comprise a sample of a mutant protein. However, it is preferred that the control sample comprises the material to be tested. For example, the controls may be a sample of genomic DNA or a cloned portion of the CYP2D6 P450 gene. However, where the sample to be tested is genomic DNA, the control sample is preferably a highly purified sample of genomic DNA;

"disorder associated allele" or "an allele associated with a disorder" refers to an allele whose presence in a subject indicates that the subject has or has an increased propensity for developing a particular disorder. An allele associated with the CYP2D6 C5816TA mutant polymorphic allele of the invention is the CYP2D6 G5799C polymorphism;

"disruption of the gene" and "targeted disruption" or any similar phrase refers to the site specific interruption of a DNA sequence so as to prevent expression of that gene in the cell as compared to the non-disrupted copy of the gene. The interruption may be caused by deletions, insertions or modifications to the gene, or any combination thereof;

"evolutionarily related to," with respect to amino acid sequences of CYP2D6 proteins, refers to both polypeptides having amino acid sequences which have arisen naturally, and also to mutational variants of human CYP2D6 polypeptides which are derived, for example, by combinatorial mutagenesis;

"haplotype" is intended to refer to a set of alleles that are inherited together as a group (are in linkage disequilibrium) at statistically significant levels (pcorr < 0.05). As used herein, the phrase "a CYP2D6 P450 haplotype" refers to a haplotype including CYP2D6 P450 loci;

"genetic deficiency for drug metabolism" refers to an altered level of drug metabolism in certain individuals, particularly, of drugs that are substrates of P450CYP2D6, when compared to the majority of the population. To illustrate, the majority of the population may be characterized as "extensive metabolizers" and exhibit normal activity of the CYP2D6 enzyme. However, genetic variation of this gene locus results in altered enzymatic activity, and some individuals possess slightly reduced activity (intermediate metabolizers) and some individuals lack enzyme activity (poor metabolizers).

"homology" or "identity" or "similarity" refers to sequence similarity between two peptides or between two nucleic acid molecules. Homology can be determined by comparing a position in each sequence that may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of amino acids, i.e. structurally related, at positions shared by the amino acid sequences. An "unrelated" or "non-homologous" sequence shares less than 40% identity, though preferably less than 25 % identity, with one of the sequences of the present invention;

"increased risk" refers to a higher frequency of occurrence of the disease or disorder in an individual in comparison to the frequency of occurrence of the disease or disorder in a population. A factor identified to be associated with increased risk is termed a "risk factor." Carrying a particular polymorphic allele is a risk factor for a particular condition such as drug sensitivity;

"interact" is meant to include detectable relationships or associations (e.g., biochemical interactions) between molecules, such as interactions between protein-protein, protein-nucleic acid, nucleic acid-nucleic acid and protein-small molecule or nucleic acid-small molecule in nature;

"isolated" with respect to nucleic acids, such as DNA or RNA, refers to molecules separated from other DNAs, or RNAs, respectively that are present in the natural source of the macromolecule. For example, an isolated nucleic acid encoding CYP2D6 P450 preferably includes no more than 10 kilobases (kb) of nucleic acid sequence which naturally immediately flanks the CYP2D6 P450 gene in genomic DNA, more preferably no more than 5 kb of such naturally occurring flanking sequences, and most preferably less than 1.5 kb of such naturally occurring flanking sequence. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments, which are not naturally occurring as fragments and would not be found in the natural state. "Isolated" also refers to polypeptides that are isolated from other cellular proteins and is meant to encompass both purified and recombinant polypeptides;

"knock-in" transgenic animal refers to an animal that has had a modified gene introduced into its genome and the modified gene can be of exogenous or endogenous origin;

"knock-out" transgenic animal refers to an animal in which there is partial or complete suppression of the expression of an endogenous gene (e.g., based on deletion of at least a portion of the gene, replacement of at least a portion of the gene with a second sequence, introduction of stop codons, the mutation of bases encoding critical amino acids, or the removal of an intron junction, etc.);

"knock-out construct" refers to a nucleic acid sequence that can be used to decrease or suppress expression of a protein encoded by endogenous DNA sequences in a cell. In one example, the knock-out construct is comprised of a gene, such as the CYP2D6 P450 gene, with a deletion in a critical portion of the gene so that active protein cannot be expressed therefrom. Alternatively, a number of termination codons can be added to the native gene to cause early termination of the protein or an intron junction can be inactivated. In a typical knock-out construct, some portion of the gene is replaced with a selectable marker (such as the neo gene) so that the gene can be represented as follows: CYP2D6 P450 5'/neo/ CYP2D6 P450 3', where 5' and 3', refer to genomic or cDNA sequences which are, respectively, upstream and downstream relative to a portion of the CYP2D6 P450 gene and where neo refers to a neomycin resistance gene. In another knock-out construct, a second selectable marker is added in a flanking position so that the gene can be represented as: CYP2D6 P450 5'/neo/CYP2D6 P450 3'/TK, where TK is a thymidine kinase gene which can be added to either the 5' or 3' sequence of the preceding construct and which further can be selected against (i.e., is a negative selectable marker) in appropriate media. This two-marker construct allows the selection of homologous recombination events, which removes the flanking TK marker, from non-homologous recombination events which typically retain the TK sequences. The gene deletion and/or replacement can be from the exons, introns, especially intron junctions, and/or the regulatory regions such as promoters;

"linkage disequilibrium" refers to co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in "linkage disequilibrium". The cause of linkage disequilibrium is often unclear. It can be due to selection for certain allele combinations or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in the specific chromosomal region. When referring to allelic patterns that are comprised of more than one allele, a first allelic pattern is in linkage disequilibrium with a second allelic pattern if all the alleles that comprise the first allelic pattern are in linkage disequilibrium with at least one of the alleles of the second allelic pattern;

"marker" refers to a sequence in the genome that is known to vary among individuals.

"modulate" refers to the ability of a substance to affect bioactivity. When applied to a CYP2D6 P450 bioactivity, an agonist or antagonist can modulate bioactivity for example by agonizing or antagonizing a CYP2D6 P450 synthesis, or monooxygenase activity;

"non-human animal" includes mammals such as rodents, non-human primates, sheep, dogs, cows, goats, etc., amphibians, such as members of the Xenopus genus, and transgenic avians (e.g., chickens, birds, etc.). The term "chimeric animal" is used herein to refer to animals in which the recombinant gene is found, or in which the recombinant gene is expressed in some but not all cells of the animal. The term "tissue-specific chimeric animal" indicates that one of the recombinant CYP2D6 P450 genes is present and/or expressed or disrupted in some tissues but not others. The term "non-human mammal" refers to any member of the class Mammalia, except for humans;

"nucleic acid" refers to polynucleotides or oligonucleotides such as deoxyribonucleic acid (DNA), and, where appropriate, ribonucleic acid (RNA). The term should also be understood to include, as equivalents, analogs of either RNA or DNA made from nucleotide analogs (e.g., peptide nucleic acids) and as applicable to the embodiment being described, single (sense or antisense) and double-stranded polynucleotides;

"nucleotide sequence complementary to the nucleotide sequence set forth in SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a noncoding strand. When referring to double stranded nucleic acids, the complement of a nucleic acid having SEQ ID NO. x refers to the complementary strand of the strand having SEQ ID NO. x or to any nucleic acid having the nucleotide sequence of the complementary strand of SEQ ID NO. x. When referring to a single stranded nucleic acid having the nucleotide sequence SEQ ID NO. x, the complement of this nucleic acid is a nucleic acid having a nucleotide sequence which is complementary to that of SEQ ID NO. x. The nucleotide sequences and complementary sequences thereof are always given in the 5' to 3' direction;

"percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences; other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases; Databases with individual sequences are described in Methods in Enzymology, ed. Doolittle, *supra.* Databases include Genbank, EMBL, and DNA Database of Japan (DDBJ); preferred nucleic acids have a sequence at least 70%, and more preferably 80% identical and more preferably 90% and even more preferably at least 95% identical to an nucleic acid sequence of a sequence shown in one of SEQ ID NOs. of the invention. Nucleic acids at least 90%, more preferably 95%, and most preferably at least about 98-99% identical with a nucleic sequence represented in one of SEQ ID NOs: 1-4 are of course also within the scope of the invention. In preferred embodiments, the nucleic acid is mammalian. In comparing a new nucleic acid with known sequences, several alignment tools are available. Examples include PileUp, which creates a multiple sequence alignment, and is described in Feng et al., *J. Mol. Evol.* (1987) 25:351-360. Another method, GAP, uses the alignment method of Needleman et al., *J. Mol. Biol.* (1970) 48:443-453. GAP is best suited for global alignment of sequences. A third method, BestFit, functions by inserting gaps to maximize the number of matches using the local homology algorithm of Smith and Waterman, *Adv. Appl. Math.* (1981) 2:482-489.

"polymorphism" refers to the coexistence of more than one form of a gene, or portion (e.g., allelic variant) thereof, in a given population. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long;

"promoter" means a DNA sequence that regulates expression of a selected DNA sequence operably linked to the promoter, and which effects expression of the selected DNA sequence in cells. The term encompasses "tissue specific" promoters, i.e. promoters, which effect expression of the selected DNA sequence only in specific cells (e.g. cells of a specific tissue). The term also covers so-called "leaky" promoters, which regulate expression of a selected DNA primarily in one tissue, but cause expression in other tissues as well. The term also encompasses non-tissue specific promoters and promoters that constitutively express or that are inducible (i.e. expression levels can be controlled);

"protein", "polypeptide" and "peptide" are used interchangeably herein when referring to a gene product;

"recombinant protein" refers to a polypeptide of the present invention which is produced by recombinant DNA techniques, wherein generally, DNA encoding a polypeptide is inserted into a suitable expression vector which is in turn used to transform a host cell to produce the heterologous protein. Moreover, the phrase "derived from", with respect to a recombinant gene, is meant to include within the meaning of "recombinant protein" those proteins having an amino acid sequence of a native polypeptide, or an amino acid sequence similar thereto which is generated by mutations including substitutions and deletions (including truncation) of a naturally occurring form of the polypeptide;

"specifically hybridizes" or "specifically detects" refers to the ability of a nucleic acid molecule of the invention to hybridize to at least approximately 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 100, 150, 200, 300, 350, or 400 consecutive nucleotides of a vertebrate, preferably a CYP2D6 gene. In certain instances the invention provides nucleic acids which hybridize under stringent conditions to a nucleic acid represented by SEQ ID Nos. 1, 2, 3, or 4 or complement thereof or the nucleic acids. Appropriate stringency conditions which promote DNA hybridization, for example, 6.0 x sodium chloride/sodium citrate (SSC) at about 45° C, followed by a wash of 2.0 x SSC at 50°C, are known to those skilled in the art or can be found in Current Protocols in Molecular Biology, John Wiley & Sons, N.Y. (1989), 6.3.1-6.3.6 or in Molecular Cloning: A Laboratory Manual, Cold Spring Harbor Press (1989). For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22°C, to high stringency conditions at about 65°C. Both temperature and salt may be varied, or temperature and salt concentration may be held constant while the other variable is changed. In a preferred embodiment, an htrb nucleic acid of the present invention will bind to one of SEQ ID NOs. 1, 2, 3, or 4 or complement thereof under moderately stringent conditions, for example at about 2.0 x SSC and about 40° C. In a particularly preferred embodiment, a CYP2D6 nucleic acid of the present invention will bind to one of SEQ ID NOs. 1, 2, 3, or 4, or complement thereof, under high stringency conditions. In another particularly preferred embodiment, a CYP2D6 nucleic acid sequence of the present invention will bind to one of SEQ ID NO. 1, 2, 3, or 4 which correspond to the CYP2D6 cDNA, preferably open reading frame (ORF) nucleic acid sequences, under high stringency conditions;

"susceptibility" to disease or condition or any similar phrase, means that certain alleles are hereby discovered to be associated with or predictive of a subject's incidence of developing a particular disease or condition (particularly a sensitivity to drugs). The alleles are thus over-represented in frequency in individuals with drug sensitivity as compared to normal individuals. These alleles are understood to relate to the drug sensitivity condition;

"small molecule" as used herein, is meant to refer to a composition, which preferably has a molecular weight of less than about 5kD and most preferably less than about 4kD. Small molecules include nucleic acids, peptides, peptidomimetics, carbohydrates, lipids and other organic and inorganic molecules;

"specifically hybridizes" or "specifically detects" refers to the ability of a nucleic acid molecule to hybridize to at least approximately 6 consecutive nucleotides of a sample nucleic acid.

"transcriptional regulatory sequence" is a generic term used throughout the specification to refer to DNA sequences, such as initiation signals, enhancers, and promoters, which induce or control transcription of protein coding sequences with which they are operably linked;

"transfection" means the introduction of a nucleic acid, e.g., via an expression vector, into a recipient cell by nucleic acid-mediated gene transfer.

"transformation" refers to a process in which a cell's genotype is changed as a result of the cellular uptake of exogenous DNA or RNA, and, for example, the transformed cell expresses a recombinant form of a polypeptide or, in the case of anti-sense expression from the transferred gene, the expression of a naturally-occurring form of a polypeptide is disrupted;

"transgene" means a nucleic acid sequence (encoding, e.g., one of the CYP2D6 P450 polypeptides, or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid;

"transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. This molecule may be integrated within a chromosome, or it may be extrachromosomally replicating DNA. In the typical transgenic animals described herein, the transgene causes cells to express a recombinant form of one of a CYP2D6 P450 polypeptide, e.g., either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination or antisense techniques. The term is intended to include all progeny generations. Thus, the founder animal and all F1, F2, F3, and so on, progeny thereof are included;

"treating," "treat" or "treatment" includes, *inter alia,* preventative (e.g., prophylactic), palliative and curative treatment, including, for example, ameliorating at least one symptom of a disease or at least one abnormality associated with a condition or disorder, e.g., decreased or over expression of a peptide of the invention. Treating a cardiovascular disorder can take place, for example, by administering a cardiovascular disorder therapeutic. Treating a cardiovascular disorder can also take place, for example, by modifying risk factors that are related to the cardiovascular disorder;

"vector" refers to a nucleic acid molecule, which is capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto;

"wild-type allele" or "normal allele" refer to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes. In general a "wild-type allele" is the most common allele in a population; and

"xenobiotic" refers to any substance that does not occur naturally but will affect living systems.

The cytochrome P450 family of enzymes is primarily responsible for the metabolism of xenobiotics such as drugs, carcinogens and environmental chemicals, as well as several classes of endobiotics such as steroids and prostaglandins. These generally lipophilic compounds must be metabolized to more polar products before they can be excreted. This metabolic process, which is primarily catalyzed by hepatic enzymes, consists of a sequence of enzymatic steps that includes oxidation by a member of the cytochrome P450-dependent monooxygenases (phase I metabolism), followed by conjugation involving sulfation, glucoronidation or acetylation (phase II metabolism).

Many cytochrome P450 genes which encodes products involved in phase I metabolism are known. There are multiple forms of these P450s, and each of these individual forms exhibit degrees of specificity towards individual chemicals in the above classes of compounds. In some cases, a substrate, whether it be a drug or a carcinogen, is metabolized by more then one of the cytochrome P450s. Genetic polymorphisms of cytochrome P450s result in phenotypically-distinct subpopulations that differ in their ability to metabolize particular drugs and other chemical compounds. As those skilled in the art will understand, these phenotypic distinctions have important implications for the selection of drugs for any given patient.

For example, some individuals may have a defect in an enzyme required for detoxification of a particular drug, while some individuals may lack an enzyme required for conversion of the drug to a metabolically active form. Further, individuals lacking a biotransformation enzyme are often susceptible to cancers from environmental chemicals due to inability to detoxify the chemicals (see Eichelbaum et al., (1992) *Toxicology Letters* 64165: 155-122). Accordingly, those skilled in the art will appreciate that it is advantageous to identify individuals who are deficient in a particular P450 enzyme. Cytochrome P450 2D6 (or P450IID6), also known as debrisoquine hydroxylase, is the best characterized polymorphic P450 in the human population (see e.g. Gonzalez et al. (1998)). The cytochrome P450 2D6 gene represents a major Phase I drug metabolizing enzyme and is involved in the metabolism of numerous drugs. While CYP2D6 contributes only approximately 1.5% of the P450 protein present in human liver, it is responsible for approximately 24% of P450 drug metabolism activity (see Wolf & Smith (2000) *Brit Med Bull* 55: 366-386). The CYP2D6-encoded P450 appears to be particularly important in the metabolism of drugs targeted to the central nervous system. CYP2D6 is found in the brain (Gilham et al. (1997) *Xenobiotica* 27: 111-25) and may, therefore, have evolved to protect cells from environmental neurotoxins (Smith et al. (1992) *Lancet* 339: 1365-72).

Genetic variation of this gene locus results in various altered enzymatic activities of this gene with the majority of individuals possessing normal activity (extensive metabolizers), some individuals possessing slightly reduced activity (intermediate metabolizers), and some individuals with increased enzyme activity, in part due to gene duplications (rapid metabolizers). Individuals who lack enzyme activity, due to inactivating mutations in both copies of the CYP2D6 gene, are unable to metabolize drugs that require the CYP2D6 enzyme and are referred to as CYP2D6 poor metabolizers. A number of mutations in the CYP2D6 gene that result in poor or intermediate metabolizer phenotypes, depending upon whether only one or both copies of the CYP2D6 gene are affected by mutation, have already been described (see, for example, U.S. Patent No. 5,648,482).

Genetic screening (or genotyping), involves testing to determine if a patient has mutations (or alleles or polymorphisms) that either cause a disease state, contribute to a disease state (i.e., are a risk factor associated with a disease state), are "linked" to the mutation causing a disease state, or are "linked" to the mutation which contributes to the disease state. Linkage refers to the phenomenon wherein DNA sequences that are close together in the genome have a tendency to be inherited together. Two sequences may be linked because of some selective advantage of co-inheritance. More typically, however, two polymorphic sequences are co-inherited because of the relative infrequency with which meiotic recombination events occur within the region between the two polymorphisms. The co-inherited polymorphic alleles are said to be in linkage disequilibrium with one another because, in a given human population, they tend to either both occur together or else not occur at all in any particular member of the population.

The present invention provides a novel human CYP2D6 polymorphic variation, i.e., at position 5816 of the CYP2D6 gene corresponding to a substitution of the sequence "TA" for the "C" at position 5816 of the CYP2D6 genomic sequence (GenBank Accession No. M33388; SEQ ID NO. 1 shown in FIGURE 2) and at position 1474 of the CYP2D6 cDNA sequence (GenBank Accession No. NM_000106; SEQ ID NO. 3 shown in FIGURE 4). The cytochrome P450 2D6 gene represents a major phase I drug metabolizing enzyme and is involved in the metabolism of numerous drugs. The present invention provides a novel mutation in the CYP2D6 gene that is an insertion of two nucleotides in exon 9 of the gene. This mutation results in a frameshift within the critical carboxy-terminal region of the gene which, in the wild-type enzyme, is required for catalytic activity. In addition, an individual who possesses this C5816TA CYP2D6 gene mutation was found to further carry a nucleotide change of G to C at position 5799 of the CYP2D6 genomic sequence (GenBank Accession No. M33388; SEQ ID NO. 1 shown in FIGURE 2) and at position 1457 of the CYP2D6 cDNA sequence (GenBank Accession No. NM_000106; SEQ ID NO. 3 shown in FIGURE 4). Analysis of the drug metabolizing phenotype of the individual who possesses this gene mutation, in combination with another CYP2D6 PM allele, revealed the individual to possess a PM phenotype indicating that this novel C5816TA allele also results in a non-functional CYP2D6 allele. Accordingly, the present invention provides methods and reagents for predicting susceptibility to diminished metabolism of drugs by detecting a cytochrome P450 CYP2D6 polymorphism of the invention.

The present invention provides CYP2D6 genomic and cDNA nucleic acids, homologs thereof, and portions thereof. Preferred nucleic acids have a sequence at least about 60%, 61%, 62%, 63%, 64%, 65%, 66%, 67%, 68%, 69%, 70%, 71%, 72%, 73%, 74%, 75%, 76%, 77%, 78%, 79%, 80%, and more preferably 85% homologous and more preferably 90% and more preferably 95% and even more preferably at least 99% homologous with a nucleotide sequence of a CYP2D6 gene, e.g., such as a sequence shown in one of SEQ ID NOs: 1, 2, 3, or 4 or complement thereof of the CYP2D6 nucleic acids having the GenBank Accession Nos. M33388 (genomic CYP2D6 locus) or NM_000106 (CYP2D6 cDNA sequence). Nucleic acids at least 90%, more preferably 95%, and most preferably at least about 98-99% identical with a nucleic sequence represented in one of SEQ ID NOs. 1, 2, 3, or 4, or complement thereof, are of course also within the scope of the invention. In a preferred embodiment, the nucleic acid is human, preferably mammalian and in a particularly preferred embodiment, includes all or a portion of the nucleotide sequence corresponding to the coding region of the CYP2D6 P450 polypeptide, or mutant variant thereof, such as the nucleic acid set forth in SEQ ID NOs. 1-4.

The invention also provides isolated nucleic acids comprising a nucleotide sequence encoding CYP2D6 polypeptides, variants and/or equivalents of such nucleic acids. The term equivalent is understood to include nucleotide sequences encoding functionally equivalent CYP2D6 polypeptides or functionally equivalent peptides having an activity of an CYP2D6 protein such as described herein. Those skilled in the art will understand that equivalent nucleotide sequences will include sequences that differ by one or more nucleotide substitution, addition or deletion, such as allelic variants; and will, therefore, include sequences that differ from the nucleotide sequence of the *CYP2D6* gene shown in SEQ ID NOs. 1, 2, 3, or 4 due to the degeneracy of the genetic code.

In a preferred embodiment, the nucleic acids are vertebrate CYP2D6 nucleic acids. In a particularly preferred embodiment, the nucleic acids are mammalian CYP2D6 nucleic acids. Regardless of species, particularly preferred CYP2D6 nucleic acids encode polypeptides that are at least 60%, 65%, 70%, 72%, 74%, 76%, 78%, 80%, 90%, or 95% similar or identical to an amino acid sequence of a vertebrate CYP2D6 protein. In one embodiment, the nucleic acid is a cDNA encoding a polypeptide having at least one bio-activity of the subject CYP2D6 polypeptide. Preferably, the nucleic acid includes all or a portion of the nucleotide sequence corresponding to the nucleic acid of SEQ ID NOs. 1, 2, 3 or 4.

Still other preferred nucleic acids of the present invention encode CYP2D6 polypeptides which are comprised of at least 2, 5, 10, 25, 50, 100, 150 or 200 amino acid residues. For example, such nucleic acids can comprise about 50, 60, 70, 80, 90, or 100 base pairs. Also within the scope of the invention are nucleic acid molecules for use as probes/primer or antisense molecules (i.e. noncoding nucleic acid molecules), which can comprise at least about 6, 12, 20, 30, 50, 60, 70, 80, 90 or 100 base pairs in length.

Another aspect of the invention provides a nucleic acid which hybridizes under stringent conditions to a nucleic acid represented by SEQ ID NOs. 1, 2, 3, or 4 or complement thereof. Appropriate stringency conditions which promote DNA hybridization, for example, 6.0 x SSC at about 45° C, followed by a wash of 2.0 x SSC at 50°C, are known to those skilled in the art or can be found in Current Protocols in Molecular Biology, John Wiley & Sons, N.Y. (1989), 6.3.1-6.3.6 or in Molecular Cloning: A Laboratory Manual, Cold Spring Harbor Press (1989). For example, the salt concentration in the wash step can be selected from a low stringency of about 2.0 x SSC at 50°C to a high stringency of about 0.2 x SSC at 50°C. In addition, the temperature in the wash step can be increased from low stringency conditions at room temperature, about 22°C, to high stringency conditions at about 65°C. Both temperature and salt may be varied, or temperature and salt concentration may be held constant while the other variable is changed. In a preferred embodiment, an CYP2D6 nucleic acid of the present invention will bind to one of SEQ ID NOs. 1, 2, 3, or 4 or complement thereof under moderately stringent conditions, for example at about 2.0 x SSC and about 40° C. In a particularly preferred embodiment, a CYP2D6 nucleic acid of the present invention will bind to one of SEQ ID NOs. 1, 2, 3, or 4 or complement thereof under high stringency conditions. In another particularly preferred embodiment, a CYP2D6 nucleic acid sequence of the present invention will bind to one of SEQ ID NOs. 3, which correspond to CYP2D6 ORF nucleic acid sequences, under high stringency conditions.

Nucleic acids having a sequence that differs from the nucleotide sequences shown in one of SEQ ID NOs. of the present invention, or complement thereof, due to degeneracy in the genetic code are also within the scope of the invention. Such nucleic acids encode functionally equivalent peptides (i.e., peptides having a biological activity of a CYP2D6 polypeptide) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a CYP2D6 polypeptide. However, it is expected that DNA sequence polymorphisms that do lead to changes in the amino acid sequences of the subject CYP2D6 polypeptides will exist among mammals. One skilled in the art will appreciate that these variations in one or more nucleotides (e.g., up to about 3-5% of the nucleotides) of the nucleic acids encoding polypeptides having an activity of an CYP2D6 polypeptide may exist among individuals of a given species due to natural allelic variation.

The nucleotide sequences determined from the cloning of CYP2D6 genes from mammalian organisms will further allow for the generation of probes and primers designed for detecting a CYP2D6 C5816TA allelic variant by any means such as by detection of an amplification product using a C5816TA allele-specific primer or by detecting the presence of the CYP2D6 C5816TA allelic variant using an ASO detector probe. Those skilled in the art based upon the present description will understand how to design other probes including those for use in restriction fragment length polymorphism (RFLP) analyses to detect any of a number of restriction sites which are altered by the C5816TA mutation. TABLE 1 below is a listing of exemplary oligonucleotide sequences (or subsequences) for use in the present invention.

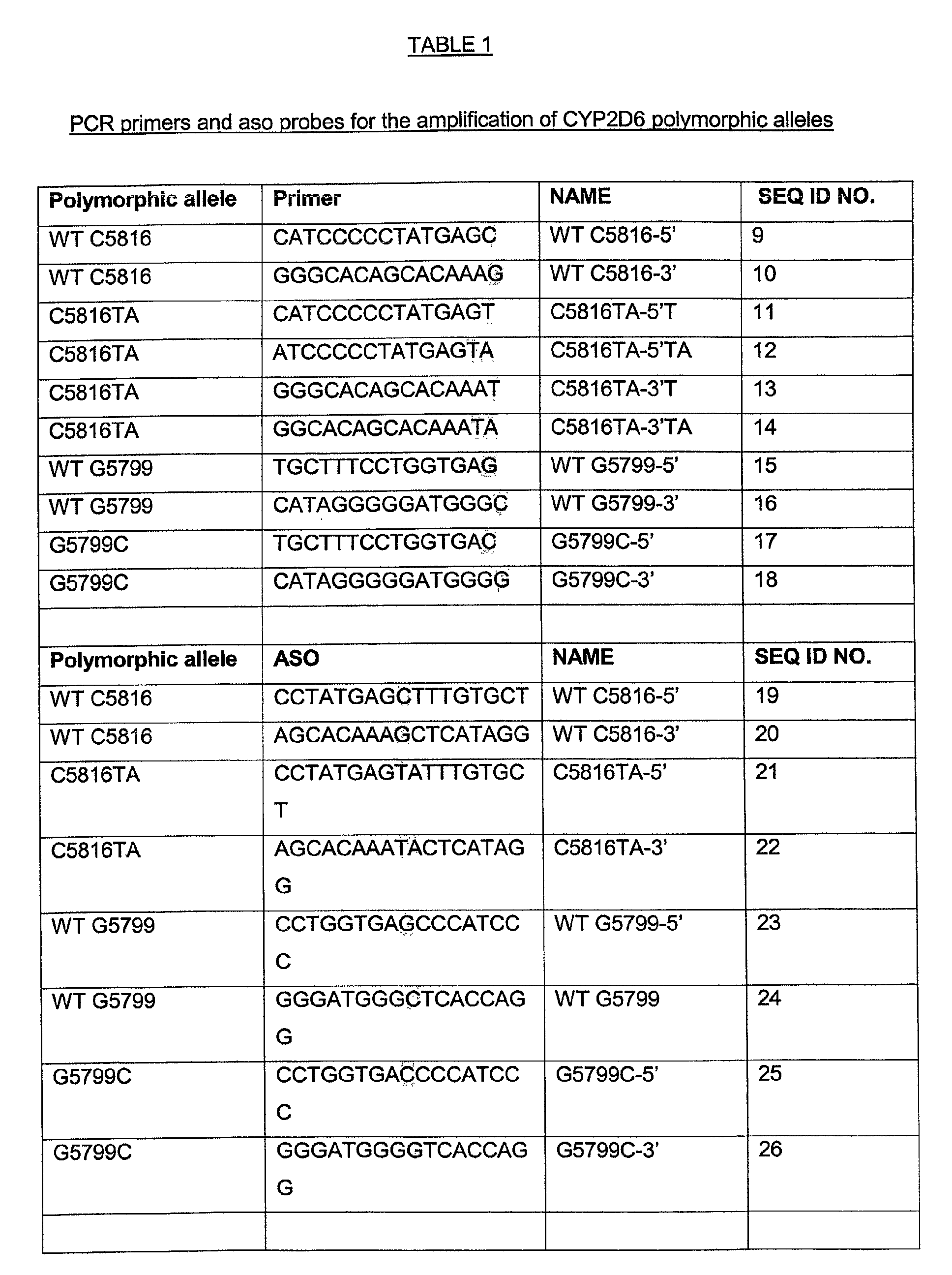

In a preferred embodiment, the CYP2D6 primers are designed so as to optimize specificity and avoid secondary structures which affect the efficiency of priming. Optimized PCR primers of the present invention are designed so that "upstream" and "downstream" primers have approximately equal melting temperatures such as can be estimated using the formulae: Tₘ = 81.5° C - 16.6(log₁₀[Na⁺]) + 0.41 (%G+C) - 0.63 (%formamide) - (600/length); or Tₘ(° C) = 2(A/T) + 4(G/C). Optimized CYP2D6 primers may also be designed by using various programs, such as "Primer3" provided by the Whitehead Institute for Biomedical Research at http://www-genome.wi.mit.edu/cgi-bin/primer/primer3.cgi.

In preferred embodiments, the CYP2D6 probes and primers can be used to detect CYP2D6 locus polymorphisms which occur within and surrounding the CYP2D6 gene sequence, in particular the C5816TA and/or the G5799C wild-type and mutant polymorphic alleles. Genetic variations within the CYP2D6 locus are associated with sensitivity to drugs metabolized by the CYP2D6 P450 monooxygenase. Accordingly, the invention provides probes and primers for CYP2D6 locus polymorphisms, including polymorphisms associated with the human and mouse CYP2D6 gene. PCR primers of the invention include those which flank an CYP2D6 human polymorphism and allow amplification and analysis of this region of the genome. Analysis of polymorphic allele identity may be conducted, for example, by direct sequencing or by the use of allele-specific capture probes or by the use of molecular beacon probes. Alternatively, the polymorphic allele may allow for direct detection by the creation or elimination of a restriction endonuclease recognition site(s) within the PCR product or after an appropriate sequence modification is designed into at least one of the primers such that the altered sequence of the primer, when incorporated into the PCR product resulting from amplification of a specific CYP2D6 polymorphic allele, creates a unique restriction site in combination with at least one allele but not with at least one other allele of that polymorphism. CYP2D6 polymorphisms corresponding to variable number of tandem repeat (VNTR) polymorphisms may be detected by the electrophoretic mobility and hence size of a PCR product obtained using primers which flank the VNTR. Still other CYP2D6 polymorphisms corresponding to RFLPs may be detected directly by the mobility of bands on a Southern blot using appropriate CYP2D6 locus probes and genomic DNA or cDNA obtained from an appropriate sample organism such as a human or a non-human animal.

Likewise, probes based on the subject CYP2D6 sequences can be used to detect transcripts or genomic sequences encoding the same or homologous proteins, for use, e.g, in prognostic or diagnostic assays (further described below). The present invention provides probes which are common to alternatively-spliced variants of the CYP2D6 transcript, such as those corresponding to at least 12 consecutive nucleotides complementary to a sequence found in any of SEQ ID NOs. of the present invention. In addition, the present nvention provides probes which hybridize specifically to alternatively spliced forms of the CYP2D6 transcript. As those skilled in the art will appreciate based upon the present description, any suitable probes and primers can be prepared and modified, e.g., as previously described herein for other types of nucleic acids.

In one embodiment, the present invention provides methods for determining whether an individual has a genetic deficiency for metabolizing certain agents, e.g., has a poor metabolizer genetype by detecting the presence of a cytochrome P450 CYP2D6 gene C5816TA polymorphism in a genomic DNA or cDNA sample from the individual. Any suitable methods for detecting the presence of this polymorphism are included within the scope of the instant invention; however, in a particularly preferred embodiment of such methods, the method includes the step of an initial amplification, preferably by PCR, of a segment of the CYP2D6 gene which includes the 5816 polymorphic locus, and the subsequent detection of an amplification product which includes the C5816TA sequence change. In a preferred embodiment, such amplification is achieved with allele specific oligonucleotide primers having 3' terminal nucleotide sequence which correspond to the wild-type 5816 nucleotide sequence (i.e. "C"), or complement thereof, or at least one nucleotide of the mutant 5816-5817 TA sequence. As those skilled in the art will appreciate, in general, such allele-specific amplification primers fail to produce an amplification product unless the allele which they are specific for is present in the patient genomic or cDNA sample.

Suitable C5816TA- specific amplification primers comprise a sequence of at least 10 consecutive nucleotides of SEQ ID NO. 2 or SEQ ID NO. 4, or complement thereof, and further feature a 3' terminal nucleotide which is the T at position 5816 of SEQ ID NO. 2, the T at position 1474 of SEQ ID NO. 4, the A at position 5817 of SEQ ID NO. 2, or the A at position 1475 of SEQ ID NO. 4. Particularly preferred C5816TA- specific amplification primers of the invention include, for example, the sequences CATCCCCCTATGAGT (SEQ ID NO. 11), ATCCCCCTATGAGTA (SEQ ID NO. 12), GGGCACAGCACAAAT (SEQ ID NO. 13), or GGCACAGCACAAATA (SEQ ID NO. 14).

When such C5816TA allele-specific amplification primers are utilized, the presence of the C5816TA polymorphism in a patient's genomic or cDNA sample is indicated by the production of an amplification product with the C5816TA allele specific primer; however, other suitable methods of detecting the C5816TA polymorphic variation are also included in the present invention. For example, when the amplification primers are chosen so that an amplification product is obtained from either the wild-type or C5816TA mutant CYP2D6 locus, an allele-specific oligonucleotide (ASO) detector that includes the TA sequence at position 5816-5817 of SEQ ID NO. 2 may be used to detect the C5816TA mutation. Preferred ASO detector oligonucleotides of the invention include: CCTATGAGTATTTGTGCT (SEQ ID NO. 21) and AGCACAAATACTCATAGG (SEQ ID NO. 22). Alternatively, detection of the C5816TA allelic variant may be achieved by a restriction endonuclease analysis such as by RFLP analysis (i.e. a Southern blot following restriction digestion of an unamplified genomic or cDNA sample) or by restriction of an appropriate amplification product. This aspect of the invention is possible because the CYP2D6 C5816TA mutation destroys an Alul and a CviJl site which is present in the wild-type CYP2D6 sequence at this position. In addition, a second alteration in susceptibility to restriction endonuclease digestion is caused by a G5799C mutation which was found associated with the C5816TA mutation in a patient sample. In particular, the presence of the G5799C allelic variant of CYP2D6 exon 9 is indicated by the loss of Ban II, CviJI, or Bsp1286I restriction sites which are present in the corresponding position of a wild type CYP2D6 DNA.

Another embodiment of the present invention provides primers capable of amplifying the C5816TA allelic variant. Generally, such primers include a sequence of at least 10 consecutive nucleotides of SEQ ID NO. 2 or SEQ ID NO. 4, or complement thereof, and further possess a 3' terminal nucleotide that is C5816TA allele-specific such as the T at position 5816 of SEQ ID NO. 2, the T at position 1474 of SEQ ID NO. 4, the A at position 5817 of SEQ ID NO. 2, or the A at position 1475 of SEQ ID NO. 4. Particularly preferred mutant allele-specific primers feature a 3' sequence such as CATCCCCCTATGAGT (SEQ ID NO. 11), ATCCCCCTATGAGTA (SEQ ID NO. 12), GGGCACAGCACAAAT (SEQ ID NO. 13), or GGCACAGCACAAATA (SEQ ID NO. 14). Other allele specific oligonucleotide for the detection of the C5816TA allelic variant include a sequence of at least 10 consecutive nucleotides of SEQ ID NO. 2 or SEQ ID NO. 4, or complement thereof, and further feature the nucleotide pair TA at position 5816-5817 of SEQ ID NO. 2 and position 1474-1475 of SEQ ID NO. 4, or complement thereof. Preferred allele specific oligonucleotides of this type include, for example, sequences CCTATGAGTATTTGTGCT (SEQ ID NO. 21) or AGCACAAATACTCATAGG (SEQ ID NO. 22).

The present invention also provides methods for determining whether an individual is susceptible to being a PM of drugs by detecting the presence of a G5799C sequence change which was found associated with the cytochrome P450 CYP2D6 gene C5816TA polymorphic change in a patient sample. In this aspect of the invention, a PM phenotype resulting from the C5816TA P450 2D6-inactivating mutation is inferred from the presence of the closely linked G5799C mutation. A further application of this principle may be employed to determine an entire C5816TA haplotype of polymorphisms associated with the C5816TA inactivating mutation. Primers of the present invention which are capable of amplifying the G5799C allelic variant of CYP2D6 exon 9 preferably include a 3'oligonucleotide sequence such as TGCTTTCCTGGTGAC (SEQ ID NO. 17) or CATAGGGGGATGGGG (SEQ ID NO. 18). Detection of amplified DNA that codes for the G5799C allelic variant of CYP2D6 exon 9, can also be achieved using allele specific oligonucleotides such as CCTGGTGACCCCATCCC (SEQ ID NO. 25), or GGGATGGGGTCACCAGG (SEQ ID NO. 26).

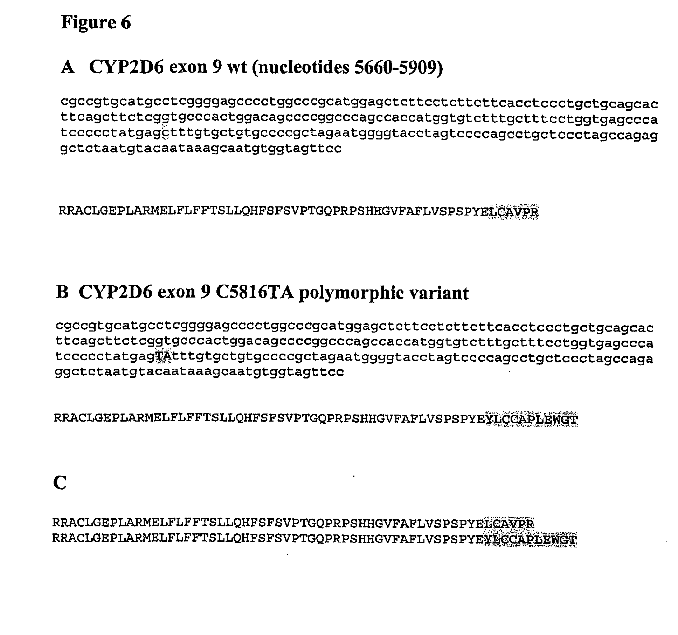

In yet another embodiment, the present invention provides protein-based methods for detecting the C5816TA PM polymorphism. In particular, the C5816TA mutation results in a frame shift in the critical carboxy-terminal domain of this P450 open reading frame. The frame-shift results in the production of a mutant polypeptide with an altered carboxy-terminus (i.e., YLCCAPLEWGT (SEQ ID NO. 27) in place of the normal CYP2D6 carboxy-terminal sequence LCAVPR (SEQ ID NO. 28), see FIGURE 6). Accordingly, the presence of a stable mutant CYP2D6 C5816TA polypeptide can be detected in a patient protein sample by the use of an appropriate antibody, such as a monoclonal antibody that recognizes an epitope of the YLCCAPLEWGT mutant carboxy-terminal sequence, prepared based upon the present description.

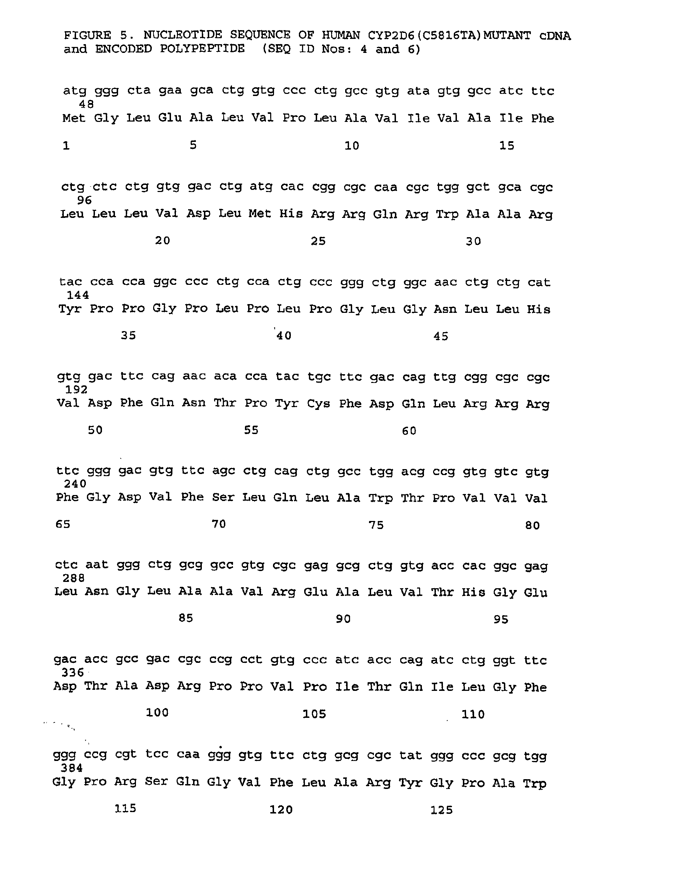

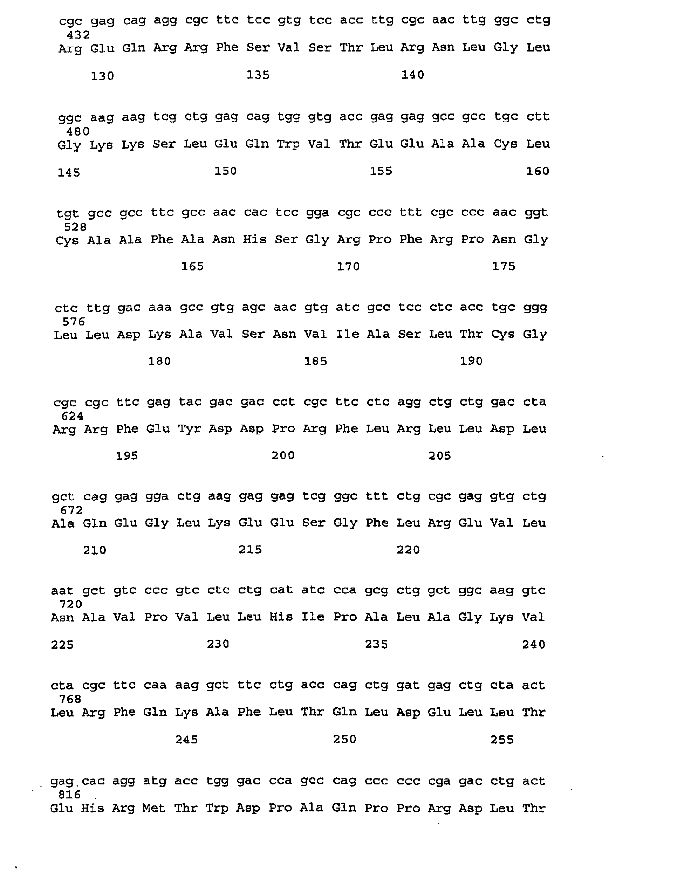

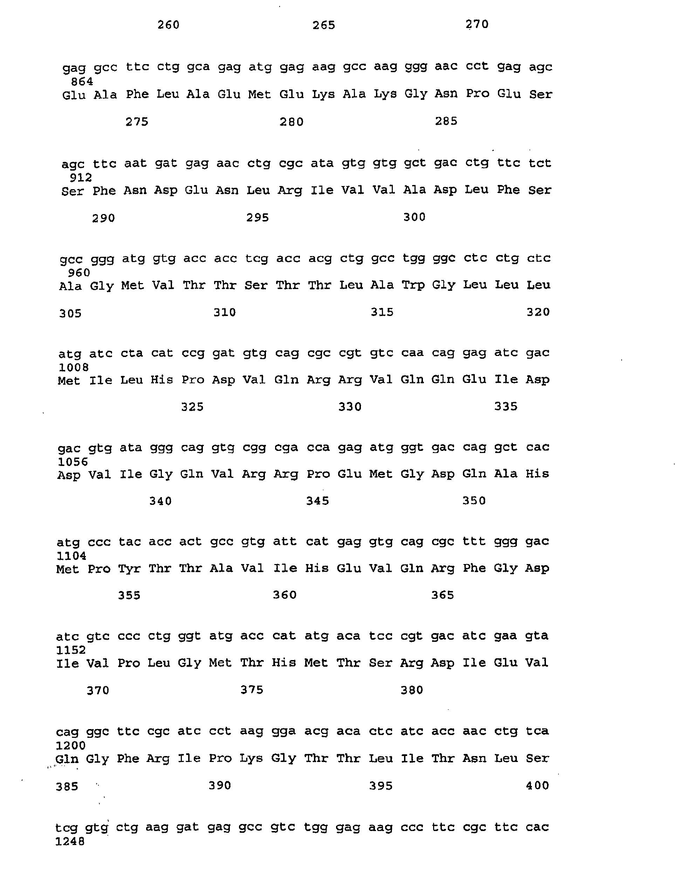

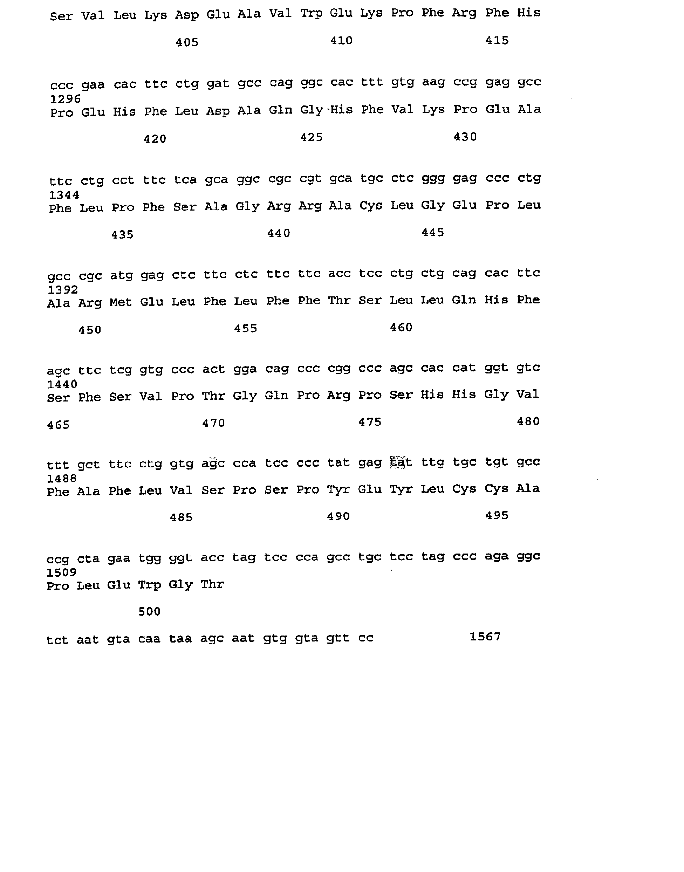

The present invention relates to the discovery of a novel genetic polymorphism in exon 9 of the CYP2D6 gene that results in a frame shift in the CYP2D6 P450 gene product and loss of P450 enzymatic activity. The sequence change corresponds to a mutation at position 5816 of the CYP2D6 gene as numbered in GenBank Accession No. M33388 and as depicted in FIGURE 2. The mutant frame-shifted allele contains the sequence "TA" inserted in place of the "C" at position 5816 of the wild type CYP2D6 genomic sequence (FIGURE 2, SEQ ID NO. 1), resulting in the creation of a mutant C5816TA genomic sequence (FIGURE 3, SEQ ID NO. 2). The mutant frame-shift is within exon 9 of the CYP2D6 gene and occurs at position 1474 of the CYP2D6 cDNA (FIGURE 4; GenBank Accession No. NM_000106; and SEQ ID NO. 3), resulting in the creation of a mutant C5816TA cDNA sequence (FIGURE 5; SEQ ID NO. 4).

The presence of the CYP2D6 C5816TA mutant allele is associated with an altered enzyme activity potentially leading (i) to undesirable effects when individuals are treated with standard doses of certain agents; (ii) to increased susceptibility to cancer following environmental exposures; or (iii) other clinical conditions. Detection of DNA variants at the CYP2D6 locus offers a strategy for identifying individuals at risk based on their genotype, prior to treatment. In accordance with the present invention, the detection of the CYP2D6 C5816TA mutant allele may be effected using any known state-of-the-art hybridization approaches, including, but not limited to, Southern blot, reverse dot-blot and liquid phase hybridization, as those skilled in the art will understand how to perform based upon the present description.

Reverse dot blot refers to a treatment of a support (such as nylon membrane) to which is attached an ASO capable of hybridizing with a labeled complementary probe (such as amplified DNA). In accordance with another embodiment of the present invention, the detection of specific mutations within a gene of interest is through hybridization of PCR products with ASO probes for the wild type or variant alleles utilized in parallel hybridizations. Only the oligonucleotide that precisely hybridizes to the target sequences produces a signal from a labeled probe. This genotyping method, which require small amounts of nucleated cells derived from a variety of sources, is not affected by the underlying disease or by drugs taken by the patient, and it provides results within a suitable period of time, such as, for example, within about 24-48 hours, thus allowing for relatively rapid intervention. The present invention provides diagnostic tests, e.g., to identify individuals with altered xenobiotics-metabolizing activities based on their genotypes. Such diagnostic tests to determine the genotypes of individuals are advantageous, e.g., because, as those skilled in the art will appreciate, the measurement of enzymatic activities can have many undesirable limitations. Accordingly, tests are provided for detecting mutations in the CYP2D6 gene. In certain embodiments, these tests involve the amplification of all, or a portion of, the CYP269 genomic locus, or cDNA, where the mutations of interest are found. Following amplification, the amplified fragments can then be assayed for the presence, or absence, of the specific mutation of interest (i.e. at least one of the mutations) by using hybridization with ASO probes.

Although much of these assays can be done in any molecular biology facilities, procedures and kits are designed that contain all the reagents, primers and solutions for the genotyping test to facilitate the procedure for use in general clinical laboratories, such as those found in a typical hospital, clinic and even private reference laboratories.

The present invention provides isolated oligonucleotide molecules comprising sequences hybridizing to genes encoding xenobiotic metabolizing enzyme CYP2D6, wild type and mutant alleles thereof; wherein said sequences are sufficiently complementary to said genes to hybridize therewith, respectively. In accordance with the present invention, there is provided an isolated oligonucleotide molecule comprising a mutant allele of CYP2D6 which contains a point mutation at position 5816 corresponding to a C to TA substitution and which, further, may optionally also contain a point mutation at position 5799 corresponding to a G to C substitution. In Preferred mutant oligonucleotide molecules of the present invention include, for example, those having a nucleic acid sequence of at least about 10 to 25 consecutive nucleotides of SEQ ID NO. 2 or 4; while preferred wild type oligonucleotide molecules include, for example, those having a nucleic acid sequence of at least about 10 to 25 consecutive nucleotides of SEQ ID NO. 1 or 3.

In accordance with the present invention there are provided diagnostic assays for determining genetic variants in a CYP2D6 gene in a subject, which comprise the steps of: a) obtaining a genomic DNA sample of said subject; b) using the DNA sample of step a), amplifying a fragment comprising a polymorphic site of the CYP2D6 genes; c) hybridizing the amplified fragment of step b) with ASO probes corresponding to wild type and variant alleles to determine the CYP2D6 genotype of the subject.

In accordance with a preferred embodiment of the present invention, the amplifying step b) is effected with PCR primers as set forth below. In accordance with a preferred embodiment of the present invention, the method further comprises a step i) before step c) consisting in subjecting the amplified fragment of step b) to Southern dot blot transfer on membrane, and wherein step c) is effected by hybridizing the dot blots with the oligonucleotide. In accordance with a preferred embodiment of the present invention, a labeled ASO probe is used in step c) and is selected from the sequences set forth below and hybridizes under stringent conditions.

The invention further provides diagnostic kits for determining DNA variations in the CYP2D6 gene in a subject, which comprises: a) at least one of PCR primer sets; and b) at least one of the ASO probe.

In certain embodiments, the invention utilizes methods of detecting the presence of other CYP2D6 polymorphisms, in combination with the CYP2D6 C5816TA polymorphic variation of the invention. Several null CYP2D6 alleles have been characterized and PCR-RFLP assays have been developed for convenient genotyping (Gonzalez and Meyer 1991). The most common alleles are CYP2D6 *3 (1bp deletion at pos. A2637) and *4 (splice-site mutation G1934A), accounting for over 96% of all null alleles ( as described in WO/0024926, the content of which are incorporated herein by reference). Individuals homozygous for any of these null alleles, completely lacking CYP2D6 activity, will be considered phenotypically PMs. There are several other less common polymorphisms: C188T, C212A, insT226, G971C, C1111T, G1726C, delT1795, G1846T, G1846A, G2064A, delA2701-A2703, delG2702-G2704, and A3023C. There are significant interethnic differences in the prevalence of the PM phenotype of CYP2D6. For example, in North American and European Caucasian populations, the prevalence of poor metabolisers is about 5%. In contrast, the prevalence is about 1.8% in American blacks, about 1.0% in Chinese, and apparently absent in the Japanese population.

The methods of the present invention provide means for determining if a subject has (diagnostic) or is at risk of developing (prognostic) aan agent sensitivity condition or disorder that is associated with an aberrant CYP2D6 activity, e.g., an aberrant level of CYP2D6 protein or an aberrant CYP2D6 bioactivity. Examples of drugs to which CYP2D6 mutations cause sensitivity include: chlorpromazine, clomipramine, clozapine, desipramine, fluoxetine, fluphenazine, fluvoxamine, haloperidol, levopromazine, mianserin, nortryptiline, paroxetine, perphenazine, risperidone, sertraline, thioridazine, trifluperidol, trimipramine and zuclopenthixol (see Wolf & Smith (1999) Brit Med Bull 55: 366-86). Still the drugs metabolized by P450 CYP2D6 include: alprenolol, amiflavine, amiodorone, amitryptline, apigenin, budesonide, bufuralol, bupranolol, chloral hydrate, clonidine, clotrimazole, codeine, cyclobenzaprine, dexfenfluramine, dextromethorphan, dibucaine, dihydroergotamine, dolasetron, doxorubicin, encainide, ethinylestradiol, ethylmorphine, fenoterol, flecainide, formoterol, guanoxan, 4-hydroxy amphetamine, imipramine, indoramine, ketoconazole, laudanosine, loratadine, MDMA (ecstacy), mefloquine, methoxamine HCI, methoxyphenamine, methoxypsoralen, methysergide HCI, metoclopramide, metoprolol, minaprine, moclobemide, MPTP, mexiletine, nicergoline, nimodipine, nitrendipine, olanzapine, ondansetron, oxprenolol, perhexiline, phenformin, phenylpropanolamine, procainamide, promethazine, N-propylajmaline, propafenone, propranolol, pyrimethamine, quercitin, rifampicin, ritonavir, roxithromycin, serotonin, sparteine, sulfasalazine, tacrine, tamoxifen, timolol, tomoxetine, tranylcypomine, and tropisetron. Preferred methods for detecting altered CYP2D6 activity resulting from a CYP2D6 polymorphism include genetic assays such as RFLP (restriction fragment length polymorphism), ASO PCR (allele specific oligonucleotide hybridization to PCR products or PCR using mutant/wildtype specific oligo primers), SSCP (single stranded conformation polymorphism) and TGGE/DGGE (temperature or denaturing gradient gel electrophoresis), and MDE (mutation detection electrophoresis).

Accordingly, the invention provides methods for determining whether a subject has or is likely to develop, a disease or condition that is caused by or contributed to by an abnormal CYP2D6 level or bioactivity, for example, comprising determining the level of a CYP2D6 gene or protein, a CYP2D6 bioactivity and/or the presence of a mutation or particular polymorphic variant in the CYP2D6 gene.

In one embodiment of the present invention, the method comprises determining whether a subject has an abnormal mRNA and/or protein level of CYP2D6, such as by Northern blot analysis, reverse transcription-polymerase chain reaction (RT-PCR), *in situ* hybridization, immunoprecipitation, Western blot hybridization, or immunohistochemistry. According to the method, cells are obtained from a subject and the CYP2D6 protein or mRNA level is determined and compared to the level of CYP2D6 protein or mRNA level in a healthy subject. An abnormal level of CYP2D6 polypeptide or mRNA level is likely to be indicative of an aberrant CYP2D6 activity. In particular, the invention provides methods and reagents for detecting CYP2D6 C5816TA nucleic acid or encoded protein sequence changes in a patient sample.

In another embodiment of the present invention, the method comprises measuring at least one activity of CYP2D6, such as a monoxygenase activity, using techniques known in the art, and those skilled in the art based upon the present description will understand how to perform such measurements. Comparison of the results obtained with results from similar analysis performed on CYP2D6 proteins from healthy subjects is indicative of whether a subject has an abnormal CYP2D6 activity.

In preferred embodiments, the methods for determining whether a subject has or is at risk for developing a disease, which is caused by or contributed to by an aberrant CYP2D6 activity is characterized as comprising detecting, in a sample of cells from the subject, the presence or absence of a genetic alteration characterized by at least one of (i) an alteration affecting the integrity of a gene encoding a CYP2D6 polypeptide, particularly a C5816TA mutation, or (ii) the mis-expression of the CYP2D6 gene. For example, such genetic alterations can be detected by ascertaining the existence of at least one of (i) a deletion of one or more nucleotides from a CYP2D6 gene, (ii) an addition of one or more nucleotides to a CYP2D6 gene, (iii) a substitution of one or more nucleotides of a CYP2D6 gene, (iv) a gross chromosomal rearrangement of a CYP2D6 gene, (v) a gross alteration in the level of a messenger RNA transcript of a CYP2D6 gene, (vii) aberrant modification of a CYP2D6 gene, such as of the methylation pattern of the genomic DNA, (vii) the presence of a non-wild type splicing pattern of a messenger RNA transcript of a CYP2D6 gene, (viii) a non-wild type level of a CYP2D6 polypeptide, (ix) allelic loss of a CYP2D6 gene, and/or (x) inappropriate post-translational modification of a CYP2D6 polypeptide. As set out below, the present invention provides a large number of assay techniques for detecting alterations in a CYP2D6 gene. These methods include, but are not limited to, methods involving sequence analysis, Southern blot hybridization, restriction enzyme site mapping, and methods involving detection of absence of nucleotide pairing between the nucleic acid to be analyzed and a probe. These and other methods are further described *infra.*

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene, such as a single nucleotide polymorphism ("SNP"), in a subject can render the subject susceptible to developing a specific disease or disorder. Polymorphic regions in genes, e.g, CYP2D6 genes, can be identified, by determining the nucleotide sequence of genes in populations of individuals. If a polymorphic region, e.g., SNP is identified, then the link with a specific disease can be determined by studying specific populations of individuals, e.g, individuals which developed a specific disease, such as congestive heart failure, hypertension, hypotension, or a cancer (e.g. a cancer involving growth of a steroid responsive tumor or tumors). A polymorphic region can be located in any region of a gene, e.g., exons, in coding or non coding regions of exons, introns, and promoter region.

Those skilled in the art will understand that it is likely that CYP2D6 genes comprise polymorphic regions, specific alleles of which may be associated with specific diseases or conditions or with an increased likelihood of developing such diseases or conditions. Thus, the invention provides methods for determining the identity of the allele or allelic variant of a polymorphic region of a CYP2D6 gene in a subject, to thereby determine whether the subject has or is at risk of developing a disease or disorder associated with a specific allelic variant of a polymorphic region.

In an exemplary embodiment, there is provided a nucleic acid composition comprising a nucleic acid probe including a region of nucleotide sequence which is capable of hybridizing to a sense or antisense sequence of a CYP2D6 gene or naturally occurring mutants thereof, or 5' or 3' flanking sequences or intronic sequences naturally associated with the subject CYP2D6 genes or naturally occurring mutants thereof. The nucleic acid of a cell is rendered accessible for hybridization, the probe is contacted with the nucleic acid of the sample, and the hybridization of the probe to the sample nucleic acid is detected. Such techniques can be used to detect alterations or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridizing specifically to allelic variants, such as single nucleotide polymorphisms, are attached to a solid phase support, e.g., a "chip". Oligonucleotides can be bound to a solid support by a variety of processes, including lithography. For example a chip can hold up to 250,000 oligonucleotides. Mutation detection analysis using these chips comprising oligonucleotides, also termed "DNA probe arrays" is described e.g., in Cronin et al., (1996) *Human Mutation* 7:244. In one embodiment, a chip comprises all the allelic variants of at least one polymorphic region of a gene. The solid phase support is then contacted with a test nucleic acid and hybridization to the specific probes is detected. Accordingly, the methods of the present invention provide for the identification of numerous allelic variants of one or more genes in a simple hybridization experiment.

In certain embodiments, detection of the alteration comprises utilizing the probe/primer in a PCR (see, e.g. U.S. Patent Nos. 4,683,195 and 4,683,202), such as anchor PCR or RACE PCR, or, alternatively, in a ligase chain reaction (LCR) (see, e.g., Landegran et al. (1988) *Science* 241:1077-1080; and Nakazawa et al. (1994) *PNAS* 91:360-364), the latter of which can be particularly useful for detecting point mutations in the CYP2D6 gene (see Abravaya et al. (1995) *Nuc Acid Res* 23:675-682). In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize to a CYP2D6 gene under conditions such that hybridization and amplification of the CYP2D6 gene (if present) occurs, and (iv) detecting the presence or absence of an amplification product, or detecting the size of the amplification product and comparing the length to a control sample. It should be understood by those skilled in the art that it is anticipated that PCR and/or LCR may be desirable to use as a preliminary amplification step in conjunction with any of the techniques used for detecting mutations described herein.

Alternative amplification methods for use in the present invention include: self sustained sequence replication (Guatelli, J.C. et al., 1990, *Proc. Natl. Acad. Sci. USA* 87:1874-1878), transcriptional amplification system (Kwoh, D.Y. et al., 1989, *Proc. Natl. Acad. Sci. USA* 86:1173-1177), Q-Beta Replicase (Lizardi, P.M. et al., 1988, *Bio*/*Technology* 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In a preferred embodiment of the subject assay, mutations in, or allelic variants, of a CYP2D6 gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover, the use of sequence specific ribozymes (see, for example, U.S. Patent No. 5,498,531) can be used to score for the presence of specific mutations by development or loss of a ribozyme cleavage site.

In yet another embodiment, any of a variety of sequencing reactions known in the art can be used to directly sequence the CYP2D6 gene and detect mutations by comparing the sequence of the sample CYP2D6 with the corresponding wild-type (control) sequence. Exemplary sequencing reactions include those based on techniques developed by Maxim and Gilbert (*Proc. Natl Acad Sci USA* (1977) 74:560) or Sanger (Sanger et al (1977) *Proc*. *Nat. Acad. Sci* 74:5463). It is also contemplated that any of a variety of automated sequencing procedures may be utilized when performing the subject assays (*Biotechniques* (1995) 19:448), including sequencing by mass spectrometry (see, for example PCT publication WO 94/16101; Cohen et al. (1996) *Adv Chromatogr* 36:127-162; and Griffin et al. (1993) *Appl Biochem Biotechnol* 38:147-159). It will be evident to one skilled in the art that, for certain embodiments, the occurrence of only one, two or three of the nucleic acid bases need be determined in the sequencing reaction. For instance, A-track or the like, e.g., where only one nucleic acid is detected, can be carried out.

In a further embodiment, protection from cleavage agents (such as a nuclease, hydroxylamine or osmium tetroxide and with piperidine) can be used to detect mismatched bases in RNA/RNA or RNA/DNA or DNA/DNA heteroduplexes (Myers, et al. (1985) *Science* 230:1242). In general, the art technique of "mismatch cleavage" starts by providing heteroduplexes formed by hybridizing (labelled) RNA or DNA containing the wild-type CYP2D6 sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with S1 nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al, (1988) *Proc*. *Natl Acad Sci USA* 85:4397; Saleeba et al, (1992) *Methods Enzymol.* 217:286-295. In a preferred embodiment, the control DNA or RNA can be labeled for detection using methods known in the art based upon the present description.

In still another embodiment, the mismatch cleavage reaction employs one or more proteins that recognize mismatched base pairs in double-stranded DNA (so called "DNA mismatch repair" enzymes) in defined systems for detecting and mapping point mutations in CYP2D6 cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) *Carcinogenesis* 15:1657-1662). According to an exemplary embodiment, a probe based on a CYP2D6 sequence, e.g., a wild-type CYP2D6 sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations or the identity of the allelic variant of a polymorphic region in CYP2D6 genes. For example, SSCP may be used to detect differences in electrophoretic mobility between mutant and wild type nucleic acids (Orita et al. (1989) *Proc Natl*. *Acad. Sci USA* 86:2766, see also Cotton (1993) *Mutat Res* 285:125-144; and Hayashi (1992) *Genet Anal Tech Appl* 9:73-79). Single-stranded DNA fragments of sample and control CYP2D6 nucleic acids are denatured and allowed to renature. The secondary structure of single-stranded nucleic acids varies according to sequence, the resulting alteration in electrophoretic mobility enables the detection of even a single base change. The DNA fragments may be labelled or detected with labelled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers et al (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing agent gradient to identify differences in the mobility of control and sample DNA (Rosenbaum and Reissner (1987) *Biophys Chem* 265:12753).

Examples of other techniques for detecting point mutations or the identity of the allelic variant of a polymorphic region include, but are not limited to, selective oligonucleotide hybridization, selective amplification, or selective primer extension. For example, oligonucleotide primers may be prepared in which the known mutation or nucleotide difference (e.g., in allelic variants) is placed centrally and then hybridized to target DNA under conditions which permit hybridization only if a perfect match is found (Saiki et al. (1986) *Nature* 324:163); Saiki et al (1989) *Proc. Natl Acad. Sci USA* 86:6230). Such allele specific oligonucleotide hybridization techniques may be used to test one mutation or polymorphic region per reaction when oligonucleotides are hybridized to PCR amplified target DNA or a number of different mutations or polymorphic regions when the oligonucleotides are attached to the hybridizing membrane and hybridized with labelled target DNA.

Alternatively, allele specific amplification technology which depends on selective PCR amplification may be used in conjunction with the instant invention. Oligonucleotides used as primers for specific amplification may carry the mutation or polymorphic region of interest in the center of the molecule (so that amplification depends on differential hybridization) (Gibbs et al (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238. In addition it may be desirable to introduce a novel restriction site in the region of the mutation to create cleavage-based detection (Gasparini et al (1992) *Mol. Cell Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

In another embodiment, identification of the allelic variant is carried out using an oligonucleotide ligation assay (OLA), as described, e.g., in U.S. Pat. No. 4,998,617 and in Landegren, U. et al., *Science* 241:1077-1080 (1988). The OLA protocol uses two oligonucleotides which are designed to be capable of hybridizing to abutting sequences of a single strand of a target. One of the oligonucleotides is linked to a separation marker, e.g,. biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson, D. A. et al. have described a nucleic acid detection assay that combines attributes of PCR and OLA (Nickerson, D. A. et al., *Proc. Natl*. *Acad. Sci.* (*U.S.A.*) 87:8923-8927 (1990). In this method, PCR is used to achieve the exponential amplification of target DNA, which is then detected using OLA.

Several techniques based on this OLA method have been developed and can be used to detect specific allelic variants of a polymorphic region of a CYP2D6 gene. For example, U.S. Patent No. 5,593,826 discloses an OLA using an oligonucleotide having 3'-amino group and a 5'-phosphorylated oligonucleotide to form a conjugate having a phosphoramidate linkage. In another variation of OLA described in Tobe et al. ((1996) *Nucleic Acids Res* 24: 3728), OLA combined with PCR permits typing of two alleles in a single microtiter well. By marking each of the allele-specific primers with a unique hapten, i.e. digoxigenin and fluorescein, each OLA reaction can be detected by using hapten specific antibodies that are labeled with different enzyme reporters, alkaline phosphatase or horseradish peroxidase. This system permits the detection of the two alleles using a high throughput format that leads to the production of two different colors.

The invention further provides methods for detecting single nucleotide polymorphisms in a CYP2D6 gene. Because single nucleotide polymorphisms constitute sites of variation flanked by regions of invariant sequence, their analysis requires no more than the determination of the identity of the single nucleotide present at the site of variation and it is unnecessary to determine a complete gene sequence for each patient. Several methods have been developed to facilitate the analysis of such single nucleotide polymorphisms.

In one embodiment, the single base polymorphism can be detected by using a specialized exonuclease-resistant nucleotide, as disclosed, e.g., in Mundy, C. R. (U.S. Pat. No.4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amounts of extraneous sequence data.

In another embodiment of the invention, a solution-based method is used for determining the identity of the nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA™ is described by Goelet, P. et al., (PCT Appln. No. WO92/15712, and U.S. Patent Nos. 5,762,876, 5,888,819, and 6,004,744). The method of Goelet, P. et al., uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. is preferably a heterogeneous phase assay, in which the primer or the target molecule is immobilized to a solid phase.

Recently, several primer-guided nucleotide incorporation procedures for assaying polymorphic sites in DNA have been described (Komher, J. S. et al., *Nucl. Acids. Res.* 17:7779-7784 (1989); Sokolov, B. P., Nucl. Acids Res. 18:3671 (1990); Syvanen, A. -C., et al., *Genomics* 8:684-692 (1990); Kuppuswamy, M. N. et al., *Proc. Natl. Acad. Sci.* (U.S.A.) 88:1143-1147 (1991); Prezant, T. R. et al., Hum. Mutat. 1:159-164 (1992); Ugozzoli, L. et al., *GATA* 9:107-112 (1992); Nyren, P. et al., *Anal. Biochem.* 208:171-175 (1993)). These methods differ from GBA™ in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A. -C., et al., *Amer.J. Hum. Genet.* 52:46-59 (1993)).

For mutations that produce premature termination of protein translation, the protein truncation test (PTT) offers an efficient diagnostic approach (Roest, et. al., (1993) *Hum. Mol.* Genet. 2:1719-21; van der Luijt, et. al., (1994) *Genomics* 20:1-4). For PTT, RNA is initially isolated from available tissue and reverse-transcribed, and the segment of interest is amplified by PCR. The products of reverse transcription PCR are then used as a template for nested PCR amplification with a primer that contains an RNA polymerase promoter and a sequence for initiating eukaryotic translation. After amplification of the region of interest, the unique motifs incorporated into the primer permit sequential *in vitro* transcription and translation of the PCR products. Upon sodium dodecyl sulfate-polyacrylamide gel electrophoresis of translation products, the appearance of truncated polypeptides signals the presence of a mutation that causes premature termination of translation. In a variation of this technique, DNA (as opposed to RNA) is used as a PCR template when the target region of interest is derived from a single exon.

The methods described herein may be performed, for example, by utilizing pre-packaged diagnostic kits comprising at least one probe nucleic acid, primer set; and/or antibody reagent described herein, which may be conveniently used, *e.g.*, in clinical settings to diagnose patients exhibiting symptoms or family history of a disease or illness involving a CYP2D6 polypeptide.

Any cell type or tissue may be utilized in the diagnostics described below. In a preferred embodiment a bodily fluid, e.g., blood, is obtained from the subject to determine the presence of a mutation or the identity of the allelic variant of a polymorphic region of a CYP2D6 gene. A bodily fluid, e.g, blood, can be obtained by known techniques (e.g. venipuncture). Alternatively, nucleic acid tests can be performed on dry samples (e.g. hair or skin). For prenatal diagnosis, fetal nucleic acid samples can be obtained from maternal blood as described in International Patent Application No. WO91/07660 (and counterpart U.S. Patent No. 5,641,628). Alternatively, amniocytes or chorionic villi may be obtained using methods known in the art for performing prenatal testing.

When using RNA or protein to determine the presence of a mutation or of a specific allelic variant of a polymorphic region of a CYP2D6 gene, the cells or tissues that may be utilized must express the CYP2D6 gene. Preferred cells for use in these methods include, for example, cardiac cells (see EXAMPLES). Alternative cells or tissues that can be used, can be identified by determining the expression pattern of the specific CYP2D6 gene in a subject, such as by Northern blot analysis, based upon the present description.

Diagnostic procedures may also be performed *in situ* directly upon tissue sections (fixed and/or frozen) of patient tissue obtained from biopsies or resections, such that no, or substantially no, nucleic acid purification would be deemed necessary by those skilled in the art. Nucleic acid reagents may be used as probes and/or primers for such *in situ* procedures (see, for example, Nuovo, G.J., 1992, PCR i*n situ* hybridization: protocols and applications, Raven Press, NY).

In addition to methods which focus primarily on the detection of one nucleic acid sequence, profiles may also be assessed in such detection schemes. Fingerprint profiles may be generated, for example, by utilizing a differential display procedure, Northern analysis and/or RT-PCR, based upon the present description.

Antibodies directed against wild type or mutant CYP2D6 polypeptides or allelic variants thereof, which are discussed above, may also be used in disease diagnostics and prognostics. Such diagnostic methods, may be used to detect abnormalities in the level of CYP2D6 polypeptide expression, or abnormalities in the structure and/or tissue, cellular, or subcellular location of a CYP2D6 polypeptide. Structural differences may include, for example, differences in the size, electronegativity, or antigenicity of the mutant CYP2D6 polypeptide relative to the normal CYP2D6 polypeptide. Protein from the tissue or cell type to be analyzed may be detected or isolated using techniques which are well known to one of skill in the art, including but not limited to western blot analysis. For a detailed explanation of methods for carrying out Western blot analysis, see Sambrook et al, 1989, supra, at Chapter 18. The protein detection and isolation methods employed herein may also be such as those described in Harlow and Lane, for example, (Harlow, E. and Lane, D., 1988, Antibodies: A Laboratory Manual, Cold Spring Harbor Laboratory Press, Cold Spring Harbor, New York).

This can be accomplished, for example, as will be appreciated by those skilled in the art based on the present description by immunofluorescence techniques employing a fluorescently labeled antibody (see below) coupled with light microscopic, flow cytometric, or fluorimetric detection. The antibodies (or fragments thereof) useful in the present invention may, additionally, be employed histologically, as in immunofluorescence or immunoelectron microscopy, for *in situ* detection of CYP2D6 polypeptides. *In situ* detection may be accomplished by removing a histological specimen from a patient, and applying thereto a labeled antibody of the present invention. The antibody (or fragment) is preferably applied by overlaying the labeled antibody (or fragment) onto a biological sample. Through the use of such a procedure, it is possible to determine not only the presence of the CYP2D6 polypeptide, but also its distribution in the examined tissue. Using the present invention, one of ordinary skill will readily perceive that any of a wide variety of histological methods (such as staining procedures) can be modified in order to achieve such *in situ* detection.

Often a solid phase support or carrier is used as a support capable of binding an antigen or an antibody. Well-known supports or carriers include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include, for example, polystyrene beads. Those skilled in the art will know many other suitable carriers for binding antibody or antigen, or will be able to ascertain the same by use of routine experimentation.

One means for labeling an anti-CYP2D6 polypeptide specific antibody is via linkage to an enzyme and use in an enzyme immunoassay (EIA) (Voller, "The Enzyme Linked Immunosorbent Assay (ELISA)", *Diagnostic Horizons* 2:1-7, 1978, Microbiological Associates Quarterly Publication, Walkersville, MD; Voller, et al., *J*. *Clin. Pathol.* 31:507-520 (1978); Butler, *Meth. Enzymol.* 73:482-523 (1981); Maggio, (ed.) Enzyme Immunoassay, CRC Press, Boca Raton, FL, 1980; Ishikawa, et al., (eds.) Enzyme Immunoassay, Kgaku Shoin, Tokyo, 1981). The enzyme which is bound to the antibody will react with an appropriate substrate, preferably a chromogenic substrate, in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate, dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may also be accomplished using any of a variety of other immunoassays. For example, by radioactively labeling the antibodies or antibody fragments, it is possible to detect fingerprint gene wild type or mutant peptides through the use of a radioimmunoassay (RIA) (see, for example, Weintraub, B., Principles of Radioimmunoassays, Seventh Training Course on Radioligand Assay Techniques, The Endocrine Society, March, 1986). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labeling compounds are fluorescein isothiocyanate, rhodamine, phycoerythrin, phycocyanin, allophycocyanin, o-phthaldehyde and fluorescamine.

The antibody can also be detectably labeled using fluorescence emitting metals such as 152Eu, or others of the lanthanide series. These metals can be attached to the antibody using such metal chelating groups as diethylenetriaminepentacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in, which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

Moreover, it will be understood that any of the above methods for detecting alterations in a gene or gene product or polymorphic variants can be used to monitor the course of treatment or therapy.

Large scale detection methods allow faster, less expensive analysis of the expression levels of many genes simultaneously. Such methods typically involve an ordered array of probes affixed to a solid substrate. Each probe is capable of hybridizing to a different set of nucleic acids. In one method, probes are generated by amplifying or synthesizing a substantial portion of the coding regions of various genes of interest. These genes are then spotted onto a solid support. mRNA samples are obtained, converted to cDNA, amplified and labeled (usually with a fluorescence label). The labeled cDNAs are then applied to the array, and cDNAs hybridize to their respective probes in a manner that is linearly related to their concentration. Detection of the label allows measurement of the amount of each cDNA adhered to the array.

Many methods for performing such DNA array experiments are well known in the art. Exemplary methods are described below but are not intended to be limiting.

Arrays are often divided into microarrays and macroarrays, where microarrays have a much higher density of individual probe species per area. Microarrays may have as many as 1000 or more different probes in a 1 cm² area. There is no concrete cut-off to demarcate the difference between micro- and macroarrays, and both types of arrays are contemplated for use with the invention. However, because of their small size, microarrays provide great advantages in speed, automation and cost-effectiveness.

Microarrays are known in the art and consist of a surface to which probes that correspond in sequence to gene products (e.g., cDNAs, mRNAs, oligonucleotides) are bound at known positions. In one embodiment, the microarray is an array (i.e., a matrix) in which each position represents a discrete binding site for a product encoded by a gene (e.g., a protein or RNA), and in which binding sites are present for products of most or almost all of the genes in the organism's genome. In a preferred embodiment, the "binding site" (hereinafter, "site") is a nucleic acid or nucleic acid analogue to which a particular cognate cDNA can specifically hybridize. The nucleic acid or analogue of the binding site can be, e.g., a synthetic oligomer, a full-length cDNA, a less-than full length cDNA, or a gene fragment.

Although in a preferred embodiment the microarray contains binding sites for products of all or almost all genes in the target organism's genome, such comprehensiveness is not necessarily required. Usually the microarray will have binding sites corresponding to at least 100 genes and more preferably, 500, 1000, 4000 or more. In certain embodiments, the most preferred arrays will have about 98-100% of the genes of a particular organism represented. In other embodiments, the invention provides customized microarrays that have binding sites corresponding to fewer, specifically selected genes. Microarrays with fewer binding sites are cheaper, smaller and easier to produce. In particular, the invention provides microarrays customized for the determination of graft status. In preferred embodiments customized microarrays comprise binding sites for fewer than 4000, fewer than 1000, fewer than 200 or fewer than 50 genes, and comprise binding sites for at least 2, preferably at least 3, 4, 5 or more genes of any of clusters A, B, C, D, E, F or G. Preferably, the microarray has binding sites for genes relevant to testing and confirming a biological network model of interest. Several exemplary human microarrays are publically available. The Affymetrix GeneChip HUM 6.8K is an oligonucleotide array composed of 7,070 genes. A microarray with 8,150 human cDNAs was developed and published by Research Genetics (Bittner et al., 2000, *Nature* 406:443-546).

The probes to be affixed to the arrays are typically polynucleotides. These DNAs can be obtained by, e.g., polymerase chain reaction (PCR) amplification of gene segments from genomic DNA, cDNA (e.g., by RT-PCR), or cloned sequences. PCR primers are chosen, based on the known sequence of the genes or cDNA, that result in amplification of unique fragments (i.e. fragments that do not share more than 10 bases of contiguous identical sequence with any other fragment on the microarray). Computer programs are useful in the design of primers with the required specificity and optimal amplification properties. See, e.g., Oligo pl version 5.0 (National Biosciences). In the case of binding sites corresponding to very long genes, it will sometimes be desirable to amplify segments near the 3' end of the gene so that when oligo-dT primed cDNA probes are hybridized to the microarray, less-than-full length probes will bind efficiently. Random oligo-dT priming may also be used to obtain cDNAs corresponding to as yet unknown genes, known as ESTs. Certain arrays use many small oligonucleotides corresponding to overlapping portions of genes. Such oligonucleotides may be chemically synthesized by a variety of well known methods. Synthetic sequences are between about 15 and about 500 bases in length, more typically between about 20 and about 50 bases. In some embodiments, synthetic nucleic acids include non-natural bases, e.g., inosine. As noted above, nucleic acid analogues may be used as binding sites for hybridization. An example of a suitable nucleic acid analogue is peptide nucleic acid (see, e.g., Egholm et al., 1993, PNA hybridizes to complementary oligonucleotides obeying the Watson-Crick hydrogen-bonding rules, *Nature* 365:566-568; see also U.S. Pat. No. 5,539,083).

In an alternative embodiment, the binding (hybridization) sites are made from plasmid or phage clones of genes, cDNAs (e.g., expressed sequence tags), or inserts therefrom (Nguyen et al., 1995, Differential gene expression in the murine thymus assayed by quantitative hybridization of arrayed cDNA clones, *Genomics* 29:207-209). In yet another embodiment, the polynucleotide of the binding sites is RNA.

The nucleic acids or analogues are attached to a solid support, which may be made from glass, plastic (e.g., polypropylene, nylon), polyacrylamide, nitrocellulose, or other materials. A preferred method for attaching the nucleic acids to a surface is by printing on glass plates, as is described generally by Schena et al., 1995, *Science* 270:467-470. This method is especially useful for preparing microarrays of cDNA. (See also DeRisi et al., 1996, *Nature Genetics* 14:457-460; Shalon et al., 1996, *Genome Res*. 6:639-645; and Schena et al., 1995, *Proc. Natl. Acad. Sci. USA* 93:10539-11286).

A second preferred method for making microarrays is by making high-density oligonucleotide arrays. Techniques are known for producing arrays containing thousands of oligonucleotides complementary to defined sequences, at defined locations on a surface using photolithographic techniques for synthesis *in situ* (see, Fodor et al., 1991, *Science* 251:767-773; Pease et al., 1994, *Proc. Natl. Acad. Sci. USA* 91:5022-5026; Lockhart et al., 1996, *Nature Biotech* 14:1675; U.S. Pat. Nos. 5,578,832; 5,556,752; and 5,510,270), or other methods for rapid synthesis and deposition of defined oligonucleotides (Blanchard et al., 1996, "Sequence to array: probing the genome's secrets," Nat. Biotechnol. Dec 1996, 14(13): 1649)). When these methods are used, oligonucleotides of known sequence are synthesized directly on a surface such as a derivatized glass slide. Usually, the array produced is redundant, with several oligonucleotide molecules per RNA. Oligonucleotide probes can be chosen to detect alternatively spliced mRNAs. More recently, microarrays have been made using an ink-jet oligonucleotide synthesizer (Hughes et al., Nat. Biotechnol. April 2001, 19(4): 342-347).

Other methods for making microarrays, e.g., by masking (Maskos and Southern, 1992, *Nuc. Acids Res.* 20:1679-1684), may also be used. In principal, any type of array, for example, dot blots on a nylon hybridization membrane (see Sambrook et al., Molecular Cloning--A Laboratory Manual (2nd Ed.), Vol. 1-3, Cold Spring Harbor Laboratory, Cold Spring Harbor, N.Y., 1989), could be used, although, as will be recognized by those of skill in the art, very small arrays will be preferred because hybridization volumes will be smaller.

The nucleic acids to be contacted with the microarray may be prepared in a variety of ways. Methods for preparing total and poly(A)+ RNA are well known and are described generally in Sambrook et al., supra. Labeled cDNA is prepared from mRNA by oligo dT-primed or random-primed reverse transcription, both of which are well known in the art (see e.g., Klug and Berger, 1987, Methods Enzymol. 152:316-325). Reverse transcription may be carried out in the presence of a dNTP conjugated to a detectable label, most preferably a fluorescently labeled dNTP. Alternatively, isolated mRNA can be converted to labeled antisense RNA synthesized by in vitro transcription of double-stranded cDNA in the presence of labeled dNTPs (Lockhart et al., 1996, *Nature Biotech.* 14:1675). The cDNAs or RNAs can be synthesized in the absence of detectable label and may be labeled subsequently, e.g., by incorporating biotinylated dNTPs or rNTP, or some similar means (e.g., photo-cross-linking a psoralen derivative of biotin to RNAs), followed by addition of labeled streptavidin (e.g., phycoerythrin-conjugated streptavidin) or the equivalent.

When fluorescent labels are used, many suitable fluorophores are known, including fluorescein, lissamine, phycoerythrin, rhodamine (Perkin Elmer Cetus, Toronto, Canada), Cy2, Cy3, Cy3.5, Cy5, Cy5.5, Cy7, FluorX (Amersham, Piscataway, New Jersey, USA) and others (see, e.g., Kricka, 1992, Academic Press San Diego, Calif., USA).

In another embodiment, a label other than a fluorescent label is used. For example, a radioactive label, or a pair of radioactive labels with distinct emission spectra, can be used (see Zhao et al., 1995, *Gene* 156:207; Pietu et al., 1996, *Genome Res.* 6:492). However, use of radioisotopes is a less-preferred embodiment.

Nucleic acid hybridization and wash conditions are chosen so that the population of labeled nucleic acids will specifically hybridize to appropriate, complementary nucleic acids affixed to the matrix. As used herein, one polynucleotide sequence is considered complementary to another when, if the shorter of the polynucleotides is less than or equal to 25 bases, there are no mismatches using standard base-pairing rules or, if the shorter of the polynucleotides is longer than 25 bases, there is no more than a 5% mismatch. Preferably, the polynucleotides are perfectly complementary (no mismatches).

Optimal hybridization conditions will depend on the length (e.g., oligomer versus polynucleotide greater than 200 bases) and type (e.g., RNA, DNA, PNA) of labeled nucleic acids and immobilized polynucleotide or oligonucleotide. General parameters for specific (i.e., stringent) hybridization conditions for nucleic acids are described in Sambrook et al., supra, and in Ausubel et al., 1987, Current Protocols in Molecular Biology, Greene Publishing and Wiley-lnterscience, New York, which is incorporated in its entirety for all purposes. Non-specific binding of the labeled nucleic acids to the array can be decreased by treating the array with a large quantity of non-specific DNA -- a so-called "blocking" step.

When fluorescently labeled probes are used, the fluorescence emissions at each site of a transcript array can be, preferably, detected by scanning confocal laser microscopy. When two fluorophores are used, a separate scan, using the appropriate excitation line, is carried out for each of the two fluorophores used. Alternatively, a laser can be used that allows simultaneous specimen illumination at wavelengths specific to the two fluorophores and emissions from the two fluorophores can be analyzed simultaneously (see Shalon et al., 1996, *Genome Research* 6:639-645). In a preferred embodiment, the arrays are scanned with a laser fluorescent scanner with a computer controlled X-Y stage and a microscope objective. Sequential excitation of the two fluorophores is achieved with a multi-line, mixed gas laser and the emitted light is split by wavelength and detected with two photomultiplier tubes. Fluorescence laser scanning devices are described in Schena et al., 1996, *Genome Res.* 6:639-645 and in other references cited herein. Alternatively, the fiber-optic bundle described by Ferguson et al., 1996, *Nature Biotech.* 14:1681-1684, may be used to monitor mRNA abundance levels at a large number of sites simultaneously. Fluorescent microarray scanners are commercially available from Affymetrix, Packard BioChip Technologies, BioRobotics and many other suppliers.

Signals are recorded, quantitated and analyzed using a variety of computer software. In one embodiment the scanned image is despeckled using a graphics program (e.g., Hijaak Graphics Suite) and then analyzed using an image gridding program that creates a spreadsheet of the average hybridization at each wavelength at each site. If necessary, an experimentally determined correction for "cross talk" (or overlap) between the channels for the two fluors may be made. For any particular hybridization site on the transcript array, a ratio of the emission of the two fluorophores is preferably calculated. The ratio is independent of the absolute expression level of the cognate gene, but is useful for genes whose expression is significantly modulated by drug administration, gene deletion, or any other tested event.

According to the method of the invention, the relative abundance of an mRNA in two samples is scored as a perturbation and its magnitude determined (i.e., the abundance is different in the two sources of mRNA tested), or as not perturbed (i.e., the relative abundance is the same). As used herein, a difference between the two sources of RNA of at least a factor of about 25% (RNA from one source is 25% more abundant in one source than the other source), more usually about 50%, even more often by a factor of about 2 (twice as abundant), 3 (three times as abundant) or 5 (five times as abundant) is scored as a perturbation. Present detection methods allow reliable detection of difference of an order of about 2-fold to about 5-fold, but more sensitive methods are expected to be developed.

Preferably, in addition to identifying a perturbation as positive or negative, it is advantageous to determine the magnitude of the perturbation. This can be carried out, as noted above, by calculating the ratio of the emission of the two fluorophores used for differential labeling, or by analogous methods that will be readily apparent to those of skill in the art.

In one embodiment of the invention, transcript arrays reflecting the transcriptional state of a cell of interest are made by hybridizing a mixture of two differently labeled sets of cDNAs, to the microarray. One cell is a cell of interest, while the other is used as a standardizing control. The relative hybridization of each cell's cDNA to the microarray then reflects the relative expression of each gene in the two cell. For example, to assess gene expression in a variety of breast cancers, Perou et al. (2000, supra) hybridized fluorescently-labeled cDNA from each tumor to a microarray in conjunction with a standard mix of cDNAs obtained from a set of breast cancer cell lines. In this way, gene expression in each tumor sample was compared against the same standard, permitting easy comparisons between tumor samples.

In preferred embodiments, the data obtained from such experiments reflects the relative expression of each gene represented in the microarray. Expression levels in different samples and conditions may be compared using a variety of statistical methods.

A variety of statistical methods are available to assess the degree of relatedness in expression patterns of different genes. The statistical methods may be broken into two related portions: metrics for determining the relatedness of the expression pattern of one or more gene, and clustering methods, for organizing and classifying expression data based on a suitable metric (Sherlock, 2000, *Curr. Opin. Immunol.* 12:201-205; Butte et al., 2000, Pacific Symposium on Biocomputing, Hawaii, World Scientific, p.418-29).

In one embodiment, Pearson correlation may be used as a metric. In brief, for a given gene, each data point of gene expression level defines a vector describing the deviation of the gene expression from the overall mean of gene expression level for that gene across all conditions. Each gene's expression pattern can then be viewed as a series of positive and negative vectors. A Pearson correlation coefficient can then be calculated by comparing the vectors of each gene to each other. An example of such a method is described in Eisen et al. (1998, supra). Pearson correlation coefficients account for the direction of the vectors, but not the magnitudes.

In another embodiment, Euclidean distance measurements may be used as a metric. In these methods, vectors are calculated for each gene in each condition and compared on the basis of the absolute distance in multidimensional space between the points described by the vectors for the gene.

In a further embodiment, the relatedness of gene expression patterns may be determined by entropic calculations (Butte et al. 2000, supra). Entropy is calculated for each gene's expression pattern. The calculated entropy for two genes is then compared to determine the mutual information. Mutual information is calculated by subtracting the entropy of the joint gene expression patterns from the entropy for calculated for each gene individually. The more different two gene expression patterns are, the higher the joint entropy will be and the lower the calculated mutual information. Therefore, high mutual information indicates a non-random relatedness between the two expression patterns.

The different metrics for relatedness may be used in various ways to identify clusters of genes. In one embodiment, comprehensive pairwise comparisons of entropic measurements will identify clusters of genes with particularly high mutual information. In a preferred embodiment, expression patterns for two genes are correlated if the normalized mutual information score is greater than or equal to 0.7, and preferably greater than 0.8, greater than 0.9 or greater than 0.95. In alternative embodiments, a statistical significance for mutual information may be obtained by randomly permuting the expression measurements 30 times and determining the highest mutual information measurement obtained from such random associations. All clusters with a mutual information higher than can be obtained randomly after 30 permutations are statistically significant. In a further embodiment, expression patterns for two genes are correlated if the correlation coefficient is greater than or equal to 0.8, and preferably greater than 0.85, 0.9 or, most preferably greater than 0.95.

In another embodiment, agglomerative clustering methods may be used to identify gene clusters. In one embodiment, Pearson correlation coefficients or Euclidean metrics are determined for each gene and then used as a basis for forming a dendrogram. In one example, genes were scanned for pairs of genes with the closest correlation coefficient. These genes are then placed on two branches of a dendrogram connected by a node, with the distance between the depth of the branches proportional to the degree of correlation. This process continues, progressively adding branches to the tree. Ultimately a tree is formed in which genes connected by short branches represent clusters, while genes connected by longer branches represent genes that are not clustered together. The points in multidimensional space by Euclidean metrics may also be used to generate dendrograms.

In yet another embodiment, divisive clustering methods may be used. For example, vectors are assigned to each gene's expression pattern, and two random vectors are generated. Each gene is then assigned to one of the two random vectors on the basis of probability of matching that vector. The random vectors are iteratively recalculated to generate two centroids that split the genes into two groups. This split forms the major branch at the bottom of a dendrogram. Each group is then further split in the same manner, ultimately yielding a fully branched dendrogram.

In a further embodiment, self-organizing maps (SOM) may be used to generate clusters. In general, the gene expression patterns are plotted in n-dimensional space, using a metric such as the Euclidean metrics described above. A grid of centroids is then placed onto the n-dimensional space and the centroids are allowed to migrate towards clusters of points, representing clusters of gene expression. Finally the centroids represent a gene expression pattern that is a sort of average of a gene cluster. In certain embodiments, SOM may be used to generate centroids, and the genes clustered at each centroid may be further represented by a dendrogram. An exemplary method is described in Tamayo et al., 1999, *PNAS* 96:2907-12. Once centroids are formed, correlation must be evaluated by one of the methods described supra.

In another aspect, the invention provides probe sets. Preferred probe sets are designed to detect expression of multiple genes and provide information about the status of a graft. Preferred probe sets of the invention comprise probes that are useful for the detection of at least two genes belonging to gene clusters A, B, C, D, E, F or G. Particularly preferred probe sets will comprise probes useful for the detection of at least three, at least four or at least five genes belonging to gene clusters A, B, C, D, E, F or G. Certain probe sets may additionally comprise probes that are useful for the detection of one or more genes of gene cluster H. Probe sets of the invention do not comprise probes useful for the detection of more than 10,000 gene transcripts, and preferred probe sets will comprise probes useful for the detection of fewer than 4000, fewer than 1000, fewer than 200, and most preferably fewer than 50 gene transcripts. Probe sets of the invention are particularly useful because they are smaller and cheaper than probe sets that are intended to detect as many genes as possible in a particular genome. The probe sets of the invention are targeted at the detection of gene transcripts that are informative about transplant status. Probe sets of the invention may comprise a large or small number of probes that detect gene transcripts that are not informative about transplant status. Such probes are useful as controls and for normalization. Probe sets may be a dry mixture or a mixture in solution. In preferred embodiments, probe sets of the invention are affixed to a solid substrate to form an array of probes. It is anticipated that probe sets may also be useful for multiplex PCR.

The present invention provides wild-type and mutant CYP2D6 polypeptides which are isolated from, or otherwise substantially free of other cellular proteins. The term "substantially free of other cellular proteins" (also referred to herein as "contaminating proteins") or "substantially pure or purified preparations" are defined as encompassing preparations of CYP2D6 polypeptides having less than about 20% (by dry weight) contaminating protein, and preferably having less than about 5% contaminating protein. Functional forms of the subject polypeptides can be prepared, for the first time, as purified preparations by using a cloned gene as described herein.

Preferred CYP2D6 proteins of the invention have an amino acid sequence which is at least about 60%, 65%, 66%, 67%, 68%, 69%, 70%, 71%, 72%, 73%, 74%, 75%, 76%, 77%, 78%, 79%, 80%, 85%, 90%, or 95% identical or homologous to an amino acid sequence of the CYP2D6 P450 polypeptide. Even more preferred CYP2D6 proteins comprise an amino acid sequence of at least 10, 20, 30, or 50 residues which is at least about 70, 80, 90, 95, 97, 98, or 99% homologous or identical to an amino acid sequence of CYP2D6. Such proteins can be recombinant proteins, and can be, e.g., produced in vitro from nucleic acids comprising a nucleotide sequence set forth in SEQ ID Nos. 1,2, 3, or 4 or another nucleic acid SEQ ID No. of the invention or homologs thereof. For example, recombinant polypeptides preferred by the present invention can be encoded by a nucleic acid, which is at least 85% homologous and more preferably 90% homologous and most preferably 95% homologous with a nucleotide sequence set forth in a SEQ ID Nos. of the invention. Polypeptides which are encoded by a nucleic acid that is at least about 98-99% homologous with the sequence of SEQ ID No. of the invention are also within the scope of the invention.

In a preferred embodiment, a CYP2D6 protein of the present invention is a mammalia CYP2D6 protein. In a particularly preferred embodiment the CYP2D6 polypeptide includes a polypeptide segment of the carboxy-terminal segment of the wild type 2D6 P450 protein sequence RRACLGEPLARMELFLFFTSLL QHFSFSVPTGQPRPSHHGVFAFLVSPSPYELC-AVPR (SEQ ID NO. 32) or the CYP2D6 C5816TA mutant 2D6 P450 carboxy-terminal sequence RACLGEPLARMELFLFFTSLLQHFSFSVPTGQPRPSHHGVFAFLVSPSPYEYLOCA PLEWGT (SEQ ID NO. 34). In particularly preferred embodiments, a CYP2D6 protein has a CYP2D6 bioactivity, such as a monoxygenase activity. It will be understood that certain post-translational modifications, e.g., phosphorylation and the like, can increase the apparent molecular weight of the CYP2D6 protein relative to the unmodified polypeptide chain.

The invention also provides protein isoforms encoded by splice variants of the present invention. Such isoforms may have biological activities identical to or different from those possessed by the CYP2D6 proteins encoded by SEQ ID NOs. 1-4. Such isoforms may arise, for example, by alternative splicing of one or more CYP2D6 gene transcripts.

CYP2D6 polypeptides preferably are capable of functioning as either an agonist or antagonist of at least one biological activity of a wild-type ("authentic") CYP2D6 protein of the appended sequence listing.Full length proteins or fragments corresponding to one or more particular motifs and/or domains or to arbitrary sizes, for example, at least 5, 10, 20, 25, 50, 75 and 100, amino acids in length are within the scope of the present invention.

For example, isolated CYP2D6 polypeptides can be encoded by all or a portion of a nucleic acid sequence shown in any of SEQ ID NOs. 1, 2, 3 or 4. Isolated peptidyl portions of CYP2D6 proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a CYP2D6 polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g., "authentic") CYP2D6 protein.

A CYP2D6 polypeptide can be a membrane bound form or a soluble form. A preferred soluble CYP2D6 polypeptide is a polypeptide which does not contain a hydrophobic signal sequence domain. Such proteins can be created by genetic engineering by methods known in the art. The solubility of a recombinant polypeptide may be increased by deletion of hydrophobic domains, such as predicted transmembrane domains, of the wild type protein.

In general, polypeptides referred to herein as having an activity (e.g., are "bioactive") of a CYP2D6 protein are defined as polypeptides which include an amino acid sequence encoded by all or a portion of the nucleic acid sequences shown in one of SEQ ID No. 1, 2, 3 or 4 and which mimic or antagonize all or a portion of the blological/biochemlcal activities of a naturally occurring CYP2D6 protein. Examples of such biological activity include a region of conserved structure such as the CYP2D6 carboxy-terminal conserved domain (see FIGURE 6A, CYP2D6 SEQ ID NO. 32).

Other biological activities of the subject CYP2D6 proteins will be reasonably apparent to those skilled in the art based upon the present description. According to the present invention, a polypeptide has biological activity if it is a specific agonist or antagonist of a naturally-occurring form of a CYP2D6 protein.

Other preferred proteins of the invention are those encoded by the nucleic acids set forth in the section pertaining to nucleic acids of the invention. In particular, the invention provides fusion proteins, e.g., CYP2D6 -immunoglobulin fusion proteins. Such fusion proteins can provide, e.g., enhanced stability and solubility of CYP2D6 proteins and may thus be useful in therapy. Fusion proteins can also be used to produce an immunogenic fragment of a CYP2D6 protein. For example, the VP6 capsid protein of rotavirus can be used as an immunologic carrier protein for portions of the CYP2D6 polypeptide, either in the monomeric form or in the form of a viral particle. The nucleic acid sequences corresponding to the portion of a subject CYP2D6 protein to which antibodies are to be raised can be incorporated into a fusion gene construct which includes coding sequences for a late vaccinia virus structural protein to produce a set of recombinant viruses expressing fusion proteins comprising CYP2D6 epitopes as part of the virion. It has been demonstrated with the use of immunogenic fusion proteins utilizing the Hepatitis B surface antigen fusion proteins that recombinant Hepatitis B virions can be utilized in this role as well. Similarly, chimeric constructs coding for fusion proteins containing a portion of a CYP2D6 protein and the poliovirus capsid protein can be created to enhance immunogenicity of the set of polypeptide antigens (see, for example, EP Publication No: 0259149; and Evans et al. (1989) *Nature* 339:385; Huang et al. (1988) *J. Virol.* 62:3855; and Schlienger et al. (1992) *J*. *Virol.* 66:2).

The Multiple antigen peptide system for peptide-based immunization can also be utilized to generate an immunogen, wherein a desired portion of a CYP2D6 polypeptide is obtained directly from organo-chemical synthesis of the peptide onto an oligomeric branching lysine core (see, for example, Posnett et al. (1988) *JBC* 263:1719 and Nardelli et al. (1992) *J*. *Immunol.* 148:914). Antigenic determinants of CYP2D6 proteins can also be expressed and presented by bacterial cells.

In addition to utilizing fusion proteins to enhance immunogenicity, it is widely appreciated that fusion proteins can also facilitate the expression of proteins, and accordingly, can be used in the expression of the CYP2D6 polypeptides of the present invention. For example, CYP2D6 polypeptides can be generated as glutathione-S-transferase (GST-fusion) proteins. Such GST-fusion proteins can enable easy purification of the CYP2D6 polypeptide, as for example by the use of glutathione-derivatized matrices (see, for example, Current Protocols in Molecular Biology, eds. Ausubel et al. (N.Y.: John Wiley & Sons, 1991)). Additionally, fusion of CYP2D6 polypeptides to small epitope tags, such as the FLAG or hemagluttinin tag sequences, can be used to simplify immunological purification of the resulting recombinant polypeptide or to facilitate immunological detection in a cell or tissue sample. Fusion to the green fluorescent protein, and recombinant versions thereof which are known in the art and available commercially, may further be used to localize CYP2D6 polypeptides within living cells and tissue.

The present invention further pertains to methods of producing the subject CYP2D6 polypeptides. For example, a host cell transfected with a nucleic acid vector directing expression of a nucleotide sequence encoding the subject polypeptides can be cultured under appropriate conditions to allow expression of the peptide to occur. Suitable media for cell culture are well known in the art. The recombinant CYP2D6 polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant CYP2D6 polypeptide is a fusion protein containing a domain which facilitates its purification, such as GST fusion protein.

Moreover, it will be generally appreciated that, under certain circumstances, it may be advantageous to provide homologs of one of the subject CYP2D6 polypeptides which function in a limited capacity as one of either a CYP2D6 agonist (mimetic) or a CYP2D6 antagonist, in order to promote or inhibit only a subset of the biological activities of the naturally-occurring form of the protein. Thus, specific biological effects can be elicited by treatment with a homolog of limited function, and with fewer side effects relative to treatment with agonists or antagonists which are directed to all of the biological activities of naturally occurring forms of CYP2D6 proteins.

Homologs of each of the subject CYP2D6 proteins can be generated by mutagenesis, such as by discrete point mutation(s), or by truncation. For instance, mutation can give rise to homologs which retain substantially the same, or merely a subset, of the biological activity of the CYP2D6 polypeptide from which it was derived. Alternatively, antagonistic forms of the protein can be generated which are able to inhibit the function of the naturally occurring form of the protein, such as by competitively binding to a CYP2D6 receptor.

The recombinant CYP2D6 polypeptides of the present invention also include homologs of the wildtype CYP2D6 proteins, such as versions of those protein which are resistant to proteolytic cleavage, as for example, due to mutations which alter ubiquitination or other enzymatic targeting associated with the protein.

CYP2D6 polypeptides may also be chemically modified to create CYP2D6 derivatives by forming covalent or aggregate conjugates with other chemical moieties, such as glycosyl groups, lipids, phosphate, acetyl groups and the like. Covalent derivatives of CYP2D6 proteins can be prepared by linking the chemical moieties to functional groups on amino acid sidechains of the protein or at the N-terminus or at the C-terminus of the polypeptide.

Modification of the structure of the subject CYP2D6 polypeptides can be for such purposes as enhancing therapeutic or prophylactic efficacy, stability (e.g., ex vivo shelf life and resistance to proteolytic degradation), or post-translational modifications (e.g., to alter phosphorylation pattern of protein). Such modified peptides, when designed to retain at least one activity of the naturally-occurring form of the protein, or to produce specific antagonists thereof, are considered functional equivalents of the CYP2D6 polypeptides described in more detail herein. Such modified peptides can be produced, for instance, by amino acid substitution, deletion, or addition. The substitutional variant may be a substituted conserved amino acid or a substituted non-conserved amino acid.

For example, it is reasonable to expect that an isolated replacement of a leucine with an isoleucine or valine, an aspartate with a glutamate, a threonine with a serine, or a similar replacement of an amino acid with a structurally related amino acid (i.e. isosteric and/or isoelectric mutations) will not have a major effect on the biological activity of the resulting molecule. Conservative replacements are those that take place within a family of amino acids that are related in their side chains. Genetically encoded amino acids can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur -containing = cysteine and methionine. (see, for example, Biochemistry, 2^{nd} ed., Ed. by L. Stryer, WH Freeman and Co.: 1981). Whether a change in the amino acid sequence of a peptide results in a functional CYP2D6 homolog (e.g., functional in the sense that the resulting polypeptide mimics or antagonizes the wild-type form) can be readily determined by assessing the ability of the variant peptide to produce a response in cells in a fashion similar to the wild-type protein, or competitively inhibit such a response. Polypeptides in which more than one replacement has taken place can readily be tested in the same manner.

This invention further contemplates a method for generating sets of combinatorial mutants of the subject CYP2D6 proteins as well as truncation mutants, and is especially useful for identifying potential variant sequences (e.g., homologs). The purpose of screening such combinatorial libraries is to generate, for example, novel CYP2D6 homologs which can act as, e.g., agonists or antagonist, or alternatively, possess novel activities all together. Thus, combinatorially-derived homologs can be generated to have an increased potency relative to a naturally occurring form of the protein.

In one embodiment, the variegated CYP2D6 libary of CYP2D6 variants is generated by combinatorial mutagenesis at the nucleic acid level, and is encoded by a variegated gene CYP2D6 library. For instance, a mixture of synthetic oligonucleotides can be enzymatically ligated into gene sequences such that the degenerate set of potential CYP2D6 sequences are expressible as individual polypeptides, or alternatively, as a set of larger fusion proteins (e.g., for phage display) containing the set of CYP2D6 sequences therein.

There are many ways by which such libraries of potential CYP2D6 homologs can be generated from a degenerate oligonucleotide sequence. Chemical synthesis of a degenerate gene sequence can be carried out in an automatic DNA synthesizer, and the synthetic genes then ligated into an appropriate expression vector. The purpose of a degenerate set of genes is to provide, in one mixture, all of the sequences encoding the desired set of potential CYP2D6 sequences. The synthesis of degenerate oligonucleotides is well known in the art (see for example, Narang, SA (1983) *Tetrahedron* 39:3; Itakura et al. (1981) Recombinant DNA, Proc 3^{rd} Cleveland Sympos. Macromolecules, ed. AG Walton, Amsterdam: Elsevier pp 273-289; Itakura et al. (1984) *Annu. Rev. Biochem.* 53:323; Itakura et al. (1984) *Science* 198:1056; Ike et al. (1983) *Nucleic Acid Res.* 11:477. Such techniques have been employed in the directed evolution of other proteins (see, for example, Scott et al. (1990) *Science* 249:386-390; Roberts et al. (1992) *PNAS* 89:2429-2433; Devlin et al. (1990) *Science* 249: 404-406; Cwirla et al. (1990) *PNAS* 87: 6378-6382; as well as U.S. Patents Nos. 5,223,409, 5,198,346, and 5,096,815).

Likewise, a library of coding sequence fragments can be provided for a CYP2D6 clone in order to generate a variegated population of CYP2D6 fragments for screening and subsequent selection of bioactive fragments. A variety of techniques are known in the art for generating such libraries, including chemical synthesis. In one embodiment, a library of coding sequence fragments can be generated by (i) treating a double stranded PCR fragment of a CYP2D6 coding sequence with a nuclease under conditions wherein nicking occurs only about once per molecule; (ii) denaturing the double stranded DNA; (iii) renaturing the DNA to form double stranded DNA which can include sense/antisense pairs from different nicked products; (iv) removing single stranded portions from reformed duplexes by treatment with S1 nuclease; and (v) ligating the resulting fragment library into an expression vector. By this exemplary method, an expression library can be derived which codes for N-terminal, C-terminal and internal fragments of various sizes.

A wide range of techniques are known in the art for screening gene products of combinatorial libraries made by point mutations or truncation, and for screening cDNA libraries for gene products having a certain property. Such techniques will be generally adaptable for rapid screening of the gene libraries generated by the combinatorial mutagenesis of CYP2D6 homologs. The most widely used techniques for screening large gene libraries typically comprises cloning the gene library into replicable expression vectors, transforming appropriate cells with the resulting libraries of vectors, and expressing the combinatorial genes under conditions in which detection of a desired activity facilitates relatively easy isolation of the vector encoding the gene whose product was detected. Each of the illustrative assays described below are amenable to high through-put analysis as necessary to screen large numbers of degenerate CYP2D6 sequences created by combinatorial mutagenesis techniques. Combinatorial mutagenesis has a potential to generate very large libraries of mutant proteins, e.g., in the order of 1026 molecules. Combinatorial libraries of this size may be technically challenging to screen even with high throughput screening assays. To overcome this problem, a new technique has been developed recently, recrusive ensemble mutagenesis (REM), which allows one to avoid the very high proportion of non-functional proteins in a random library and simply enhances the frequency of functional proteins, thus decreasing the complexity required to achieve a useful sampling of sequence space. REM is an algorithm which enhances the frequency of functional mutants in a library when an appropriate selection or screening method is employed (Arkin and Yourvan, 1992, *PNAS USA* 89:7811-7815; Yourvan et al., 1992, Parallel Problem Solving from Nature, 2., In Maenner and Manderick, eds., Elsevir Publishing Co., Amsterdam, pp. 401-410; Delgrave et al., 1993, Protein Engineering 6(3):327-331).

The invention also provides for reduction of the CYP2D6 proteins to generate mimetics, e.g., peptide or non-peptide agents, such as small molecules, which are able to disrupt binding of a CYP2D6 polypeptide of the present invention with a molecule, e.g. target peptide. Thus, such mutagenic techniques as described above are also useful to map the determinants of the CYP2D6 proteins which participate in protein-protein interactions involved in, for example, binding of the subject CYP2D6 polypeptide to a target peptide. To illustrate, the critical residues of a subject CYP2D6 polypeptide which are involved in molecular recognition of its receptor can be determined and used to generate CYP2D6 derived peptidomimetics or small molecules which competitively inhibit binding of the authentic CYP2D6 protein with that moiety. By employing, for example, scanning mutagenesis to map the amino acid residues of the subject CYP2D6 proteins which are involved in binding other proteins, peptidomimetic compounds can be generated which mimic those residues of the CYP2D6 protein which facilitate the interaction. Such mimetics may then be used to interfere with the normal function of a CYP2D6 protein. For instance, non-hydrolyzable peptide analogs of such residues can be generated using benzodiazepine (e.g., see Freidinger et al. in Peptides: Chemistry and Biology, G.R. Marshall ed., ESCOM Publisher: Leiden, Netherlands, 1988), azepine (e.g., see Huffman et al. in Peptides: Chemistry and Biology, G.R. Marshall ed., ESCOM Publisher: Leiden, Netherlands, 1988), substituted gamma lactam rings (Garvey et al. in Peptides: Chemistry and Biology, G.R. Marshall ed., ESCOM Publisher: Leiden, Netherlands, 1988), keto-methylene pseudopeptides (Ewenson et al. (1986) *J Med Chem* 29:295; and Ewenson et al. in Peptides: Structure and Function (Proceedings of the 9^{th} American Peptide Symposium) Pierce Chemical Co. Rockland, IL, 1985), b-turn dipeptide cores (Nagai et al. (1985) *Tetrahedron Lett* 26:647; and Sato et al. (1986) *J Chem Soc Perkin Trans* 1:1231), and b-aminoalcohols (Gordon et al. (1985) *Biochem Biophys Res Commun* 126:419; and Dann et al. (1986) *Biochem Biophys Res Commun* 134:71).

Another aspect of the invention pertains to an antibody specifically reactive with a mammalia CYP2D6 protein, e.g., a wild-type or mutated CYP2D6 protein, particularly to the wild-type (SEQ ID NO. 5) and mutant (SEQ ID NO. 6, 8, or 30) CYP2D6 P450 carboxy-terminal sequences shown in FIGURE 6. For example, by using immunogens derived from a CYP2D6 protein, e.g., based on the cDNA sequences, anti-protein/anti-peptide antisera or monoclonal antibodies can be made by standard protocols (See, for example, Antibodies: A Laboratory Manual ed. by Harlow and Lane (Cold Spring Harbor Press: 1988)). A mammal, such as a mouse, a hamster or rabbit can be immunized with an immunogenic form of the peptide (e.g., a mammalia CYP2D6 polypeptide or an antigenic fragment which is capable of eliciting an antibody response, or a fusion protein as described above). Techniques for conferring immunogenicity on a protein or peptide include conjugation to carriers or other techniques well known in the art. An immunogenic portion of a CYP2D6 protein can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the subject antibodies are immunospecific for antigenic determinants of a CYP2D6 protein of a mammal, e.g., antigenic determinants of a protein set forth in SEQ ID No. 6 or 8 or closely related homologs (e.g., at least 90% homologous, and more preferably at least 94% homologous).

Following immunization of an animal with an antigenic preparation of a CYP2D6 polypeptide, anti-CYP2D6 antisera can be obtained and, if desired, polyclonal anti-CYP2D6 antibodies isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique originally developed by Kohler and Milstein ((1975) *Nature,* 256: 495-497), the human B cell hybridoma technique (Kozbar et al., (1983) *Immunology Today , 4:* 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a mammalia CYP2D6 polypeptide of the present invention and monoclonal antibodies isolated from a culture comprising such hybridoma cells. In one embodiment anti-human CYP2D6 antibodies specifically react with the protein encoded by a nucleic acid having SEQ ID NOs. 1, 2, 3 or 4.

The term antibody as used herein is intended to include fragments thereof which are also specifically reactive with one of the subject mammalia CYP2D6 polypeptides. Antibodies can be fragmented using conventional techniques and the fragments screened for utility in the same manner as described above for whole antibodies. For example, F(ab)2 fragments can be generated by treating antibody with pepsin. The resulting F(ab)2 fragment can be treated to reduce disulfide bridges to produce Fab fragments. The antibody of the present invention is further intended to include bispecific, single-chain, and chimeric and humanized molecules having affinity for a CYP2D6 protein conferred by at least one CDR region of the antibody. In preferred embodiments, the antibody further comprises a label attached thereto and able to be detected, (e.g., the label can be a radioisotope, fluorescent compound, enzyme or enzyme co-factor).

Anti-CYP2D6 antibodies can be used, e.g., to monitor CYP2D6 protein levels in an individual for determining, e.g., whether a subject has a disease or condition associated with an aberrant CYP2D6 protein level, or allowing determination of the efficacy of a given treatment regimen for an individual afflicted with such a disorder. The level of CYP2D6 polypeptides may be measured from cells in bodily fluid, such as in blood samples.

Another application of anti-CYP2D6 antibodies of the present invention is in the immunological screening of cDNA libraries constructed in expression vectors such as λ gt11, λ gt18-23, λ ZAP, and λ ORF8. Messenger libraries of this type, having coding sequences inserted in the correct reading frame and orientation, can produce fusion proteins. For instance, λ gt11 will produce fusion proteins whose amino termini consist of β-galactosidase amino acid sequences and whose carboxy termini consist of a foreign polypeptide. Antigenic epitopes of a CYP2D6 protein, e.g., other orthologs of a particular CYP2D6 protein or other paralogs from the same species, can then be detected with antibodies, as, for example, reacting nitrocellulose filters lifted from infected plates with anti-CYP2D6 antibodies. Positive phage detected by this assay can then be isolated from the infected plate. Thus, the presence of CYP2D6 homologs can be detected and cloned from other animals, as can alternate isoforms (including splice variants) from humans.

The invention further provides for transgenic animals. Transgenic animals of the invention include non-human animals containing an heterologous CYP2D6 P450 C(5816)TA variant or fragment thereof under the control of an CYP2D6 promoter or under the control of a heterologous promoter. Accordingly, the transgenic animals of the invention can be animals expressing a transgene encoding a polymorphic variant of the CYP2D6 gene. Such transgenic animals, preferably, carry at least one heterologous replacement of the wild-type CYP2D6 P450 locus with an heterologous CYP2D6 P450 C(5816)TA variant allele. Such animals can be used, e.g., to determine the effects of such variants on drug metabolism. Other non-human animals within the scope of the invention include those in which at least one copy of the endogenous CYP2D6 P450 gene has been mutated or "knocked out". For example, a CYP2D6 P450 C(5816)TA variant allele/ CYP2D6 P450 knock-out mutant animal having a poor metabolizer phenotype could be constructed and used to determine whether particular treatments, such as with a CYP2D6 P450 drug agonist compound or candidate, are capable of rescuing the poor metabolizer phenotype resulting from a CYP2D6 P450 C(5816)TA mutation. Furthermore, these knock-out animals can be crossed with other transgenic animals expressing, e.g., a mutated form of another P450 gene, thus resulting in an animal which express multiple mutated P450 protein resulting in a complex poor metabolizer phenotype. Methods for obtaining transgenic and knockout non-human animals are well known in the art.

Knowledge of the particular alteration or alterations, resulting in defective or deficient CYP2D6 genes or proteins in an individual (the CYP2D6 genetic profile), alone or in conjunction with information on other genetic defects contributing to the same disease (the genetic profile of the particular disease) allows a customization of the therapy for a particular disease to the individual's genetic profile, the goal of "pharmacogenomics". The major route of phase I drug metabolism is oxidation by cytochrome P-450 (CYP). Most clinically used drugs are metabolized to some degree by P450s. These enzymes are also principally responsible for activation of procarcinogens and promutagens. Debrisoquine 4-hydroxylase (CYP2D6 ) is the most well characterized P450 polymorphism. About 25% of prescribed drugs are metabolized by CYP2D6. The CYP2D6 C5816TA polymorphism appears to have clinical consequences in the use of cardiovascular drugs and drugs used for treatment of psychiatric disorders. Genotype has been shown to closely correlate with phenotype in this and other CYP2D6 mutations which have been examined. Subjects having a specific allele of a CYP2D6 gene may or may not exhibit symptoms of a drug sensitivity or be predisposed of developing symptoms of a particular disease, such as cancer resulting from the inability to adequately metabolize environmental mutagens or carcinogens. Further, if those subjects are symptomatic, they may or may not respond to a certain drug, e.g., a specific CYP2D6 therapeutic, but may respond to another. Thus, generation of a CYP2D6 genetic profile, (e.g., categorization of alterations in CYP2D6 genes which are associated with the development of a particular disease), from a population of subjects, who are symptomatic for a disease or condition that is caused by or contributed to by a defective and/or deficient CYP2D6 gene and/or protein (a CYP2D6 genetic population profile) and comparison of an individual's CYP2D6 profile to the population profile, permits the selection or design of drugs that are expected to be efficacious for a particular patient or patient population (i.e., a group of patients having the same genetic alteration).

For example, a CYP2D6 C5816TA population profile can be performed, by determining the CYP2D6 profile, e.g., the identity of a CYP2D6 C5816TA mutant gene in a patient population having a disease, which is caused by or contributed to by a defective or deficient CYP2D6 gene. Optionally, the CYP2D6 population profile can further include information relating to the response of the population to a CYP2D6 therapeutic, using any of a variety of methods, including, monitoring: 1) the severity of symptoms associated with the CYP2D6 related disease, 2) CYP2D6 gene expression level, 3) CYP2D6 mRNA level, and/or 4) CYP2D6 protein level. and (iii) dividing or categorizing the population based on the particular genetic alteration or alterations present in its CYP2D6 gene or a CYP2D6 pathway gene. The CYP2D6 genetic population profile can also, optionally, indicate those particular alterations in which the patient was either responsive or non-responsive to a particular therapeutic. This information or population profile, is then useful for predicting which individuals should respond to particular drugs, based on their individual CYP2D6 profile. In another embodiment, the CYP2D6 profile is a transcriptional or expression level profile and step (i) is comprised of determining the expression level of CYP2D6 proteins, alone or in conjunction with the expression level of other genes, known to contribute to the same disease. The CYP2D6 profile can be measured in many patients at various stages of the disease. Pharmacogenomic studies can also be performed using transgenic animals. For example, one can produce transgenic mice, e.g., as described herein, which contain a specific allelic variant of a CYP2D6 gene. These mice can be created, e.g, by replacing their wild-type CYP2D6 gene with an allele of the human CYP2D6 gene. The response of these mice to specific CYP2D6 therapeutics can then be determined.

The present invention is illustrated by the following examples. The foregoing and following description of the present invention and the various embodiments are not intended to be limiting of the invention but rather are illustrative thereof. Hence, it will be understood that the invention is not limited to the specific details of these examples.

### EXAMPLES

### Example 1

### METHOD FOR DETECTING A GENETIC DEFICIENCY FOR DRUG METABOLISM ( CYP2D6 POOR METABOLIZER GENOTYPE)

We utilized primers and methods of the invention to detect a genetic deficiency in a subject for metabolizing drugs, i.e., a CYP2D6 poor metabolizer genotype, who had previously been shown to have a poor metabolizer phenotype. Genomic DNA from the subject identified as a poor metabolizer was isolated using Qiagen's QiaAMP Blood isolation kit according to manufacturer's protocol. Genomic DNA was extracted from 200 ul of whole blood. Amplification of the CYP2D6 locus was achieved in two steps. First, an initial amplification of the entire CYP2D6 gene to prevent amplification of CYP2D6 pseudogene in subsequent PCR.

The primers and the associated methods used were based upon Johansson et al. (Johansson, Lundqvist, Dahl, and Ingelman-Sundberg (1996) "PCR-based genotyping for duplicated and deleted CYP2D6 genes", Pharmacogenetics 6, 351-355).

The following PCR protocol was utilized:
Initial Amplification
PCR:
Lower Mix: Per reaction
11.8 ul of water
12.0 ul of 3.3X XL Buffer II
10.0 ul of 2mM dNTPs
0.1 ul of 100 uM primer 35791-81 (CCAGAAGGCTTTGCAGGCTTCA)
0.1 ul of 100 uM primer 35791-82 (ACTGAGCCCTGGGAGGTAGGTA)
6.0 ul of 25 mM Mg(OAc)₂
40 ul

The PCR reaction mixture was subsequently sealed by adding one Ampliwax gem over this mixture in a PCR tube. The reaction mixture was heated to 80°C for 5 minutes, and then cooled to 25°C for 5 minutes. The following upper reaction mix was then added:
Upper Mix: Per reaction
30 ul of water
18 ul of 3.3X XL buffer II
2 ul of rRth, XL
10 ul of genomic DNA (sample 9070; study # 161-003)
100 ul total volume

The PCR reaction was performed using Perkin Elmer 9600 machine programmed as follows:
94°C for 1 minute
94°C for 15 seconds
62°C for 5 minutes (repeat steps 2 and 3 for 25 cycles)
94°C for 15 seconds
62°C for 5 minutes + autoextend for 15 seconds (repeat steps 4 and 5 for 10 cycles)
72°C for 10 minutes
4°C hold

This initial amplification product then serves as the template for subsequent amplification of each exon of the CYP2D6 gene. While each exon was amplified and sequenced, only the method and reagents for Exon 9, the exon in which the mutation was detected, are described below:

Nested PCR methodology was used to amplify CYP2D6 Exon 9 using M13-tagged primers as follows:
PCR:
10 ul 10 X PCR Buffer
10 ul 25mM MgCl₂
2 ul each 10-mM dNTP
2 ul 10 uM 35791-11 forward primer (*TGTAAAACGACGGCCAGT*-AGCCAGGCTCACTGA)
2ul 10 uM 35791-12 reverse primer (*CAGGAAACAGCTATGACC*-TGATCCCAACGAGGGCGTGAGCAG)
0.5 ul AmpliTaq Gold 5U/ul
62.5 ul sterile water
5.0 ul of 1:5 dilution of PCR product from initial PCR above
100 ul

The PCR reaction was performed using Perkin Elmer 9600 machine programmed as follows:
95°C for 10 minutes
95°C for 30 seconds
63°C for 45 minutes
72°C for 1 minute (repeat steps 2 to 4 for 25 cycles)
4°C hold

The amplified product was sequenced as follows:

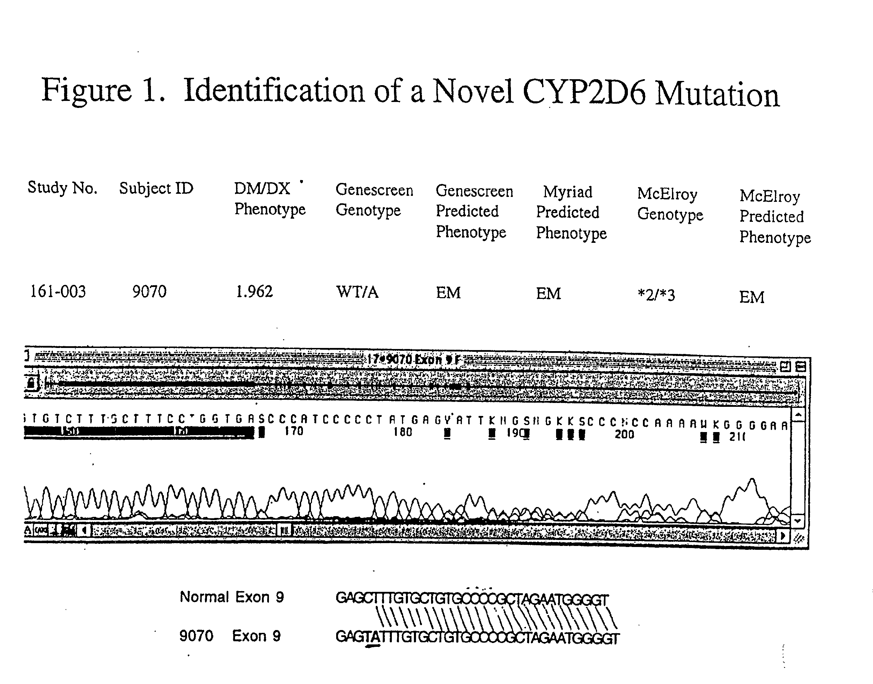

First, 100 ul of the resulting PCR product was purified using Qiagen's PCR purification kit and the DNA was eluted in 50 ul of water and diluted to 10 ng/ul. Second, sequence reactions were performed using dye-primer chemistry with M13 forward and M13-reverse primers. These and equivalent sequencing methods are known in the art. The CYP2D6 poor metabolizer genotype containing the C(5816)TA variant detected is shown in Figure 1.

### Primer Sequences:

### Initial Amplification of the CYP2D6 Gene Locus

35791-81: CCAGAAGGCTTTGCAGGCTTCA
35791-82: ACTGAGCCCTGGGAGGTAGGTA

### Sequencing Primers for Exon 9

35791-11 *TGTAAAACGACGGCCAGT* AGCCAGGCTCACTGA
35791-12 *CAGGAAACAGCTATGACC* TGATCCCAACGAGGGCGTGAGCAG

### Example 2

### EXPRESSION DATA

The CYP2D6 mutations of the invention were cloned into a eukaryotic CMV-CYP2D6 expression vector to determine the biological activity associated with each mutation identified. Individual constructs included: the CYP2D6 with the G5799C mutation, CYP2D6 with the C to TA frameshift at position 5816, and a construct which included both mutations together. Following transfection of these constructs into mammalian cells, as well as the wild type construct, CYP2D6 protein was evident based on Western blot analysis of the cell extracts. In comparison to the wild type construct the CYP2D6 activity was evident, however the turnover time was extremely rapid with the double mutant suggesting that the stability of the protein was affected by this frameshift.

Those skilled in the art will recognize, or be able to ascertain using no more than routine experimentation, many equivalents of the specific embodiments of the invention described herein. Such equivalents are intended to be encompassed by the specification and the attendant claims.

## Claims

1. An isolated nucleic acid molecule comprising: (a) a sequence of at least 20 consecutive nucleotides of an allele of a CYP2D6 gene having SEQ ID NO: 1, which sequence comprises a polymorphic region selected from the group consisting of nucleotide 5816, and nucleotide 5799 of SEQ ID NO: 1, wherein said polymorphic region comprises a nucleotide sequence which differs from that in SEQ ID NO: 1; or (b) a complement of the sequence in (a).

2. An isolated nucleic acid molecule comprising at least 20 contiguous nucleotides of SEQ ID NO: 3, including nucleotide 1474, wherein C is replaced by TA, or the complement thereof.

3. An isolated nucleic acid molecule comprising at least 30 contiguous nucleotides of SEQ ID NO: 3, including: (a) nucleotide 1474 wherein C is replaced by TA; (b) nucleotide 1457 wherein G is replaced by C; (c) both (a) and (b); or (d) the complement of (a), (b), or (c).

4. An isolated nucleic acid molecule encoding a polypeptide comprising an amino acid sequence selected from the group comprising SEQ ID NO.6, SEQ ID NO. 8 and SEQ ID No. 30.

5. A single-stranded DNA probe that hybridizes under stringent conditions to a variant form of the CYP2D6 gene having SEQ ID NO. 1, wherein said variant is selected from the group consisting of: (a) SEQ ID No: 1 having TA at position 5816; (b) SEQ ID No: 1 having C at position 5799; (c) SEQ ID No: 1 having TA at position 5816 and C at position 5799; and (d) the complement of (a), (b), and (c).

6. A primer capable of amplifying the C5816TA allelic variant comprising a sequence of at least 10 consecutive nucleotides of SEQ ID NO. 2 or SEQ ID NO. 4, or complement thereof, and further comprising a 3' terminal nucleotide of at least one of the nucleotides, or complements thereof, selected from the group consisting of: the T at position 5816 of SEQ ID NO. 2; the T at position 1474 of SEQ ID NO. 4; the A at position 5817 of SEQ ID NO. 2; and the A at position 1475 of SEQ ID NO.4.

7. An allele specific oligonucleotide for the detection of the C5816TA allelic variant comprising a sequence of at least 10 consecutive nucleotides of SEQ ID NO. 2 or SEQ ID NO. 4, or complement thereof, and further comprising the nucleotide pair TA at position 5816-5817 of SEQ ID NO. 2 and nucleotide 1474 and 1475 of SEQ ID NO. 4, or complement thereof.

8. An array of nucleic acid molecules attached to a support, said array comprising an oligonucleotide that will hybridize under stringent conditions to a nucleic acid sequence as set forth in SEQ ID NO. 2, under conditions wherein said oligonucleotide will not hybridize to the nucleic acid sequence of SEQ ID No. 1.

9. An isolated polypeptide having residues 481-502 of the amino acid sequence set forth in SEQ ID NO. 8.

10. A purified antibody that selectively binds to an epitope comprising residues 481-502 of the amino acid sequence as set forth in SEQ ID NO. 6.

11. A purified antibody that selectively binds to a mutant CYP2D6 polypeptide having an amino acid sequence as set forth in SEQ ID NO. 8 but not to the wild-type CYP2D6 polypeptide having an amino acid sequence as set forth in SEQ ID NO. 5.

12. A method for determining whether a subject has a genetic deficiency for metabolizing a drug comprising determining the identity of the amino acids at the C-terminal end of the CYP2D6 protein, wherein the presence of an amino acid sequence other than SEQ ID No: 28 identifies a subject having a genetic deficiency.

13. A method for evaluating therapy with a drug metabolized by P450 CYP2D6 comprising: (a) obtaining a sample of DNA from an individual; (b) determining the identity of the nucleotide at position 5816 of the genomic sequence of CYP2D6; and (c) evaluating whether the individual should undergo therapy with a drug metabolized by P450 CYP2D6.

14. A method for determining whether a subject has a genetic deficiency for metabolizing a drug comprising: providing a sample of DNA from the individual; amplifying a segment of the CYP2D6 gene with primers capable of amplifying the C5816TA allelic variant of CYP2D6 exon 9; and detecting the presence of amplified DNA that codes for the C5816TA allelic variant, wherein the presence of amplified DNA that codes for the C5816TA allelic variant indicates that the subject has a genetic deficiency for metabolizing drugs.

15. A method for determining whether an individual is susceptible to being a PM of drugs comprising detecting the presence of a cytochrome P450 CYP2D6 gene C5816TA polymorphism by: providing a sample of cellular protein from the individual; and detecting the presence of a mutant CYP2D6 C5816TA polypeptide containing the carboxy-terminal sequence YLCCAPLEWGT in said sample with an antibody which recognizes an epitope of the YLCCAPLEWGT mutant carboxy-terminal sequence, wherein the presence of the mutant CYP2D6 C5816TA polypeptide containing the carboxy-terminal sequence YLCCAPLEWGT indicates that the individual is susceptible to being a PM of drugs.


SEQUENCE LISTING

<110>  Pfizer Products Inc.

<120>  Novel Varients Of The Human CYP2D6 Gene

<130>  PC11033AGPR

<150>  60/309,111
<151>  2001-07-31

<160>  38

<170>  PatentIn version 3.1

<210>  1
<211>  9432
<212>  DNA
<213>  HOMO SAPIENS

<400>  1
gaattcaaga ccagcctgga caacttggaa gaacccggtc tctacaaaaa atacaaaatt      60
agctgggatt gggtgcggtg gctcatgcct ataatcccag cactttggga gcctgaggtg     120
ggtggatcac ctgaagtcag gagttcaaga ctagcctggc caacatggtg aaaccctatc     180
tctactgaaa atacaaaaag ctagacgtgg tggcacacac ctgtaatccc agctacttag     240
gaggctgagg caggagaatt gcttgaagcc tagaggtgaa ggttgtagtg agccgagatt     300
gcatcattgc acaatggagg ggagccacca gcctgggcaa caagaggaaa tctccgtctc     360
caaaaaaaaa aaaaaaaaaa aaagaattag gctgggtggt gcctgtagtc ccagctactt     420
gggaggcagg gggtccactt gatgtcgaga ctgcagtgag ccatgatcct gccactgcac     480
tccggcctgg gcaacagagt gagaccctgt ctaaagaaaa aaaaaataaa gcaacatatc     540
ctgaacaaag gatcctccat aacgttccca ccagatttct aatcagaaac atggaggcca     600
gaaagcagtg gaggaggacg accctcaggc agcccgggag gatgttgtca caggctgggg     660
caagggcctt ccggctacca actgggagct ctgggaacag ccctgttgca aacaagaagc     720
catagcccgg ccagagccca ggaatgtggg ctgggctggg agcagcctct ggacaggagt     780
ggtcccatcc aggaaacctc cggcatggct gggaagtggg gtacttggtg ccgggtctgt     840

atgtgtgtgt gactggtgtg tgtgagagag aatgtgtgcc ctaagtgtca gtgtgagtct 900
gtgtatgtgt gaatattgtc tttgtgtggg tgattttctg cgtgtgtaat cgtgtccctg 960
caagtgtgaa caagtggaca agtgtctggg agtggacaag agatctgtgc accatcaggt 1020
gtgtgcatag cgtctgtgca tgtcaagagt gcaaggtgaa gtgaagggac caggcccatg 1080
atgccactca tcatcaggag ctctaaggcc ccaggtaagt gccagtgaca gataagggtg 1140
ctgaaggtca ctctggagtg ggcaggtggg ggtagggaaa gggcaaggcc atgttctgga 1200
ggaggggttg tgactacatt agggtgtatg agcctagctg ggaggtggat ggccgggtcc 1260
actgaaaccc tggttatccc agaaggcttt gcaggcttca ggagcttgga gtggggagag 1320
ggggtgactt ctccgaccag gcccctccac cggcctaccc tgggtaaggg cctggagcag 1380
gaagcagggg caagaacctc tggagcagcc catacccgcc ctggcctgac tctgccactg 1440
gcagcacagt caacacagca ggttcactca cagcagaggg caaaggccat catcagctcc 1500
ctttataagg gaagggtcac gcgctcggtg tgctgagagt gtcctgcctg gtcctctgtg 1560
cctggtgggg tgggggtgcc aggtgtgtcc agaggagccc atttggtagt gaggcaggta 1620
tggggctaga agcactggtg cccctggccg tgatagtggc catcttcctg ctcctggtgg 1680
acctgatgca ccggcgccaa cgctgggctg cacgctaccc accaggcccc ctgccactgc 1740
ccgggctggg caacctgctg catgtggact tccagaacac accatactgc ttcgaccagg 1800
tgagggagga ggtcctggag ggcggcagag gtgctgaggc tcccctacca gaagcaaaca 1860
tggatggtgg gtgaaaccac aggctggacc agaagccagg ctgagaaggg gaagcaggtt 1920
tgggggacgt cctggagaag ggcatttata catggcatga aggactggat tttccaaagg 1980
ccaaggaaga gtagggcaag ggcctggagg tggagctgga cttggcagtg ggcatgcaag 2040
cccattgggc aacatatgtt atggagtaca aagtcccttc tgctgacacc agaaggaaag 2100
gccttgggaa tggaagatga gttagtcctg agtgccgttt aaatcacgaa atcgaggatg 2160
aagggggtgc agtgacccgg ttcaaacctt ttgcactgtg ggtcctcggg cctcactgcc 2220
tcaccggcat ggaccatcat ctgggaatgg gatgctaact ggggcctctc ggcaattttg 2280
gtgactcttg caaggtcata cctgggtgac gcatccaaac tgagttcctc catcacagaa 2340
ggtgtgaccc ccacccccgc cccacgatca ggaggctggg tctcctcctt ccacctgctc 2400
actcctggta gccccggggg tcgtccaagg ttcaaatagg actaggacct gtagtctggg 2460
gtgatcctgg cttgacaaga ggccctgacc ctccctctgc agttgcggcg ccgcttcggg 2520
gacgtgttca gcctgcagct ggcctggacg ccggtggtcg tgctcaatgg gctggcggcc 2580
gtgcgcgagg cgctggtgac ccacggcgag gacaccgccg accgcccgcc tgtgcccatc 2640
acccagatcc tgggtttcgg gccgcgttcc caaggcaagc agcggtgggg acagagacag 2700
atttccgtgg gacccgggtg ggtgatgacc gtagtccgag ctgggcagag agggcgcggg 2760
gtcgtggaca tgaaacaggc cagcgagtgg ggacagcggg ccaagaaacc acctgcacta 2820
gggaggtgtg agcatgggga cgagggcggg gcttgtgacg agtgggcggg gccactgccg 2880

agacctggca ggagcccaat gggtgagcgt ggcgcatttc ccagctggaa tccggtgtcg 2940
aagtgggggc ggggaccgca cctgtgctgt aagctcagtg tgggtggcgc ggggcccgcg 3000
gggtcttccc tgagtgcaaa ggcggtcagg gtgggcagag acgaggtggg gcaaagcctg 3060
ccccagccaa gggagcaagg tggatgcaca aagagtgggc cctgtgacca gctggacaga 3120
gccagggact gcgggagacc agggggagca tagggttgga gtgggtggtg gatggtgggg 3180
ctaatgcctt catggccacg cgcacgtgcc cgtcccaccc ccaggggtgt tcctggcgcg 3240
ctatgggccc gcgtggcgcg agcagaggcg cttctccgtg tccaccttgc gcaacttggg 3300
cctgggcaag aagtcgctgg agcagtgggt gaccgaggag gccgcctgcc tttgtgccgc 3360
cttcgccaac cactccggtg ggtgatgggc agaagggcac aaagcgggaa ctgggaaggc 3420
gggggacggg gaaggcgacc ccttacccgc atctcccacc cccaggacgc ccctttcgcc 3480
ccaacggtct cttggacaaa gccgtgagca acgtgatcgc ctccctcacc tgcgggcgcc 3540
gcttcgagta cgacgaccct cgcttcctca ggctgctgga cctagctcag gagggactga 3600
aggaggagtc gggctttctg cgcgaggtgc ggagcgagag accgaggagt ctctgcaggg 3660
cgagctcccg agaggtgccg gggctggact ggggcctcgg aagagcagga tttgcataga 3720
tgggtttggg aaaggacatt ccaggagacc ccactgtaag aagggcctgg aggaggaggg 3780
gacatctcag acatggtcgt gggagaggtg tgcccgggtc agggggcacc aggagaggcc 3840
aaggactctg tacctcctat ccacgtcaga gatttcgatt ttaggtttct cctctgggca 3900
aggagagagg gtggaggctg gcacttgggg agggacttgg tgaggtcagt ggtaaggaca 3960
ggcaggccct gggtctacct ggagatggct ggggcctgag acttgtccag gtgaacgcag 4020
agcacaggag ggattgagac cccgttctgt ctggtgtagg tgctgaatgc tgtccccgtc 4080
ctcctgcata tcccagcgct ggctggcaag gtcctacgct tccaaaaggc tttcctgacc 4140
cagctggatg agctgctaac tgagcacagg atgacctggg acccagccca gcccccccga 4200
gacctgactg aggccttcct ggcagagatg gagaaggtga gagtggctgc cacggtgggg 4260
ggcaagggtg gtgggttgag cgtcccagga ggaatgaggg gaggctgggc aaaaggttgg 4320
accagtgcat cacccggcga gccgcatctg ggctgacagg tgcagaattg gaggtcattt 4380
gggggctacc ccgttctgtc ccgagtatgc tctcggccct gctcaggcca aggggaaccc 4440
tgagagcagc ttcaatgatg agaacctgcg catagtggtg gctgacctgt tctctgccgg 4500
gatggtgacc acctcgacca cgctggcctg gggcctcctg ctcatgatcc tacatccgga 4560
tgtgcagcgt gagcccatct gggaaacagt gcaggggccg agggaggaag ggtacaggcg 4620
ggggcccatg aactttgctg ggacacccgg ggctccaagc acaggcttga ccaggatcct 4680
gtaagcctga cctcctccaa cataggaggc aagaaggagt gtcagggccg gaccccctgg 4740
gtgctgaccc attgtgggga cgcatgtctg tccaggccgt gtccaacagg agatcgacga 4800
cgtgataggg caggtgcggc gaccagagat gggtgaccag gctcacatgc cctacaccac 4860
tgccgtgatt catgaggtgc agcgctttgg ggacatcgtc cccctgggtg tgacccatat 4920

gacatcccgt gacatcgaag tacagggctt ccgcatccct aaggtaggcc tggcgccctc 4980
ctcaccccag ctcagcacca gcacctggtg atagccccag catggctact gccaggtggg 5040
cccactctag gaaccctggc cacctagtcc tcaatgccac cacactgact gtccccactt 5100
gggtgggggg tccagagtat aggcagggct ggcctgtcca tccagagccc ccgtctagtg 5160
gggagacaaa ccaggacctg ccagaatgtt ggaggaccca acgcctgcag ggagaggggg 5220
cagtgtgggt gcctctgaga ggtgtgactg cgccctgctg tggggtcgga gagggtactg 5280
tggagcttct cgggcgcagg actagttgac agagtccagc tgtgtgccag gcagtgtgtg 5340
tcccccgtgt gtttggtggc aggggtccca gcatcctaga gtccagtccc cactctcacc 5400
ctgcatctcc tgcccaggga acgacactca tcaccaacct gtcatcggtg ctgaaggatg 5460
aggccgtctg ggagaagccc ttccgcttcc accccgaaca cttcctggat gcccagggcc 5520
actttgtgaa gccggaggcc ttcctgcctt tctcagcagg tgcctgtggg gagcccggct 5580
ccctgtcccc ttccgtggag tcttgcaggg gtatcaccca ggagccaggc tcactgacgc 5640
ccctcccctc cccacaggcc gccgtgcatg cctcggggag cccctggccc gcatggagct 5700
cttcctcttc ttcacctccc tgctgcagca cttcagcttc tcggtgccca ctggacagcc 5760
ccggcccagc caccatggtg tctttgcttt cctggtgagc ccatccccct atgagctttg 5820
tgctgtgccc cgctagaatg gggtacctag tccccagcct gctccctagc cagaggctct 5880
aatgtacaat aaagcaatgt ggtagttcca actcgggtcc cctgctcacg ccctcgttgg 5940
gatcatcctc ctcagggcaa ccccacccct gcctcattcc tgcttacccc accgcctggc 6000
cgcatttgag acaggggtac gttgaggctg agcagatgtc agttaccctt gcccataatc 6060
ccatgtcccc cactgaccca actctgactg cccagattgg tgacaaggac tacattgtcc 6120
tggcatgtgg ggaaggggcc agaatgggct gactagaggt gtcagtcagc cctggatgtg 6180
gtggagaggg caggactcag cctggaggcc catatttcag gcctaactca gcccacccca 6240
catcagggac agcagtcctg ccagcaccat cacaacagtc acctcccttc atatatgaca 6300
ccccaaaacg gaagacaaat catggcgtca gggagctata tgccagggct acctacctcc 6360
cagggctcag tcggcaggtg ccagaacgtt ccctgggaag gccccatgga agcccaggac 6420
tgagccacca ccctcagcct cgtcacctca ccacaggact ggctacctct ctgggccctc 6480
agggatgctg ctgtacagac ccctgaccag tgacgagttc gcactcaggg ccaggctggc 6540
gctggaggag gacacttgtt tggctccaac cctaggtacc atcctcccag tagggatcag 6600
gcagggccca caggcctgcc ctagggacag gagtcaacct tggacccata aggcactggg 6660
gcgggcagag aaggaggagg tggcatgggc agctgagagc cagagaccct gaccctagtc 6720
cttgctctgc cattaccccg tgtgaccccg ggcccaccct tccccaccct tccccacccc 6780
gggcttctgt ttccttctgc caacgagaag gctgcttcac ctgccccgag tcctgtcttc 6840
ctgctctgcc ttctggggct gtggcccttg ctggcctgga gccccaacca agggcaggga 6900
ctgctgtcct ccacgtctgt cctcaccgac ataatgggct gggctgggca cacaggcagt 6960

gcccaagagt ttctaatgag catatgatta cctgagtcct gggcagacct tcttagggaa 7020
cagcctggga cagagaacca cagacactct gaggagccac cctgaggcct cttttgccag 7080
aggaccctac agcctccctg gcagcagttc cgccagcatt tctgtaaatg ccctcatgcc 7140
agggtgcggc ccggctgtca gcacgagagg gacgttggtc tgtcccctgg caccgagtca 7200
gtcagaaggg tggccagggc ccccttgggc ccctccagag acaatccact gtggtcacac 7260
ggctcggtgg caggaagtgc tgttcctgca gctgtgggga cagggagtgt ggatgaagcc 7320
aggctgggtt tgtctgaaga cggaggcccc gaaaggtggc agcctggcct atagcagcag 7380
caactcttgg atttattgga aagattttct tcacggttct gagtcttggg ggtgttagag 7440
gctcagaacc agtccagcca gagctctgtc atgggcacgt agacccggtc ccagggcctt 7500
tgctctttgc tgtcctcaga ggcctctgca aagtagaaac aggcagcctt gtgagtcccc 7560
tcctgggagc aaccaaccct ccctctgaga tgccccgggg ccaggtcagc tgtggtgaaa 7620
ggtagggatg cagccagctc agggagtggc ccagagttcc tgcccaccca aggaggctcc 7680
caggaaggtc aaggcacctg actcctgggc tgcttccctc ccctcccctc cccaggtcag 7740
gaaggtggga aagggctggg gtgtctgtga ccctggcagt cactgagaag cagggtggaa 7800
gcagccccct gcagcacgct gggtcagtgg tcttaccaga tggatacgca gcaacttcct 7860
tttgaacctt tttattttcc tggcaggaag aagagggatc cagcagtgag atcaggcagg 7920
ttctgtgttg cacagacagg gaaacaggct ctgtccacac aaagtcggtg gggccaggat 7980
gaggcccagt ctgttcacac atggctgctg cctctcagct ctgcacagac gtcctcgctc 8040
ccctgggatg gcagcttggc ctgctggtct tggggttgag ccagcctcca gcactgcctc 8100
cctgccctgc tgcctcccac tctgcagtgc tccatggctg ctcagttgga cccacgctgg 8160
agacgttcag tcgaagcccc gggctgtcct tacctcccag tctggggtac ctgccacctc 8220
ctgctcagca ggaatggggc taggtgcttc ctcccctggg gacttcacct gctctccctc 8280
ctgggataag acggcagcct cctccttggg ggcagcagca ttcagtcctc caggtctcct 8340
gggggtcgtg acctgcagga ggaataagag ggcagactgg gcagaaaggc cttcagagca 8400
cctcatcctc ctgttctcac actggggtgt cacagtcctg ggaagttctt ccttttcagt 8460
tgagctgtgg taaccttgtg agtttcctgg agggggcctg ccactaccct tgggactccc 8520
tgccgtgtgt ctgggtctaa ctgagctctg aaaggagaga gccccagccc tgggccttcc 8580
aggggaagcc ttacctcaga ggttggcttc ttcctactct tgactttgcg tctctgcaga 8640
gggaggtggg aggggtgaca caaccctgac acccacacta tgagtgatga gtagtcctgc 8700
cccgactggc ccatcctttc caggtgcagt cccccttact gtgtctgcca agggtgccag 8760
cacagccgcc ccactccagg ggaagaggag tgccagccct taccacctga gtgggcacag 8820
tgtagcattt attcattagc ccccacactg gcctgaccat ctcccctgtg ggctgcatga 8880
caaggagaga gaacaggctg aggtgagagc tactgtcaac acctaaacct aaaaaatcta 8940
taattgggct gggcagggtg gctcacgcct gtaatcccag cactttggga ggccgagatg 9000

ggtggatcac ctgaggtcag atgttcgaga ccagcctggc caacatggtg aaaccccgtc 9060
tctactaaaa atacaaaaaa ttagctgggc gtggtggtgg gtgcctgtaa tcccagctac 9120
tcaggaggct gaggcaggag aattgcttga acctgggagg cagaggctgc agtgagccga 9180
gatcgcatca ttgcactcca gcctggtcaa caagagtgaa actgtcttaa aaaaaaaatc 9240
tataattgat atctttagaa agataaaact ttgcattcat gaaataagaa taggagggtc 9300
taaaataaaa atgttcaaac acccaccacc actaattctt gacaaaaata tagtctgggt 9360
gccttagctc atgcctgtaa tcccagcatt ttgggaggct aaggcaggag gattgtttga 9420
gcctaggaat tc 9432

<210> 2
<211> 9433
<212> DNA
<213> HOMO SAPIENS

<400> 2
gaattcaaga ccagcctgga caacttggaa gaacccggtc tctacaaaaa atacaaaatt 60
agctgggatt gggtgcggtg gctcatgcct ataatcccag cactttggga gcctgaggtg 120
ggtggatcac ctgaagtcag gagttcaaga ctagcctggc caacatggtg aaaccctatc 180
tctactgaaa atacaaaaag ctagacgtgg tggcacacac ctgtaatccc agctacttag 240
gaggctgagg caggagaatt gcttgaagcc tagaggtgaa ggttgtagtg agccgagatt 300
gcatcattgc acaatggagg ggagccacca gcctgggcaa caagaggaaa tctccgtctc 360
caaaaaaaaa aaaaaaaaaa aaagaattag gctgggtggt gcctgtagtc ccagctactt 420
gggaggcagg gggtccactt gatgtcgaga ctgcagtgag ccatgatcct gccactgcac 480
tccggcctgg gcaacagagt gagaccctgt ctaaagaaaa aaaaaataaa gcaacatatc 540
ctgaacaaag gatcctccat aacgttccca ccagatttct aatcagaaac atggaggcca 600
gaaagcagtg gaggaggacg accctcaggc agcccgggag gatgttgtca caggctgggg 660
caagggcctt ccggctacca actgggagct ctgggaacag ccctgttgca aacaagaagc 720
catagcccgg ccagagccca ggaatgtggg ctgggctggg agcagcctct ggacaggagt 780
ggtcccatcc aggaaacctc cggcatggct gggaagtggg gtacttggtg ccgggtctgt 840
atgtgtgtgt gactggtgtg tgtgagagag aatgtgtgcc ctaagtgtca gtgtgagtct 900
gtgtatgtgt gaatattgtc tttgtgtggg tgattttctg cgtgtgtaat cgtgtccctg 960
caagtgtgaa caagtggaca agtgtctggg agtggacaag agatctgtgc accatcaggt 1020
gtgtgcatag cgtctgtgca tgtcaagagt gcaaggtgaa gtgaagggac caggcccatg 1080
atgccactca tcatcaggag ctctaaggcc ccaggtaagt gccagtgaca gataagggtg 1140
ctgaaggtca ctctggagtg ggcaggtggg ggtagggaaa gggcaaggcc atgttctgga 1200

ggaggggttg tgactacatt agggtgtatg agcctagctg ggaggtggat ggccgggtcc 1260
actgaaaccc tggttatccc agaaggcttt gcaggcttca ggagcttgga gtggggagag 1320
ggggtgactt ctccgaccag gcccctccac cggcctaccc tgggtaaggg cctggagcag 1380
gaagcagggg caagaacctc tggagcagcc catacccgcc ctggcctgac tctgccactg 1440
gcagcacagt caacacagca ggttcactca cagcagaggg caaaggccat catcagctcc 1500
ctttataagg gaagggtcac gcgctcggtg tgctgagagt gtcctgcctg gtcctctgtg 1560
cctggtgggg tgggggtgcc aggtgtgtcc agaggagccc atttggtagt gaggcaggta 1620
tggggctaga agcactggtg cccctggccg tgatagtggc catcttcctg ctcctggtgg 1680
acctgatgca ccggcgccaa cgctgggctg cacgctaccc accaggcccc ctgccactgc 1740
ccgggctggg caacctgctg catgtggact tccagaacac accatactgc ttcgaccagg 1800
tgagggagga ggtcctggag ggcggcagag gtgctgaggc tcccctacca gaagcaaaca 1860
tggatggtgg gtgaaaccac aggctggacc agaagccagg ctgagaaggg gaagcaggtt 1920
tgggggacgt cctggagaag ggcatttata catggcatga aggactggat tttccaaagg 1980
ccaaggaaga gtagggcaag ggcctggagg tggagctgga cttggcagtg ggcatgcaag 2040
cccattgggc aacatatgtt atggagtaca aagtcccttc tgctgacacc agaaggaaag 2100
gccttgggaa tggaagatga gttagtcctg agtgccgttt aaatcacgaa atcgaggatg 2160
aagggggtgc agtgacccgg ttcaaacctt ttgcactgtg ggtcctcggg cctcactgcc 2220
tcaccggcat ggaccatcat ctgggaatgg gatgctaact ggggcctctc ggcaattttg 2280
gtgactcttg caaggtcata cctgggtgac gcatccaaac tgagttcctc catcacagaa 2340
ggtgtgaccc ccacccccgc cccacgatca ggaggctggg tctcctcctt ccacctgctc 2400
actcctggta gccccggggg tcgtccaagg ttcaaatagg actaggacct gtagtctggg 2460
gtgatcctgg cttgacaaga ggccctgacc ctccctctgc agttgcggcg ccgcttcggg 2520
gacgtgttca gcctgcagct ggcctggacg ccggtggtcg tgctcaatgg gctggcggcc 2580
gtgcgcgagg cgctggtgac ccacggcgag gacaccgccg accgcccgcc tgtgcccatc 2640
acccagatcc tgggtttcgg gccgcgttcc caaggcaagc agcggtgggg acagagacag 2700
atttccgtgg gacccgggtg ggtgatgacc gtagtccgag ctgggcagag agggcgcggg 2760
gtcgtggaca tgaaacaggc cagcgagtgg ggacagcggg ccaagaaacc acctgcacta 2820
gggaggtgtg agcatgggga cgagggcggg gcttgtgacg agtgggcggg gccactgccg 2880
agacctggca ggagcccaat gggtgagcgt ggcgcatttc ccagctggaa tccggtgtcg 2940
aagtgggggc ggggaccgca cctgtgctgt aagctcagtg tgggtggcgc ggggcccgcg 3000
gggtcttccc tgagtgcaaa ggcggtcagg gtgggcagag acgaggtggg gcaaagcctg 3060
ccccagccaa gggagcaagg tggatgcaca aagagtgggc cctgtgacca gctggacaga 3120
gccagggact gcgggagacc agggggagca tagggttgga gtgggtggtg gatggtgggg 3180
ctaatgcctt catggccacg cgcacgtgcc cgtcccaccc ccaggggtgt tcctggcgcg 3240

ctatgggccc gcgtggcgcg agcagaggcg cttctccgtg tccaccttgc gcaacttggg 3300
cctgggcaag aagtcgctgg agcagtgggt gaccgaggag gccgcctgcc tttgtgccgc 3360
cttcgccaac cactccggtg ggtgatgggc agaagggcac aaagcgggaa ctgggaaggc 3420
gggggacggg gaaggcgacc ccttacccgc atctcccacc cccaggacgc ccctttcgcc 3480
ccaacggtct cttggacaaa gccgtgagca acgtgatcgc ctccctcacc tgcgggcgcc 3540
gcttcgagta cgacgaccct cgcttcctca ggctgctgga cctagctcag gagggactga 3600
aggaggagtc gggctttctg cgcgaggtgc ggagcgagag accgaggagt ctctgcaggg 3660
cgagctcccg agaggtgccg gggctggact ggggcctcgg aagagcagga tttgcataga 3720
tgggtttggg aaaggacatt ccaggagacc ccactgtaag aagggcctgg aggaggaggg 3780
gacatctcag acatggtcgt gggagaggtg tgcccgggtc agggggcacc aggagaggcc 3840
aaggactctg tacctcctat ccacgtcaga gatttcgatt ttaggtttct cctctgggca 3900
aggagagagg gtggaggctg gcacttgggg agggacttgg tgaggtcagt ggtaaggaca 3960
ggcaggccct gggtctacct ggagatggct ggggcctgag acttgtccag gtgaacgcag 4020
agcacaggag ggattgagac cccgttctgt ctggtgtagg tgctgaatgc tgtccccgtc 4080
ctcctgcata tcccagcgct ggctggcaag gtcctacgct tccaaaaggc tttcctgacc 4140
cagctggatg agctgctaac tgagcacagg atgacctggg acccagccca gcccccccga 4200
gacctgactg aggccttcct ggcagagatg gagaaggtga gagtggctgc cacggtgggg 4260
ggcaagggtg gtgggttgag cgtcccagga ggaatgaggg gaggctgggc aaaaggttgg 4320
accagtgcat cacccggcga gccgcatctg ggctgacagg tgcagaattg gaggtcattt 4380
gggggctacc ccgttctgtc ccgagtatgc tctcggccct gctcaggcca aggggaaccc 4440
tgagagcagc ttcaatgatg agaacctgcg catagtggtg gctgacctgt tctctgccgg 4500
gatggtgacc acctcgacca cgctggcctg gggcctcctg ctcatgatcc tacatccgga 4560
tgtgcagcgt gagcccatct gggaaacagt gcaggggccg agggaggaag ggtacaggcg 4620
ggggcccatg aactttgctg ggacacccgg ggctccaagc acaggcttga ccaggatcct 4680
gtaagcctga cctcctccaa cataggaggc aagaaggagt gtcagggccg gaccccctgg 4740
gtgctgaccc attgtgggga cgcatgtctg tccaggccgt gtccaacagg agatcgacga 4800
cgtgataggg caggtgcggc gaccagagat gggtgaccag gctcacatgc cctacaccac 4860
tgccgtgatt catgaggtgc agcgctttgg ggacatcgtc cccctgggtg tgacccatat 4920
gacatcccgt gacatcgaag tacagggctt ccgcatccct aaggtaggcc tggcgccctc 4980
ctcaccccag ctcagcacca gcacctggtg atagccccag catggctact gccaggtggg 5040
cccactctag gaaccctggc cacctagtcc tcaatgccac cacactgact gtccccactt 5100
gggtgggggg tccagagtat aggcagggct ggcctgtcca tccagagccc ccgtctagtg 5160
gggagacaaa ccaggacctg ccagaatgtt ggaggaccca acgcctgcag ggagaggggg 5220
cagtgtgggt gcctctgaga ggtgtgactg cgccctgctg tggggtcgga gagggtactg 5280

tggagcttct cgggcgcagg actagttgac agagtccagc tgtgtgccag gcagtgtgtg 5340
tcccccgtgt gtttggtggc aggggtccca gcatcctaga gtccagtccc cactctcacc 5400
ctgcatctcc tgcccaggga acgacactca tcaccaacct gtcatcggtg ctgaaggatg 5460
aggccgtctg ggagaagccc ttccgcttcc accccgaaca cttcctggat gcccagggcc 5520
actttgtgaa gccggaggcc ttcctgcctt tctcagcagg tgcctgtggg gagcccggct 5580
ccctgtcccc ttccgtggag tcttgcaggg gtatcaccca ggagccaggc tcactgacgc 5640
ccctcccctc cccacaggcc gccgtgcatg cctcggggag cccctggccc gcatggagct 5700
cttcctcttc ttcacctccc tgctgcagca cttcagcttc tcggtgccca ctggacagcc 5760
ccggcccagc caccatggtg tctttgcttt cctggtgacc ccatccccct atgagtattt 5820
gtgctgtgcc ccgctagaat ggggtaccta gtccccagcc tgctccctag ccagaggctc 5880
taatgtacaa taaagcaatg tggtagttcc aactcgggtc ccctgctcac gccctcgttg 5940
ggatcatcct cctcagggca accccacccc tgcctcattc ctgcttaccc caccgcctgg 6000
ccgcatttga gacaggggta cgttgaggct gagcagatgt cagttaccct tgcccataat 6060
cccatgtccc ccactgaccc aactctgact gcccagattg gtgacaagga ctacattgtc 6120
ctggcatgtg gggaaggggc cagaatgggc tgactagagg tgtcagtcag ccctggatgt 6180
ggtggagagg gcaggactca gcctggaggc ccatatttca ggcctaactc agcccacccc 6240
acatcaggga cagcagtcct gccagcacca tcacaacagt cacctccctt catatatgac 6300
accccaaaac ggaagacaaa tcatggcgtc agggagctat atgccagggc tacctacctc 6360
ccagggctca gtcggcaggt gccagaacgt tccctgggaa ggccccatgg aagcccagga 6420
ctgagccacc accctcagcc tcgtcacctc accacaggac tggctacctc tctgggccct 6480
cagggatgct gctgtacaga cccctgacca gtgacgagtt cgcactcagg gccaggctgg 6540
cgctggagga ggacacttgt ttggctccaa ccctaggtac catcctccca gtagggatca 6600
ggcagggccc acaggcctgc cctagggaca ggagtcaacc ttggacccat aaggcactgg 6660
ggcgggcaga gaaggaggag gtggcatggg cagctgagag ccagagaccc tgaccctagt 6720
ccttgctctg ccattacccc gtgtgacccc gggcccaccc ttccccaccc ttccccaccc 6780
cgggcttctg tttccttctg ccaacgagaa ggctgcttca cctgccccga gtcctgtctt 6840
cctgctctgc cttctggggc tgtggccctt gctggcctgg agccccaacc aagggcaggg 6900
actgctgtcc tccacgtctg tcctcaccga cataatgggc tgggctgggc acacaggcag 6960
tgcccaagag tttctaatga gcatatgatt acctgagtcc tgggcagacc ttcttaggga 7020
acagcctggg acagagaacc acagacactc tgaggagcca ccctgaggcc tcttttgcca 7080
gaggacccta cagcctccct ggcagcagtt ccgccagcat ttctgtaaat gccctcatgc 7140
cagggtgcgg cccggctgtc agcacgagag ggacgttggt ctgtcccctg gcaccgagtc 7200
agtcagaagg gtggccaggg cccccttggg cccctccaga gacaatccac tgtggtcaca 7260
cggctcggtg gcaggaagtg ctgttcctgc agctgtgggg acagggagtg tggatgaagc 7320

caggctgggt ttgtctgaag acggaggccc cgaaaggtgg cagcctggcc tatagcagca 7380
gcaactcttg gatttattgg aaagattttc ttcacggttc tgagtcttgg gggtgttaga 7440
ggctcagaac cagtccagcc agagctctgt catgggcacg tagacccggt cccagggcct 7500
ttgctctttg ctgtcctcag aggcctctgc aaagtagaaa caggcagcct tgtgagtccc 7560
ctcctgggag caaccaaccc tccctctgag atgccccggg gccaggtcag ctgtggtgaa 7620
aggtagggat gcagccagct cagggagtgg cccagagttc ctgcccaccc aaggaggctc 7680
ccaggaaggt caaggcacct gactcctggg ctgcttccct cccctcccct ccccaggtca 7740
ggaaggtggg aaagggctgg ggtgtctgtg accctggcag tcactgagaa gcagggtgga 7800
agcagccccc tgcagcacgc tgggtcagtg gtcttaccag atggatacgc agcaacttcc 7860
ttttgaacct ttttattttc ctggcaggaa gaagagggat ccagcagtga gatcaggcag 7920
gttctgtgtt gcacagacag ggaaacaggc tctgtccaca caaagtcggt ggggccagga 7980
tgaggcccag tctgttcaca catggctgct gcctctcagc tctgcacaga cgtcctcgct 8040
cccctgggat ggcagcttgg cctgctggtc ttggggttga gccagcctcc agcactgcct 8100
ccctgccctg ctgcctccca ctctgcagtg ctccatggct gctcagttgg acccacgctg 8160
gagacgttca gtcgaagccc cgggctgtcc ttacctccca gtctggggta cctgccacct 8220
cctgctcagc aggaatgggg ctaggtgctt cctcccctgg ggacttcacc tgctctccct 8280
cctgggataa gacggcagcc tcctccttgg gggcagcagc attcagtcct ccaggtctcc 8340
tgggggtcgt gacctgcagg aggaataaga gggcagactg ggcagaaagg ccttcagagc 8400
acctcatcct cctgttctca cactggggtg tcacagtcct gggaagttct tccttttcag 8460
ttgagctgtg gtaaccttgt gagtttcctg gagggggcct gccactaccc ttgggactcc 8520
ctgccgtgtg tctgggtcta actgagctct gaaaggagag agccccagcc ctgggccttc 8580
caggggaagc cttacctcag aggttggctt cttcctactc ttgactttgc gtctctgcag 8640
agggaggtgg gaggggtgac acaaccctga cacccacact atgagtgatg agtagtcctg 8700
ccccgactgg cccatccttt ccaggtgcag tcccccttac tgtgtctgcc aagggtgcca 8760
gcacagccgc cccactccag gggaagagga gtgccagccc ttaccacctg agtgggcaca 8820
gtgtagcatt tattcattag cccccacact ggcctgacca tctcccctgt gggctgcatg 8880
acaaggagag agaacaggct gaggtgagag ctactgtcaa cacctaaacc taaaaaatct 8940
ataattgggc tgggcagggt ggctcacgcc tgtaatccca gcactttggg aggccgagat 9000
gggtggatca cctgaggtca gatgttcgag accagcctgg ccaacatggt gaaaccccgt 9060
ctctactaaa aatacaaaaa attagctggg cgtggtggtg ggtgcctgta atcccagcta 9120
ctcaggaggc tgaggcagga gaattgcttg aacctgggag gcagaggctg cagtgagccg 9180
agatcgcatc attgcactcc agcctggtca acaagagtga aactgtctta aaaaaaaaat 9240
ctataattga tatctttaga aagataaaac tttgcattca tgaaataaga ataggagggt 9300
ctaaaataaa aatgttcaaa cacccaccac cactaattct tgacaaaaat atagtctggg 9360

tgccttagct catgcctgta atcccagcat tttgggaggc taaggcagga ggattgtttg 9420
agcctaggaa ttc 9433

<210> 3
<211> 1567
<212> DNA
<213> Homo Sapien

<400> 3
atggggctag aagcactggt gcccctggcc gtgatagtgg ccatcttcct gctcctggtg 60
gacctgatgc accggcgcca acgctgggct gcacgctacc caccaggccc cctgccactg 120
cccgggctgg gcaacctgct gcatgtggac ttccagaaca caccatactg cttcgaccag 180
ttgcggcgcc gcttcgggga cgtgttcagc ctgcagctgg cctggacgcc ggtggtcgtg 240
ctcaatgggc tggcggccgt gcgcgaggcg ctggtgaccc acggcgagga caccgccgac 300
cgcccgcctg tgcccatcac ccagatcctg ggtttcgggc cgcgttccca aggggtgttc 360
ctggcgcgct atgggcccgc gtggcgcgag cagaggcgct tctccgtgtc caccttgcgc 420
aacttgggcc tgggcaagaa gtcgctggag cagtgggtga ccgaggaggc cgcctgcctt 480
tgtgccgcct tcgccaacca ctccggacgc ccctttcgcc ccaacggtct cttggacaaa 540
gccgtgagca acgtgatcgc ctccctcacc tgcgggcgcc gcttcgagta cgacgaccct 600
cgcttcctca ggctgctgga cctagctcag gagggactga aggaggagtc gggctttctg 660
cgcgaggtgc tgaatgctgt ccccgtcctc ctgcatatcc cagcgctggc tggcaaggtc 720
ctacgcttcc aaaaggcttt cctgacccag ctggatgagc tgctaactga gcacaggatg 780
acctgggacc cagcccagcc cccccgagac ctgactgagg ccttcctggc agagatggag 840
aaggccaagg ggaaccctga gagcagcttc aatgatgaga acctgcgcat agtggtggct 900
gacctgttct ctgccgggat ggtgaccacc tcgaccacgc tggcctgggg cctcctgctc 960
atgatcctac atccggatgt gcagcgccgt gtccaacagg agatcgacga cgtgataggg 1020
caggtgcggc gaccagagat gggtgaccag gctcacatgc cctacaccac tgccgtgatt 1080
catgaggtgc agcgctttgg ggacatcgtc cccctgggta tgacccatat gacatcccgt 1140
gacatcgaag tacagggctt ccgcatccct aagggaacga cactcatcac caacctgtca 1200
tcggtgctga aggatgaggc cgtctgggag aagcccttcc gcttccaccc cgaacacttc 1260
ctggatgccc agggccactt tgtgaagccg gaggccttcc tgcctttctc agcaggccgc 1320
cgtgcatgcc tcggggagcc cctggcccgc atggagctct tcctcttctt cacctccctg 1380
ctgcagcact tcagcttctc ggtgcccact ggacagcccc ggcccagcca ccatggtgtc 1440
tttgctttcc tggtgagccc atccccctat gagctttgtg ctgtgccccg ctagaatggg 1500
gtacctagtc cccagcctgc tcctagccca gaggctctaa tgtacaataa agcaatgtgg 1560

tagttcc                                                              1567

<210>  4
<211>  1568
<212>  DNA
<213>  Homo Sapien

<400>  4
atggggctag aagcactggt gcccctggcc gtgatagtgg ccatcttcct gctcctggtg      60
gacctgatgc accggcgcca acgctgggct gcacgctacc caccaggccc cctgccactg     120
cccgggctgg gcaacctgct gcatgtggac ttccagaaca caccatactg cttcgaccag     180
ttgcggcgcc gcttcgggga cgtgttcagc ctgcagctgg cctggacgcc ggtggtcgtg     240
ctcaatgggc tggcggccgt gcgcgaggcg ctggtgaccc acggcgagga caccgccgac     300
cgcccgcctg tgcccatcac ccagatcctg ggtttcgggc cgcgttccca aggggtgttc     360
ctggcgcgct atgggcccgc gtggcgcgag cagaggcgct tctccgtgtc caccttgcgc     420
aacttgggcc tgggcaagaa gtcgctggag cagtgggtga ccgaggaggc cgcctgcctt     480
tgtgccgcct tcgccaacca ctccggacgc ccctttcgcc ccaacggtct cttggacaaa     540
gccgtgagca acgtgatcgc ctccctcacc tgcgggcgcc gcttcgagta cgacgaccct     600
cgcttcctca ggctgctgga cctagctcag gagggactga aggaggagtc gggctttctg     660
cgcgaggtgc tgaatgctgt ccccgtcctc ctgcatatcc cagcgctggc tggcaaggtc     720
ctacgcttcc aaaaggcttt cctgacccag ctggatgagc tgctaactga gcacaggatg     780
acctgggacc cagcccagcc cccccgagac ctgactgagg ccttcctggc agagatggag     840
aaggccaagg ggaaccctga gagcagcttc aatgatgaga acctgcgcat agtggtggct     900
gacctgttct ctgccgggat ggtgaccacc tcgaccacgc tggcctgggg cctcctgctc     960
atgatcctac atccggatgt gcagcgccgt gtccaacagg agatcgacga cgtgataggg    1020
caggtgcggc gaccagagat gggtgaccag gctcacatgc cctacaccac tgccgtgatt    1080
catgaggtgc agcgctttgg ggacatcgtc cccctgggta tgacccatat gacatcccgt    1140
gacatcgaag tacagggctt ccgcatccct aagggaacga cactcatcac caacctgtca    1200
tcggtgctga aggatgaggc cgtctgggag aagcccttcc gcttccaccc cgaacacttc    1260
ctggatgccc agggccactt tgtgaagccg gaggccttcc tgcctttctc agcaggccgc    1320
cgtgcatgcc tcggggagcc cctggcccgc atggagctct tcctcttctt cacctccctg    1380
ctgcagcact tcagcttctc ggtgcccact ggacagcccc ggcccagcca ccatggtgtc    1440
tttgctttcc tggtgagccc atccccctat gagtatttgt gctgtgcccc gctagaatgg    1500
ggtacctagt ccccagcctg ctcctagccc agaggctcta atgtacaata aagcaatgtg    1560
gtagttcc                                                             1568

<210>  5

<211>  497

<212>  PRT

<213>  Homo Sapien


<400>  5

Met Gly Leu Glu Ala Leu Val Pro Leu Ala Val Ile Val Ala Ile Phe
1               5                   10                  15

Leu Leu Leu Val Asp Leu Met His Arg Arg Gln Arg Trp Ala Ala Arg
            20                  25                  30

Tyr Pro Pro Gly Pro Leu Pro Leu Pro Gly Leu Gly Asn Leu Leu His
        35                  40                  45

Val Asp Phe Gln Asn Thr Pro Tyr Cys Phe Asp Gln Leu Arg Arg Arg
    50                  55                  60

Phe Gly Asp Val Phe Ser Leu Gln Leu Ala Trp Thr Pro Val Val Val
65                  70                  75                  80

Leu Asn Gly Leu Ala Ala Val Arg Glu Ala Leu Val Thr His Gly Glu
                85                  90                  95

Asp Thr Ala Asp Arg Pro Pro Val Pro Ile Thr Gln Ile Leu Gly Phe
            100                 105                 110

Gly Pro Arg Ser Gln Gly Val Phe Leu Ala Arg Tyr Gly Pro Ala Trp
        115                 120                 125

Arg Glu Gln Arg Arg Phe Ser Val Ser Thr Leu Arg Asn Leu Gly Leu
    130                 135                 140

Gly Lys Lys Ser Leu Glu Gln Trp Val Thr Glu Glu Ala Ala Cys Leu
145                 150                 155                 160

Cys Ala Ala Phe Ala Asn His Ser Gly Arg Pro Phe Arg Pro Asn Gly
                165                 170                 175

Leu Leu Asp Lys Ala Val Ser Asn Val Ile Ala Ser Leu Thr Cys Gly
            180                 185                 190

Arg Arg Phe Glu Tyr Asp Asp Pro Arg Phe Leu Arg Leu Leu Asp Leu
        195                 200                 205

Ala Gln Glu Gly Leu Lys Glu Glu Ser Gly Phe Leu Arg Glu Val Leu
    210                 215                 220

Asn Ala Val Pro Val Leu Leu His Ile Pro Ala Leu Ala Gly Lys Val
225                 230                 235                 240

Leu Arg Phe Gln Lys Ala Phe Leu Thr Gln Leu Asp Glu Leu Leu Thr
                245                 250                 255

Glu His Arg Met Thr Trp Asp Pro Ala Gln Pro Pro Arg Asp Leu Thr
            260                 265                 270

Glu Ala Phe Leu Ala Glu Met Glu Lys Ala Lys Gly Asn Pro Glu Ser
        275                 280                 285

Ser Phe Asn Asp Glu Asn Leu Arg Ile Val Val Ala Asp Leu Phe Ser
    290                 295                 300

Ala Gly Met Val Thr Thr Ser Thr Thr Leu Ala Trp Gly Leu Leu Leu
305                 310                 315                 320

Met Ile Leu His Pro Asp Val Gln Arg Arg Val Gln Gln Glu Ile Asp
                325                 330                 335

Asp Val Ile Gly Gln Val Arg Arg Pro Glu Met Gly Asp Gln Ala His
            340                 345                 350

Met Pro Tyr Thr Thr Ala Val Ile His Glu Val Gln Arg Phe Gly Asp
        355                 360                 365

Ile Val Pro Leu Gly Met Thr His Met Thr Ser Arg Asp Ile Glu Val
    370                 375                 380

Gln Gly Phe Arg Ile Pro Lys Gly Thr Thr Leu Ile Thr Asn Leu Ser
385                 390                 395                 400

Ser Val Leu Lys Asp Glu Ala Val Trp Glu Lys Pro Phe Arg Phe His
                405                 410                 415

Pro Glu His Phe Leu Asp Ala Gln Gly His Phe Val Lys Pro Glu Ala
            420                 425                 430

Phe Leu Pro Phe Ser Ala Gly Arg Arg Ala Cys Leu Gly Glu Pro Leu
        435                 440                 445

Ala Arg Met Glu Leu Phe Leu Phe Phe Thr Ser Leu Leu Gln His Phe
    450                 455                 460

Ser Phe Ser Val Pro Thr Gly Gln Pro Arg Pro Ser His His Gly Val
465                 470                 475                 480

Phe Ala Phe Leu Val Ser Pro Ser Pro Tyr Glu Leu Cys Ala Val Pro
                485                 490                 495

Arg

<210> 6

<211> 502

<212> PRT

<213> Homo Sapien

<400> 6

Met Gly Leu Glu Ala Leu Val Pro Leu Ala Val Ile Val Ala Ile Phe
1               5                   10                  15

Leu Leu Leu Val Asp Leu Met His Arg Arg Gln Arg Trp Ala Ala Arg
            20                  25                  30

Tyr Pro Pro Gly Pro Leu Pro Leu Pro Gly Leu Gly Asn Leu Leu His
        35                  40                  45

Val Asp Phe Gln Asn Thr Pro Tyr Cys Phe Asp Gln Leu Arg Arg Arg
    50                  55                  60

Phe Gly Asp Val Phe Ser Leu Gln Leu Ala Trp Thr Pro Val Val Val
65                  70                  75                  80

Leu Asn Gly Leu Ala Ala Val Arg Glu Ala Leu Val Thr His Gly Glu
                85                  90                  95

Asp Thr Ala Asp Arg Pro Pro Val Pro Ile Thr Gln Ile Leu Gly Phe
            100                 105                 110

Gly Pro Arg Ser Gln Gly Val Phe Leu Ala Arg Tyr Gly Pro Ala Trp
        115                 120                 125

Arg Glu Gln Arg Arg Phe Ser Val Ser Thr Leu Arg Asn Leu Gly Leu
    130                 135                 140

Gly Lys Lys Ser Leu Glu Gln Trp Val Thr Glu Glu Ala Ala Cys Leu
145                 150                 155                 160

Cys Ala Ala Phe Ala Asn His Ser Gly Arg Pro Phe Arg Pro Asn Gly
                165                 170                 175

Leu Leu Asp Lys Ala Val Ser Asn Val Ile Ala Ser Leu Thr Cys Gly
            180                 185                 190

Arg Arg Phe Glu Tyr Asp Asp Pro Arg Phe Leu Arg Leu Leu Asp Leu
        195                 200                 205

Ala Gln Glu Gly Leu Lys Glu Glu Ser Gly Phe Leu Arg Glu Val Leu
    210                 215                 220

Asn Ala Val Pro Val Leu Leu His Ile Pro Ala Leu Ala Gly Lys Val
225                 230                 235                 240

Leu Arg Phe Gln Lys Ala Phe Leu Thr Gln Leu Asp Glu Leu Leu Thr
                245                 250                 255

Glu His Arg Met Thr Trp Asp Pro Ala Gln Pro Pro Arg Asp Leu Thr
            260                 265                 270

Glu Ala Phe Leu Ala Glu Met Glu Lys Ala Lys Gly Asn Pro Glu Ser
        275                 280                 285

Ser Phe Asn Asp Glu Asn Leu Arg Ile Val Val Ala Asp Leu Phe Ser
    290                 295                 300

Ala Gly Met Val Thr Thr Ser Thr Thr Leu Ala Trp Gly Leu Leu Leu
305                 310                 315                 320

Met Ile Leu His Pro Asp Val Gln Arg Arg Val Gln Gln Glu Ile Asp
                325                 330                 335

Asp Val Ile Gly Gln Val Arg Arg Pro Glu Met Gly Asp Gln Ala His
            340                 345                 350

Met Pro Tyr Thr Thr Ala Val Ile His Glu Val Gln Arg Phe Gly Asp
        355                 360                 365

Ile Val Pro Leu Gly Met Thr His Met Thr Ser Arg Asp Ile Glu Val
    370                 375                 380

Gln Gly Phe Arg Ile Pro Lys Gly Thr Thr Leu Ile Thr Asn Leu Ser
385                 390                 395                 400

Ser Val Leu Lys Asp Glu Ala Val Trp Glu Lys Pro Phe Arg Phe His
                405                 410                 415

Pro Glu His Phe Leu Asp Ala Gln Gly His Phe Val Lys Pro Glu Ala
            420                 425                 430

Phe Leu Pro Phe Ser Ala Gly Arg Arg Ala Cys Leu Gly Glu Pro Leu
        435                 440                 445

Ala Arg Met Glu Leu Phe Leu Phe Phe Thr Ser Leu Leu Gln His Phe
    450                 455                 460

Ser Phe Ser Val Pro Thr Gly Gln Pro Arg Pro Ser His His Gly Val
465                 470                 475                 480

Phe Ala Phe Leu Val Ser Pro Ser Pro Tyr Glu Tyr Leu Cys Cys Ala
                485                 490                 495

Pro Leu Glu Trp Gly Thr
            500

<210>  7

<211>  1565

<212>  DNA

<213>  Homo Sapien


<400>  7
atggggctag aagcactggt gcccctggcc gtgatagtgg ccatcttcct gctcctggtg      60

gacctgatgc accggcgcca acgctgggct gcacgctacc caccaggccc cctgccactg     120

cccgggctgg gcaacctgct gcatgtggac ttccagaaca caccatactg cttcgaccag     180

ttgcggcgcc gcttcgggga cgtgttcagc ctgcagctgg cctggacgcc ggtggtcgtg     240

ctcaatgggc tggcggccgt gcgcgaggcg ctggtgaccc acggcgagga caccgccgac     300

cgcccgcctg tgcccatcac ccagatcctg ggtttcgggc cgcgttccca aggggtgttc     360

ctggcgcgct atgggcccgc gtggcgcgag cagaggcgct tctccgtgtc caccttgcgc     420

aacttgggcc tgggcaagaa gtcgctggag cagtgggtga ccgaggaggc cgcctgcctt     480

tgtgccgcct tcgccaacca ctccggacgc ccctttcgcc ccaacggtct cttggacaaa     540

gccgtgagca acgtgatcgc ctccctcacc tgcgggcgcc gcttcgagta cgacgaccct     600

cgcttcctca ggctgctgga cctagctcag gagggactga aggaggagtc gggctttctg     660

cgcgaggtgc tgaatgctgt ccccgtcctc ctgcatatcc cagcgctggc tggcaaggtc     720

ctacgcttcc aaaaggcttt cctgacccag ctggatgagc tgctaactga gcacaggatg     780

acctgggacc cagcccagcc cccccgagac ctgactgagg ccttcctggc agagatggag     840

aaggccaagg ggaaccctga gagcagcttc aatgatgaga acctgcgcat agtggtggct     900

gacctgttct ctgccgggat ggtgaccacc tcgaccacgc tggcctgggg cctcctgctc     960

atgatcctac atccggatgt gcagcgccgt gtccaacagg agatcgacga cgtgataggg    1020

caggtgcggc gaccagagat gggtgaccag gctcacatgc cctacaccac tgccgtgatt    1080

catgaggtgc agcgctttgg ggacatcgtc cccctgggta tgacccatat gacatcccgt    1140

gacatcgaag tacagggctt ccgcatccct aagggaacga cactcatcac caacctgtca    1200

tcggtgctga aggatgaggc cgtctgggag aagcccttcc gcttccaccc cgaacacttc    1260

ctggatgccc agggccactt tgtgaagccg gaggccttcc tgcctttctc agcaggccgc    1320

cgtgcatgcc tcggggagcc cctggcccgc atggagctct tcctcttctt cacctccctg    1380

ctgcagcact tcagcttctc ggtgcccact ggacagcccc ggcccagcca ccatggtgtc    1440

tttgctttcc tggtgacccc atccccctat gagtatttgt gctgtgcccc gctagaatgg 1500
ggtacctccc cagcctgctc ctagcccaga ggctctaatg tacaataaag caatgtggta 1560
gttcc 1565

<210> 8
<211> 502
<212> PRT
<213> Homo Sapien

<400> 8
Met Gly Leu Glu Ala Leu Val Pro Leu Ala Val Ile Val Ala Ile Phe
1               5                   10                  15

Leu Leu Leu Val Asp Leu Met His Arg Arg Gln Arg Trp Ala Ala Arg
            20                  25                  30

Tyr Pro Pro Gly Pro Leu Pro Leu Pro Gly Leu Gly Asn Leu Leu His
        35                  40                  45

Val Asp Phe Gln Asn Thr Pro Tyr Cys Phe Asp Gln Leu Arg Arg Arg
    50                  55                  60

Phe Gly Asp Val Phe Ser Leu Gln Leu Ala Trp Thr Pro Val Val Val
65                  70                  75                  80

Leu Asn Gly Leu Ala Ala Val Arg Glu Ala Leu Val Thr His Gly Glu
                85                  90                  95

Asp Thr Ala Asp Arg Pro Pro Val Pro Ile Thr Gln Ile Leu Gly Phe
            100                 105                 110

Gly Pro Arg Ser Gln Gly Val Phe Leu Ala Arg Tyr Gly Pro Ala Trp
        115                 120                 125

Arg Glu Gln Arg Arg Phe Ser Val Ser Thr Leu Arg Asn Leu Gly Leu
    130                 135                 140

Gly Lys Lys Ser Leu Glu Gln Trp Val Thr Glu Glu Ala Ala Cys Leu
145                 150                 155                 160

Cys Ala Ala Phe Ala Asn His Ser Gly Arg Pro Phe Arg Pro Asn Gly
                165                 170                 175

Leu Leu Asp Lys Ala Val Ser Asn Val Ile Ala Ser Leu Thr Cys Gly
            180                 185                 190

Arg Arg Phe Glu Tyr Asp Asp Pro Arg Phe Leu Arg Leu Leu Asp Leu
        195                 200                 205

Ala Gln Glu Gly Leu Lys Glu Glu Ser Gly Phe Leu Arg Glu Val Leu
    210                 215                 220

Asn Ala Val Pro Val Leu Leu His Ile Pro Ala Leu Ala Gly Lys Val
225                 230                 235                 240

Leu Arg Phe Gln Lys Ala Phe Leu Thr Gln Leu Asp Glu Leu Leu Thr
                245                 250                 255

Glu His Arg Met Thr Trp Asp Pro Ala Gln Pro Pro Arg Asp Leu Thr
            260                 265                 270

Glu Ala Phe Leu Ala Glu Met Glu Lys Ala Lys Gly Asn Pro Glu Ser
        275                 280                 285

Ser Phe Asn Asp Glu Asn Leu Arg Ile Val Val Ala Asp Leu Phe Ser
    290                 295                 300

Ala Gly Met Val Thr Thr Ser Thr Thr Leu Ala Trp Gly Leu Leu Leu
305                 310                 315                 320

Met Ile Leu His Pro Asp Val Gln Arg Arg Val Gln Gln Glu Ile Asp
                325                 330                 335

Asp Val Ile Gly Gln Val Arg Arg Pro Glu Met Gly Asp Gln Ala His
            340                 345                 350

Met Pro Tyr Thr Thr Ala Val Ile His Glu Val Gln Arg Phe Gly Asp
        355                 360                 365

Ile Val Pro Leu Gly Met Thr His Met Thr Ser Arg Asp Ile Glu Val
    370                 375                 380

Gln Gly Phe Arg Ile Pro Lys Gly Thr Thr Leu Ile Thr Asn Leu Ser
385                 390                 395                 400

Ser Val Leu Lys Asp Glu Ala Val Trp Glu Lys Pro Phe Arg Phe His
                405                 410                 415

Pro Glu His Phe Leu Asp Ala Gln Gly His Phe Val Lys Pro Glu Ala
            420                 425                 430

Phe Leu Pro Phe Ser Ala Gly Arg Arg Ala Cys Leu Gly Glu Pro Leu
        435                 440                 445

Ala Arg Met Glu Leu Phe Leu Phe Phe Thr Ser Leu Leu Gln His Phe
    450                 455                 460

Ser Phe Ser Val Pro Thr Gly Gln Pro Arg Pro Ser His His Gly Val
465                 470                 475                 480

Phe Ala Phe Leu Val Thr Pro Ser Pro Tyr Glu Tyr Leu Cys Cys Ala
                485                 490                 495

Pro Leu Glu Trp Gly Thr
            500

<210>  9

<211>  15

<212>  DNA

<213>  Artificial Sequence

<220>

<223>  primer

<220>

<221>  primer_bind

<222>  (1)..(15)

<223>

<400>  9
catcccccta tgagc                                                        15

<210>  10

<211>  15

<212>  DNA

<213>  Artificial Sequence

<220>

<223>  primer

<400>  10
gggcacagca caaag                                                        15

<210>  11

<211>  15

<212>  DNA

<213>  Artificial Sequence

<220>

<223>  primer

<400>  11
catcccccta tgagt                                                        15

<210>  12

<211>  15

<212>  DNA

<213>  Artificial Sequence

<220>

<223>  primer

<400>  12
atccccctat gagta                                                        15

<210>  13

<211>  15

<212>  DNA

<213>  Artificial Sequence

<220>

<223>  primer

<400>  13
gggcacagca caaat                                                        15

<210>  14

<211>  15

<212>  DNA

<213>  Artificial Sequence

<220>

<223>  primer

<400>  14
ggcacagcac aaata                                                        15

<210>  15

<211>  15

<212>  DNA

<213>  Artificial Sequence

<220>

<223>  primer

<400>  15
tgctttcctg gtgag                                                        15

<210>  16

<211>  15

<212>  DNA

<213>  Artificial Sequence

<220>

<223>  primer

<400>  16
   cataggggga tgggc                                                         15

<210>  17

<211>  15

<212>  DNA

<213>  Artificial Sequence

<220>

<223>  primer

<400>  17
tgctttcctg gtgac                                                        15

<210>  18

<211>  15

<212>  DNA

<213>  Artificial Sequence

<220>

<223>  primer

<400>  18
   cataggggga tgggg                                                         15

<210>  19

<211>  17

<212>  DNA

<213>  Artificial Sequence



<220>

<223>  probe

<400>  19
cctatgagct ttgtgct                                                    17

<210>  20

<211>  17

<212>  DNA

<213>  Artificial Sequence



<220>

<223>  probe

<400>  20
agcacaaagc tcatagg                                                    17

<210>  21

<211>  18

<212>  DNA

<213>  Artificial Sequence



<220>

<223>  probe

<400>  21
cctatgagta tttgtgct                                                   18

<210>  22

<211>  18

<212>  DNA

<213>  Artificial Sequence



<220>

<223>  probe

<400>  22
agcacaaata ctcatagg                                                   18

<210>  23

<211>  17

<212>  DNA

<213>  Artificial Sequence

<220>

<223>  probe

<400>  23
cctggtgagc ccatccc                                                    17

<210>  24

<211>  17

<212>  DNA

<213>  Artificial Sequence

<220>

<223>  probe

<400>  24
gggatgggct caccagg                                                    17

<210>  25

<211>  17

<212>  DNA

<213>  Artificial Sequence

<220>

<223>  probe

<400>  25
cctggtgacc ccatccc                                                    17

<210>  26

<211>  17

<212>  DNA

<213>  Artificial Sequence

<220>

<223>  probe

<400>  26
gggatggggt caccagg                                                    17

<210>  27

<211>  9

<212>  DNA

<213>  Homo Sapien

<400>  27
tcacccgtg                                                              9

<210>  28

<211>  250

<212>  DNA

<213>  Homo Sapien

<400>  28
cgccgtgcat gcctcgggga gcccctggcc cgcatggagc tcttcctctt cttcacctcc      60
ctgctgcagc acttcagctt ctcggtgccc actggacagc cccggcccag ccaccatggt     120
gtctttgctt tcctggtgag cccatccccc tatgagcttt gtgctgtgcc ccgctagaat     180
ggggtaccta gtccccagcc tgctccctag ccagaggctc taatgtacaa taaagcaatg     240
tggtagttcc                                                            250

<210>  29

<211>  251

<212>  DNA

<213>  Homo Sapien

<400>  29
cgccgtgcat gcctcgggga gcccctggcc cgcatggagc tcttcctctt cttcacctcc      60
ctgctgcagc acttcagctt ctcggtgccc actggacagc cccggcccag ccaccatggt     120
gtctttgctt tcctggtgag cccatccccc tatgagtatt tgtgctgtgc cccgctagaa     180
tggggtacct agtccccagc ctgctcccta gccagaggct ctaatgtaca ataaagcaat     240
gtggtagttc c                                                          251

<210>  30

<211>  22

<212>  DNA

<213>  Artificial Sequence

<220>

<223>  primer

<400>  30
ccagaaggct ttgcaggctt ca                                                22

<210>  31

<211>  22

<212>  DNA

<213>  Artificial Sequence

<220>

<223>  primer

<400>  31
actgagccct gggaggtagg ta                                                22

<210>  32

<211>  33

<212>  DNA

<213>  Artificial Sequence

<220>

<223>  primer

<400>  32
tgtaaaacga cggccagtag ccaggctcac tga                                    33

<210>  33

<211>  42

<212>  DNA

<213>  Artificial Sequence

<220>

<223>  primer

<400>  33
caggaaacag ctatgacctg atcccaacga gggcgtgagc ag                          42

<210>  34

<211>  11

<212>  PRT

<213>  Artificial Sequence



<220>

<223>  mutant carboxy-terminus

<400>  34

Tyr Leu Cys Cys Ala Pro Leu Glu Trp Gly Thr
1               5                   10

<210>  35

<211>  6

<212>  PRT

<213>  Artificial Sequence



<220>

<223>  wildtype carboxy-terminus

<400>  35

Leu Cys Ala Val Pro Arg
1               5

<210>  36

<211>  496

<212>  PRT

<213>  Homo Sapien



<400>  36

Met Gly Leu Glu Ala Leu Val Pro Leu Ala Val Ile Val Ala Ile Phe
1               5                   10                  15

Leu Leu Leu Val Asp Leu Met His Arg Arg Gln Arg Trp Ala Ala Arg
            20                  25                  30

Tyr Pro Pro Gly Pro Leu Pro Leu Pro Gly Leu Gly Asn Leu Leu His
        35                  40                  45

Val Asp Phe Gln Asn Thr Pro Tyr Cys Phe Asp Gln Leu Arg Arg Arg
    50                  55                  60

Phe Gly Asp Val Phe Ser Leu Gln Leu Ala Trp Thr Pro Val Val Val
65                  70                  75                  80

Leu Asn Gly Leu Ala Ala Val Arg Glu Ala Leu Val Thr His Gly Glu
                85                  90                  95

Asp Thr Ala Asp Arg Pro Pro Val Pro Ile Thr Gln Ile Leu Gly Phe
            100                 105                 110

Gly Pro Arg Ser Gln Gly Val Phe Leu Ala Arg Tyr Gly Pro Ala Trp
        115                 120                 125

Arg Glu Gln Arg Arg Phe Ser Val Ser Thr Leu Arg Asn Leu Gly Leu
    130                 135                 140

Gly Lys Lys Ser Leu Glu Gln Trp Val Thr Glu Glu Ala Ala Cys Leu
145                 150                 155                 160

Cys Ala Ala Phe Ala Asn His Ser Gly Arg Pro Phe Arg Pro Asn Gly
                165                 170                 175

Leu Leu Asp Lys Ala Val Ser Asn Val Ile Ala Ser Leu Thr Cys Gly
            180                 185                 190

Arg Arg Phe Glu Tyr Asp Asp Pro Arg Phe Leu Arg Leu Leu Asp Leu
        195                 200                 205

Ala Gln Glu Gly Leu Lys Glu Glu Ser Gly Phe Leu Arg Glu Val Leu
    210                 215                 220

Asn Ala Val Pro Val Leu Leu His Ile Pro Ala Leu Ala Gly Lys Val
225                 230                 235                 240

Leu Arg Phe Gln Lys Ala Phe Leu Thr Gln Leu Asp Glu Leu Leu Thr
                245                 250                 255

Glu His Arg Met Thr Trp Asp Pro Ala Gln Pro Pro Arg Asp Leu Thr
            260                 265                 270

Glu Ala Phe Leu Ala Glu Met Glu Lys Ala Lys Gly Asn Pro Glu Ser
        275                 280                 285

Ser Phe Asn Asp Glu Asn Leu Arg Ile Val Val Ala Asp Leu Phe Ser
    290                 295                 300

Ala Gly Met Val Thr Thr Ser Thr Thr Leu Ala Trp Gly Leu Leu Leu
305                 310                 315                 320

Met Ile Leu His Pro Asp Val Gln Arg Arg Val Gln Gln Glu Ile Asp
                325                 330                 335

Asp Val Ile Gly Gln Val Arg Arg Pro Glu Met Gly Asp Gln Ala His
            340                 345                 350

Met Pro Tyr Thr Thr Ala Val Ile His Glu Val Gln Arg Phe Gly Asp
        355                 360                 365

Ile Val Pro Leu Gly Met Thr His Met Thr Ser Arg Asp Ile Glu Val
    370                 375                 380

Gln Gly Phe Arg Ile Pro Lys Gly Thr Thr Leu Ile Thr Asn Leu Ser
385                 390                 395                 400

Ser Val Leu Lys Asp Glu Ala Val Trp Glu Lys Pro Phe Arg Phe His
                405                 410                 415

Pro Glu His Phe Leu Asp Ala Gln Gly His Phe Val Lys Pro Glu Ala
            420                 425                 430

Phe Leu Pro Phe Ser Ala Gly Arg Arg Ala Cys Leu Gly Glu Pro Leu
        435                 440                 445

Ala Arg Met Glu Leu Phe Leu Phe Phe Thr Ser Leu Leu Gln His Phe
    450                 455                 460

Ser Phe Ser Val Pro Thr Gly Gln Pro Arg Pro Ser His His Gly Val
465                 470                 475                 480

Phe Ala Phe Leu Val Thr Pro Ser Pro Tyr Glu Leu Cys Ala Val Pro
                485                 490                 495

<210>  37

<211>  58

<212>  PRT

<213>  Homo Sapien


<400>  37

Arg Arg Ala Cys Leu Gly Glu Pro Leu Ala Arg Met Glu Leu Phe Leu
1               5                   10                  15

Phe Phe Thr Ser Leu Leu Gln His Phe Ser Phe Ser Val Pro Thr Gly
            20                  25                  30

Gln Pro Arg Pro Ser His His Gly Val Phe Ala Phe Leu Val Ser Pro
        35                  40                  45

Ser Pro Tyr Glu Leu Cys Ala Val Pro Arg
    50                  55

<210>  38

<211>  62

<212>  PRT

<213>  Homo Sapien

<400>  38

Arg Ala Cys Leu Gly Glu Pro Leu Ala Arg Met Glu Leu Phe Leu Phe
1               5                   10                  15

Phe Thr Ser Leu Leu Gln His Phe Ser Phe Ser Val Pro Thr Gly Gln
            20                  25                  30

Pro Arg Pro Ser His His Gly Val Phe Ala Phe Leu Val Ser Pro Ser
        35                  40                  45

Pro Tyr Glu Tyr Leu Cys Cys Ala Pro Leu Glu Trp Gly Thr
    50                  55                  60